# EUROPÄISCHE PATENTSCHRIFT

(11) **EP 1 523 473 B1**
(45) Veröffentlichungstag und Bekanntmachung des Hinweises auf die Patenterteilung: **27.02.2013**
(21) Anmeldenummer: 03765079.3
(22) Anmeldetag: 22.07.2003
(51) Int. Cl.: C07D 209/34, C07D 403/12, C07D 401/12, C07D 409/12, C07D 405/12, A61K 31/404, A61K 31/4045, A61P 35/00

(54) **IN 6-STELLUNG SUBSTITUIERTE INDOLINONDERIVATE, IHRE HERSTELLUNG UND IHRE VERWENDUNG ALS ARZNEIMITTEL**
INDOLINE DERIVATIVES SUBSTITUTED IN POSITION 6, PRODUCTION AND USE THEREOF AS MEDICAMENTS
DERIVES D'INDOLINONE SUBSTITUES EN POSITION 6, LEUR PREPARATION ET LEUR UTILISATION COMME MEDICAMENTS

(30) Priorität: 23.07.2002 DE 10233366; 24.06.2003 DE 10328533
(43) Veröffentlichungstag der Anmeldung: 20.04.2005
(73) Patentinhaber: Boehringer Ingelheim Pharma GmbH & Co. KG, 55216 Ingelheim am Rhein (DE)
(72) Erfinder: ROTH, Gerald, Jürgen, 88400 Biberach (DE); HECKEL, Armin, 88400 Biberach (DE); KLEY, Jörg, 88441 Mittelbiberach (DE); LEHMANN-LINTZ, Thorsten, 88416 Ochsenhausen (DE); HILBERG, Frank, A-1050 Wien (AT); TONTSCH-GRUNT, Ulrike, A-2500 Baden (AT); VAN MEEL, Jacobus (Jacques), C.A., A-2340 Moedling (AT)
(86) Internationale Anmeldenummer: PCT/EP2003/007961
(87) Internationale Veröffentlichungsnummer: WO 2004/009547

(56) Entgegenhaltungen:
- WO-A-00/18734
- WO-A-00/56710
- WO-A-03/026650
- WO-A-03/027102

## Beschreibung

Die vorliegende Erfindung betrifft in 6-Stellung substituierte Indolinonderivate der allgemeinen Formel 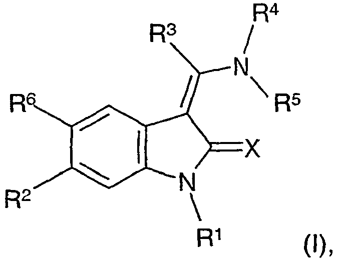 deren Tautomere, Enantiomere, Diastereomere, deren Gemische und deren Salze, insbesondere deren physiologisch verträgliche Salze, welche wertvolle pharmakologische Eigenschaften aufweisen, diese Verbindungen enthaltende Arzneimittel, deren Verwendung und Verfahren zu ihrer Herstellung.

Die obigen Verbindungen der allgemeinen Formel I weisen wertvolle pharmakologische Eigenschaften auf, insbesondere eine inhibierende Wirkung auf verschiedene Kinasen, vor allem auf Rezeptor-Tyrosinkinasen wie VEGFR1, VEGFR2, VEGFR3, PDGFRα, PDGFRβ, FGFR1, FGFR3, EGFR, HER2, c-Kit, IGF1R und HGFR, Flt-3, sowie auf die Proliferation kultivierter humaner Zellen, insbesondere die von Endothelzellen, z.B. bei der Angiogenese, aber auch auf die Proliferation anderer Zellen, insbesondere von Tumorzellen. WO00/18734 beschreibt substituierte Indolinone mit inhibierender Wirkung auf verschiedene Kinasen.

Gegenstand der vorliegenden Erfindung sind somit die obigen Verbindungen der allgemeinen Formel I, die wertvolle pharmakologische Eigenschaften aufweisen, die diese pharmakologisch wirksamen Verbindungen enthaltenden Arzneimittel, deren Verwendung und Verfahren zu ihrer Herstellung.

Gegenstand der vorliegenden Erfindung sind ausserdem die physiologisch verträglichen Salze der erfindungsgemäßen Verbindungen, die diese Verbindungen enthaltenden Arzneimittel, die zusätzlich gegebenenfalls einen oder mehrere inerte Trägerstoffe und/oder Verdünnungsmittel enthalten, sowie deren Verwendung zur Herstellung eines Arzneimittels, welches insbesondere zur Behandlung von exzessiven oder anomalen Zellproliferationen geeignet ist.

Gegenstand der vorliegenden Erfindung sind weiterhin die Verfahren zur Herstellung dieses Arzneimittels, welche insbesondere dadurch gekennzeichnet sind, dass die erfindungsgemäßen Verbindungen oder deren physiologisch verträglichen Salze in einen oder mehrere inerte Trägerstoffe und/oder Verdünnungsmittel eingearbeitet werden.
I. In der obigen allgemeinen Formel I bedeuten
   X ein Sauerstoffatom,
   R¹ ein Wasserstoffatom,
   R² ein Fluor-, Chlor- oder Brom-atom oder eine Cyanogruppe,
   R³ eine Phenylgruppe in 3- oder 4-Position
   durch eine Carboxy-C₁₋₃-alkyl-, C₁₋₄-Alkoxy-carbonyl-C₁₋₃-alkyl-, Aminocarbonyl-C₁₋₃-alkyl-, (C₁₋₂-Alkyl-amino)-carbonyl-C₁₋₃-alkyl-, oder C₁₋₄-Alkoxy-carbonyl-C₂₋₃-alkenyl-gruppe,
   substituiert,
   R⁴ eine Phenylgruppe oder eine
   durch eine endständig durch eine Amino-, Guanidino-, Mono- oder Di-(C₁₋₂-alkyl)-amino-, *N*-[ω-Di-(C₁₋₃-alkyl)-amino-C₂₋₃-alkyl]-*N*-(C₁₋₃-alkyl)-amino-, N-Methyl-(C₃₋₄-alkyl)-amino-, N-(C₁₋₃-Alkyl)-N-benzylamino-, N-(C₁₋₄-Alkoxycarbonyl)-amino-, N-(C₁₋₄-Alkoxycarbonyl)-C₁₋₄-alkylamino-, 4-(C₁₋₃-Alkyl)-piperazin-1-yl-, Imidazol-1-yl-, Pyrrolidin-1-yl-, Azetidin-1-yl-, Morpholin-4-yl-, Piperazin-1-yl-, Thiomorpholin-4-yl-gruppe substituierte C₁₋₃-Alkyl-gruppe,
   durch eine Di-(C₁₋₃-alkyl)-amino-(C₁₋₃-alkyl)-sulfonyl-, 2-[Di-(C₁₋₃-alkyl)-amino]-ethoxy-, 4-(C₁₋₃-Alkyl)-piperazin-1-yl-carbonyl-, {ω-[Di-(C₁₋₃-alkyl)-amino]-(C₂₋₃-alkyl)}-N-(C₁₋₃-alkyl)-aminocarbonyl-, 1-(C₁₋₃-Alkyl)-imidazol-2-yl-, (C₁₋₃-Alkyl)-sulfonyl-gruppe, oder
   durch eine Gruppe der Formel 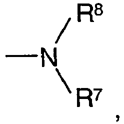 in der
   R⁷ eine C₁₋₂-Alkyl-, C₁₋₂-Alkyl-carbonyl-, Di-(C₁₋₂-alkyl)-aminocarbonyl-C₁₋₃-alkyl- oder C₁₋₃-Alkylsulfonyl-gruppe und
   R⁸ eine C₁₋₃-Alkyl-, ω-[Di-(C₁₋₂-alkyl)-amino]-C₂₋₃-alkyl-, ω-[Mono-(C₁₋₂-alkyl)-amino]-C₂₋₃-alkyl-gruppe, oder
   eine endständig durch eine Di-(C₁₋₂-alkyl)-amino-, Piperazin-1-yl-oder 4-(C₁₋₃-Alkyl)-piperazin-1-yl-gruppe substituierte (C₁₋₃-Alkyl)-carbonyl-, (C₄₋₆-Alkyl)-carbonyl-, oder Carbonyl-(C₁₋₃-alkyl)-gruppe bedeuten,
   monosubstituierte Phenylgruppe ist,
   wobei alle im Rest R⁴ enthaltenen Dialkylaminogruppen auch in quaternisierter Form vorliegen können, beispielsweise als N-Methyl-(N,N-dialkyl)-ammoniumgruppe, wobei das Gegenion vorzugsweise ausgewählt ist aus Iodid, Chlorid, Bromid, Methylsulfonat, para-Toluolsulfonat, oder Trifluoracetat,
   R⁵ ein Wasserstoffatom und
   R⁶ ein Wasserstoffatom bedeuten,
   wobei die oben erwähnten Alkylgruppen lineare und verzweigten Alkylgruppen einschließen, in denen zusätzlich ein bis 3 Wasserstoffatome durch Fluoratome ersetzt sein können,
   wobei zusätzlich eine vorhandene Carboxy-, Amino- oder Imino-gruppe durch einen in vivo abspaltbaren Rest substituiert sein kann, beziehungsweise in Form eines Prodrug-Restes vorliegen kann, beispielsweise in Form einer in-vivo in eine Carboxygruppe überführbaren Gruppe oder in Form einer in-vivo in eine Imino- oder Amino-gruppe überführbaren Gruppe,
   deren Tautomere, Enantiomere, Diastereomere, deren Gemische und deren Salze.
II. Besonders bevorzugte Verbindungen der obigen allgemeinen Formel I sind diejenigen, in denen X, R¹, R⁵ und R⁶ wie unter I. definiert sind und:
   II.i. R² und R⁴ wie unter I. definiert sind und
      R³ eine Phenylgruppe in3- oder 4-Position durch eine

      Carboxy-C₁₋₃-alkyl-C₁₋₄ Alkoxy-carbonyl-C₁₋₃-alkyl-, Aminocarbonyl-C₁₋₃-alkyl-, (C₁₋₂-Alkyl-amino)-carbonyl-C₁₋₃-alkyl-,
      oder C₁₋₄-Alkoxy-carbonyl-C₂₋₃-alkenyl-gruppe,
      substituiert sein können, wobei die Substituenten gleich oder verschieden sein können;
   II.ii. R² und R⁴ wie unter I. definiert sind und
      R³ eine
      durch eine Carboxy-C₁₋₃-alkyl-, C₁₋₄-Alkoxycarbonyl-C₁₋₃-alkyl-, Aminocarbonyl-C₁₋₃-alkyl-, (C₁₋₂-Alkylamino)-carbonyl-C₁₋₃-alkyl-,
      oder C₁₋₄-Alkoxycarbonyl-C₂₋₃-alkenyl-gruppe,
      substituierte Phenylgruppe bedeutet;
   II.iii. R² und R⁴ wie unter I. definiert sind und
      R³ eine durch eine Carboxy-C₁₋₃-alkyl- oder C₁₋₄-Alkoxy-carbonyl-C₁₋₃-alkylgruppe substituierte Phenylgruppe bedeutet;
   II.iv R³ und R⁴ wie unter I. definiert sind und
      R² ein Fluor- oder Chlor-atom ist;
   II.v. R² und R³ wie unter I. definiert sind und
      R⁴ eine Phenylgruppe oder eine
      durch eine endständig durch eine Amino-, Guanidino-, Mono- oder Di-(C₁₋₂-alkyl)-amino-, *N*-[ω-Di-(C₁₋₃-alkyl)-amino-C₂₋₃-alkyl]-*N*-(C₁₋₃-alkyl)-amino-, N-Methyl-(C₃₋₄-alkyl)-amino-, N-(C₁₋₃-Alkyl)-N-benzylamino-, N-(C₁₋₄-Alkoxycarbonyl)-amino-, N-(C₁₋₄-Alkoxycarbonyl)-C₁₋₄-alkylamino-, 4-(C₁₋₃-Alkyl)-piperazin-1-yl-, Imidazol-1-yl-, Pyrrolidin-1-yl-, Azetidin-1-yl-, Morpholin-4-yl-, Piperazin-1-yl-, Thiomorpholin-4-yl-gruppe substituierte C₁₋₃-Alkyl-gruppe,
      durch eine Di-(C₁₋₃-alkyl)-amino-(C₁₋₃-alkyl)-sulfonyl-, 2-[Di-(C₁₋₃-alkyl)-amino]-ethoxy-, 4-(C₁₋₃-Alkyl)-piperazin-1-yl-carbonyl-, {ω-[Di-(C₁₋₃-alkyl)-amino]-(C₂₋₃-alkyl)}-N-(C₁₋₃-alkyl)-amino-carbonyl-, 1-(C₁₋₃-Alkyl)-imidazol-2-yl-, (C₁₋₃-Alkyl)-sulfonyl-gruppe, oder
      durch eine Gruppe der Formel 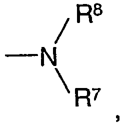 in der
      R⁷ eine C₁₋₂-Alkyl-, C₁₋₂-Alkyl-carbonyl-, Di-(C₁₋₂-alkyl)-aminocarbonyl-C₁₋₃-alkyl- oder C₁₋₃-Alkylsulfonyl-gruppe und
      R⁸ eine C₁₋₃-Alkyl-, ω-[Di-(C₁₋₂-alkyl)-amino]-C₂₋₃-alkyl-, ω-[Mono-(C₁₋₂-alkyl)-amino]-C₂₋₃-alkyl-gruppe, oder
      eine endständig durch eine Di-(C₁₋₂-alkyl)-amino-, Piperazin-1-yl- oder 4-(C₁₋₃-Alkyl)-piperazin-1-yl-gruppe substituierte (C₁₋₃-Alkyl)-carbonyl-, (C₄₋₆-Alkyl)-carbonyl-, oder Carbonyl-(C₁₋₃-alkyl)-gruppe bedeuten,
      monosubstituierte Phenylgruppe ist,
      wobei alle im Rest R⁴ enthaltenen Dialkylaminogruppen auch in quaternisierter Form vorliegen können, beispielsweise als N-Methyl-(N,N-dialkyl)-ammoniumgruppe, wobei das Gegenion vorzugsweise ausgewählt ist aus Iodid, Chlorid, Bromid, Methylsulfonat, para-Toluolsulfonat, oder Trifluoracetat.
III. Besonders zu erwähnende Untergruppen von besonders bevorzugten Verbindungen der obigen allgemeinen Formel I sind diejenigen in denen:
   111.i. X, R¹, R², R⁵ und R⁶ wie unter 1. definiert sind, R³ wie unter II.i. definiert ist, und R⁴ wie unter II.v. definiert ist;
   III.ii. X, R¹, R², R⁵ und R⁶ wie unter I. definiert sind, R³ wie unter II.ii. definiert ist, und R⁴ wie unter II.v. definiert ist;
   III.iii. X, R¹, R², R⁵ und R⁶ wie unter I. definiert sind, R³ wie unter II.iii. definiert ist, und R⁴ wie unter II.v. definiert ist;
   III.iv. X, R¹, R⁵ und R⁶ wie unter I. definiert sind, R² wie unter II.iv. definiert ist, R³ wie unter II.i, II.ii. oder II.iii. definiert ist, und R⁴ wie unter II.v. definiert ist;

Eine weitere bevorzugte Gruppe von Verbindungen der obigen allgemeinen Formel I sind diejenigen, in denen
X ein Sauerstoffatom,
R¹ ein Wasserstoffatom,
R² ein Fluor-, Chlor- oder Brom-atom oder eine Cyanogruppe,
R³ eine Phenylgruppe in 3- oder 4-Position
durch eine Carboxy-C₁₋₃-alkyl-, Aminocarbonyl-C₁₋₃-alkyl-, (C₁₋₂-Alkylamino)-carbonyl-C₁₋₃-alkyl-,
substituiert sein kann,
R⁴ eine Phenylgruppe, die
durch eine endständig durch eine Di-(C₁₋₂-alkyl)-aminogrüppe substituierte C₁-₃-Alkyl-gruppe, oder
durch eine Gruppe der Formel 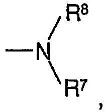 in der
R⁷ eine C₁₋₂-Alkyl-, C₁₋₂-Alkyl-carbonyl-, Di-(C₁₋₂-alkyl)-amino-carbonyl-C₁₋₃-alkyl- oder C₁₋₃-Alkylsulfonyl-gruppe und
R⁸ eine C₁₋₃-Alkyl- oder ω-[Di-(C₁₋₂-alkyl)-amino]-C₂₋₃-alkyl-gruppe, oder
eine endständig durch eine Di-(C₁₋₂-alkyl)-amino-, Piperazino- oder 4-(C₁₋₃-Alkyl)-piperazin-1-yl-gruppe substituierte C₁₋₃-Alkyl-carbonylgruppe bedeuten, substituiert ist,
R⁵ ein Wasserstoffatom und
R⁶ ein Wasserstoffatom bedeuten,
wobei die oben erwähnten Alkylgruppen lineare und verzweigten Alkylgruppen einschließen, in denen zusätzlich ein bis 3 Wasserstoffatome durch Fluoratome ersetzt sein können,
deren Tautomere, Enantiomere, Diastereomere, deren Gemische und deren Salze.

Folgende Verbindungen der allgemeinen Formel I sind besonders bevorzugt:
(a) 3-Z-[1-(4-Dimethylaminomethyl-anilino)-1-(3-(2-carboxy-ethyl)-phenyl)-methylen]-6-chlor-2-indolinon
(b) 3-Z-[1-(4-Dimethylaminomethyl-anilino)-1-(4-(2-carboxy-ethyl)-phenyl)-methylen]-6-fluor-2-indolinon
(c) 3-Z-[1-(4-Dimethylaminomethyl-anilino)-1-(3-(2-carboxy-ethyl)-phenyl)-methylen]-6-fluor-2-indolinon
(d) 3-Z-[1-(4-(N-(4-Methyl-piperazin-1-yl-methylcarbonyl)-N-methy(-amino)-anilino)-1-(4-(2-carboxy-ethyl)-phenyl)-methylen]-6-fluor-2-indolinon
(e) 3-Z-[1-(4-(N-(2-Dimethylamino-ethyl)-N-methylsulfonyl-amino)-anilino)-1-(4-(2-carboxy-ethyl)-phenyl)-methylen]-6-fluor-2-indolinon
(f) 3-Z-[1-(4-(N-(3-Dimethylamino-propyl)-N-acetyl-amino)-anilino)-1-(4-(2-carboxyethyl)-phenyl)-methylen]-6-fluor-2-indolinon
(g) 3-Z-[1-(4-(1-Methyl-imidazol-2-yl)-anilino)-1-(4-(2-carboxy-ethyl)-phenyl)-methylen]-6-fluor-2-indolinon
(h) 3-Z-[1-(4-(N-(Dimethylamino-methylcarbonyl)-N-methyl-amino)-anilino)-1-(4-(2-carboxy-ethyl)-phenyl)-methylen]-6-fluor-2-indolinon
(i) 3-Z-[1-(4-(N-(2-Dimethylamino-ethylcarbonyl)-N-methyl-amino)-anilino)-1-(4-(2-carboxy-ethyl)-phonyl)-methylen]-6-fluor-2-indolinon
(j) 3-Z-[1-(4-(Pyrrolidin-1-yl-methyl)-anilino)-1-(4-(2-carboxy-ethyl)-phenyl)-methylen]-6-fluor-2-indolinon
(k) 3-Z-[1-(4-(Diethylaminomethyl)-anilino)-1-(4-(2-carboxy-ethyl)-phenyl)-methylen]-6-fluor-2-indolinon
(I) 3-Z-[1-(4-(2-Dimethylamino-ethyl)-anilino)-1-(4-(2-carboxy-ethyl)-phenyl)-methylen]-6-chlor-2-indolinon
(m) 3-Z-[1-(4-Dimethylaminomethyl-anilino)-1-(4-(2-carboxy-ethyl)-phenyl)-methylen]-6-chlor-2-indolinon
(n) 3-Z-[1-(4-(Pyrrolidin-1-yl-methyl)-anilino)-1-(4-(2-carboxy-ethyl)-phenyl)-methylen]-6-chlor-2-indolinon
(o) 3-Z-[1-(4-(Pyrrolidin-1-yl-methyl)-anilino)-1-(4-(2-carboxy-ethyl)-phenyl)-methylen]-6-brom-2-indolinon
(p) 3-Z-[1-(4-(Dimethylaminomethyl)-anilino)-1-(4-(2-carboxy-ethyl)-phenyl)-methylen]-6-brom-2-indolinon
(q) 3-Z-[1-(4-(Diethylaminomethyl)-anilino)-1-(4-(2-carboxy-ethyl)-methylen]-6-brom-2-indolinon
sowie deren Salze.

Erfindungsgemäß erhält man die neuen Verbindungen beispielsweise nach folgenden im Prinzip literaturbekannten Verfahren:
a. Umsetzung einer Verbindung der allgemeinen Formel 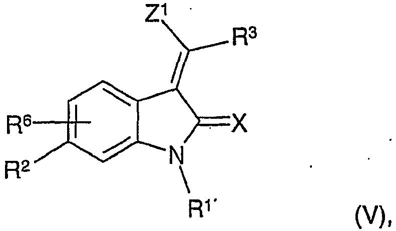 in der
   die Reste Z¹ und R³ gegebenenfalls die Positionen tauschen können,
   X, R², R³ und R⁶ wie eingangs erwähnt definiert sind,
   R¹¹ die für R¹ eingangs erwähnten Bedeutungen besitzt oder eine Schutzgruppe für das Stickstoffatom der Lactamgruppe darstellt, wobei R¹ auch eine gegebenenenfalls über einen Spacer gebildete Bindung an eine Festphase darstellen kann,
   und Z¹ ein Halogenatom, eine Hydroxy-, Alkoxy- oder Aryl-alkoxygruppe, z.B. ein Chlor- oder Bromatom, eine Methoxy-, Ethoxy- oder Benzyloxygruppe, bedeutet,
   mit einem Amin der allgemeinen Formel 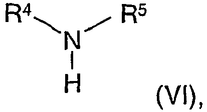 in der
   R⁴ und R⁵ wie eingangs erwähnt definiert sind,
   und erforderlichenfalls anschließende Abspaltung einer verwendeten Schutzgruppe für das Stickstoffatom der Lactamgruppe oder von einer Festphase.
   Als Schutzgruppe für das Stickstoffatom der Lactamgruppe kommt beispielsweise eine Acetyl-, Benzoyl-, Ethoxycarbonyl-, tert.Butyloxycarbonyl- oder Benzyloxycarbonylgruppe und
   als Festphase ein Harz wie ein 4-(2',4'-Dimethoxyphenylaminomethyl)-phenoxyharz, wobei die Bindung zweckmäßigerweise über die Aminogruppe erfolgt, oder ein p-Benzyloxybenzylalkoholharz, wobei die Bindung zweckmäßigerweise über ein Zwischenglied wie ein 2,5-Dimethoxy-4-hydroxy-benzylderivat erfolgt, in Betracht.
   Die Umsetzung wird zweckmäßigerweise in einem Lösungsmittel wie Dimethylformamid, Toluol, Acetonitril, Tetrahydrofuran, Dimethylsulfoxid, Methylenchlorid oder deren Gemischen gegebenenfalls in Gegenwart einer inerten Base wie Triethylamin, N-Ethyl-diisopropylamin oder Natriumhydrogencarbonat bei Temperaturen zwischen 20 und 175°C durchgeführt, wobei eine verwendete Schutzgruppe infolge Umamidierung gleichzeitig abgespalten werden kann.
   Bedeutet Z¹ in einer Verbindung der allgemeinen Formel V ein Halogenatom, dann wird die Umsetzung vorzugsweise in Gegenwart einer inerten Base bei Temperaturen zwischen 20 und 120°C, durchgeführt.
   Bedeutet Z¹ in einer Verbindung der allgemeinen Formel V eine Hydroxy-, Alkoxy- oder Arylalkoxygruppe, dann wird die Umsetzung vorzugsweise bei Temperaturen zwischen 20 und 200°C, durchgeführt.
   Die gegebenenfalls erforderliche anschließende Abspaltung einer verwendeten Schutzgruppe wird zweckmäßigerweise entweder hydrolytisch in einem wäßrigen oder alkoholischen Lösungsmittel, z.B. in Methanol/Wasser, Ethanol/Wasser, Isopropanol/Wasser, Tetrahydrofuran/Wasser, Dioxan/Wasser, Dimethylformamid/- Wasser, Methanol oder Ethanol in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C,
   oder vorteilhafterweise durch Umamidierung mit einer organischen Base wie Ammoniak, Butylamin, Dimethylamin oder Piperidin in einem Lösungsmittel wie Methanol, Ethanol, Dimethylformamid und deren Gemischen oder in einem Überschuß des eingesetzten Amins bei Temperaturen zwischen 0 und 100°C, vorzugsweise bei Temperaturen zwischen 10 und 50°C, durchgeführt.
   Die Abspaltung von einer verwendeten Festphase erfolgt vorzugsweise mittels Trifluoressigsäure und Wasser bei Temperaturen zwischen 0 und 35°C, vorzugsweise bei Raumtemperatur.
b. Zur Herstellung einer Verbindung der allgemeinen Formel I, in der R³ eine durch eine C₁₋₄-Alkoxy-carbonyl-C₂₋₃-alkenylgruppe substituierte Phenyl-gruppe darstellt,
   Umsetzung einer Verbindung der allgemeinen Formel 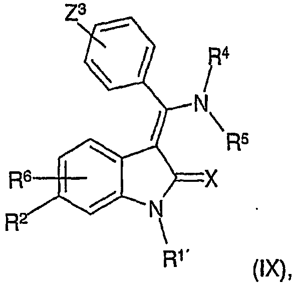 in der
   R², R⁴, R⁵, R⁶ und X wie eingangs erwähnt definiert sind,
   R¹' die für R¹ eingangs erwähnten Bedeutungen besitzt oder eine Schutzgruppe für das Stickstoffatom der Lactamgruppe darstellt, wobei R¹' auch eine gegebenenenfalls über einen Spacer gebildete Bindung an eine Festphase darstellen kann, und Z³ eine Austrittsgruppe, beispielsweise ein Halogenatom oder eine Alkyl- oder Arylsulfonyloxygruppe wie das Chlor-, Brom- oder lodatom oder die Methylsulfonyloxy-, Ethylsulfonyloxy-, p-Toluolsulfonyloxy-, oder Trifluormethansulfonyloxygruppe darstellt, mit einem Alken der allgemeinen Formel 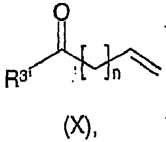 in der
   R³' eine C₁₋₄-Alkoxy-gruppe und n die Zahl 0 oder 1 bedeutet.
   Die Umsetzung erfofgt zweckmäßigerweise unter Palladium-Katalyse, beispielsweise mit Palladium(II)-acetat, Palladium(II)-chlorid, Bis-(triphenylphosphin)-palladium(II)-acetat, Bis-(triphenylphosphin)-palladium(II)-chlorid, Palladium/Aktivkohle, Bis-[1,2-Bis-(diphenylphosphino)-ethan]-palladium(0), Dichloro-(1,2-bis-(diphenylphosphino)-ethan)-palladium(II), Tetrakistriphenylphosphin-palladium(0), Tris-(dibenzyliden-aceton)-dipalladium(0), 1,1'-Bis-(diphenylphosphino)-ferrocen-dichforo-palladium(II) oder Tris-(dibenzylidenaceton)-dipalladium(0)-Chloroform-Addukt in Gegenwart einer Base wie Triethylamin, Düsopropyl-ethylamin, Lithiumcarbonat, Kaliumcarbonat, Natriumcarbonat, Cäsiumcarbonat und einem Liganden wie Triphenylphosphin,Tri-ortho-tolyl-phosphin oder Tri-(tert.butyl)-phosphin in Lösungsmitteln wie Acetonitril, N-Methyl-pyrrolidinon, Dioxan oder Dimethylformamid und deren Gemische.
   Die gegebenenfalls erforderliche Abspaltung einer verwendeten Schutzgruppe für das Stickstoffatom der Lactamgruppe oder von einer Festphase erfolgt wie vorstehend unter Verfahren (a) beschrieben.
c. Zur Herstellung einer Verbindung der allgemeinen Formel I, in der R³ eine durch
   Carboxy-C₁₋₃-alkyl-, C₁₋₄-Alkoxy-carbonyl-C₁₋₃-alkyl-, Aminocarbonyl-C₁₋₃-alkyl-oder , (C₁₋₂-Alkylamino)-carbonyl-C₁₋₃-alkyl-gruppen,
   substituierte Phenyl-gruppe darstellt,
   Hydrierung einer Verbindung der allgemeinen Formel 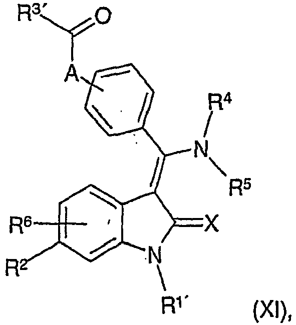 in der
   R², R⁴, R⁵, R⁶ und X wie eingangs erwähnt definiert sind,
   R¹' die für R¹ eingangs erwähnten Bedeutungen besitzt oder eine Schutzgruppe für das Stickstoffatom der Lactamgruppe darstellt, wobei R¹' auch eine gegebenenenfalls über einen Spacer gebildete Bindung an eine Festphase darstellen kann,
   A eine C₂₋₃-Alkenylgruppe und
   R³ eine Hydroxy-, C₁₋₄-Alkoxy-, Amino-oder (C₁₋₂-Alkylamino)-gruppe darstellt.

Die Hydrierung erfolgt vorzugsweise mittels katalytischer Hydrierung mit Wasserstoff in Gegenwart eines Katalysators wie Palladium/Kohle oder Platin in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die gegebenenfalls erforderliche Abspaltung einer verwendeten Schutzgruppe für das Stickstoffatom der Lactamgruppe oder von einer Festphase erfolgt wie vorstehend unter Verfahren (a) beschrieben.

Erhält man erfindungsgemäß eine Verbindung der allgemeinen Formel I, die eine Alkoxycarbonylgruppe enthält, so kann diese mittels Hydrolyse in eine entsprechende Carboxyverbindung übergeführt werden, oder
eine Verbindung der allgemeinen Formel I, die eine Amino- oder Alkylaminogruppe enthält, so kann diese mittels reduktiver Alkylierung in eine entsprechende Alkyl-amino- oder Dialkylaminoverbindung übergeführt werden, oder
eine Verbindung der allgemeinen Formel I, die eine Dialkylaminogruppe enthält, so kann diese mittels Alkylierung in eine entsprechende Trialkylammoniumverbindung übergeführt werden, oder
eine Verbindung der allgemeinen Formel I, die eine Amino- oder Alkylaminogruppe enthält, so kann diese mittels Acylierung oder Sulfonierung in eine entsprechende Acyl- oder Sulfonylverbindung übergeführt werden, oder
eine Verbindung der allgemeinen Formel I, die eine Carboxygruppe enthält, so kann diese mittels Veresterung oder Amidierung in eine entsprechende Ester- oder Aminocarbonylverbindung übergeführt werden, oder
eine Verbindung der allgemeinen Formel I, die eine Nitrogruppe enthält, so kann diese mittels Reduktion in eine entsprechende Aminoverbindung übergeführt werden, oder
eine Verbindung der allgemeinen Formel I, die Cyanogruppe enthält, so kann diese mittels Reduktion in eine entsprechende Aminomethylverbindung übergeführt werden, oder
eine Verbindung der allgemeinen Formel I, die eine Arylalkyloxygruppe enthält, so kann diese mittels Säure in eine entsprechende Hydroxyverbindung übergeführt werden, oder
eine Verbindung der allgemeinen Formel I, die eine Alkoxycarbonylgruppe enthält, so kann diese mittels Verseifung in eine entsprechende Carboxyverbindung übergeführt werden, oder
eine Verbindung der allgemeinen Formel I, in der R₄ eine durch eine Amino-, Alkyl-amino-, Aminoalkyl- oder N-Alkyl-aminogruppe substituierte Phenylgruppe darstellt, so kann diese anschliessend mittels Umsetzung mit einem entsprechenden Cyanat, Isocyanat oder Carbamoylhalogenid in eine entsprechende Harnstoffverbindung der allgemeinen Formel I übergeführt werden, oder
eine Verbindung der allgemeinen Formel I, in der R₄ eine durch eine Amino-, Alkyl-amino-, Aminoalkyl- oder N-Alkyl-aminogruppe substituierte Phenylgruppe darstellt, so kann diese anschliessend mittels Umsetzung mit einer entsprechenden die Amidinogruppe übertragenden Verbindung oder durch Umsetzung mit einem entsprechenden Nitril in eine entsprechende Guanidinoverbindung der allgemeinen Formel I übergeführt werden.

Die anschließende Hydrolyse erfolgt vorzugsweise in einem wäßrigen Lösungsmittel, z.B. in Wasser, Methanol/Wasser, Ethanol/Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die anschließende reduktive Alkylierung wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid, Natriumcyanoborhydrid oder Lithiumaluminiumhydrid bei Temperaturen zwischen 0 und 100°C, vorzugsweise bei Temperaturen zwischen 20 und 80°C, durchgeführt.

Die anschließende Alkylierung wird vorzugsweise in einem geeigneten Lösungsmittel wie Ether, Tetrahydrofuran, Dioxan, Dichlormethan, Aceton oder Acetonitril in Gegenwart von Alkylierungsmitteln wie Alkyliodiden, Alkylbromiden, Alkylchloriden, Alkyl-methansulfonsäureestern, Alkyl-para-toluolsulfonsäureestern oder Alkyltrifluoracetaten bei Temperaturen zwischen 0 und 100°C, vorzugsweise bei Temperaturen zwischen 20 und 60°C, durchgeführt.

Die anschließende Acylierung oder Sulfonylierung wird zweckmäßigerweise mit der entsprechenden freien Säure oder einer entsprechenden reaktionsfähigen Verbindung wie deren Anhydrid, Ester, Imidazolid oder Halogenid vorzugsweise in einem Lösungsmittel wie Methylenchlorid, Diethylether, Tetrahydrofuran, Toluol, Dioxan, Acetonitril, Dimethylsulfoxid oder Dimethylformamid gegebenenfalls in Gegenwart einer anorganischen oder einer tertiären organischen Base bei Temperaturen zwischen -20 und 200°C, vorzugsweise bei Temperaturen zwischen 20°C und der Siedetemperatur des verwendeten Lösungsmittel, durchgeführt. Die Umsetzung mit der freien Säure kann gegebenenfalls in Gegenwart eines die Säure aktivierenden Mittels oder eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexyl-carbödiimid/N-Hydroxysuccinimid, N,N'-Dicyclohexylcarbodiimid/1-Hydroxy-benztriazol, 2-(1 H-Benzotriazol-1-yl)-1,1,3,3-tetramethyluronium-tetrafluorborat, 2-(1 H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylamino-pyridin, N-Methyl-morpholin oder Triethylamin zweckmäßigerweise bei Temperaturen zwischen 0 und 150°C, vorzugsweise bei Temperaturen zwischen 0 und 100°C, erfolgen. Die Umsetzung mit einer entsprechenden reaktionsfähigen Verbindung kann gegebenenfalls in Gegenwart einer tertiären organischen Base wie Triethylamin, N-Ethyl-diisopropylamin, N-Methyl-morpholin oder Pyridin oder bei Verwendung eines Anhydrids bei Gegenwart der entsprechenden Säure bei Temperaturen zwischen 0 und 150°C, vorzugsweise bei Temperaturen zwischen 50 und 100°C, erfolgen.

Die anschließende Veresterung oder Amidierung wird zweckmäßigerweise durch Umsetzung eines reaktionsfähigen entsprechenden Carbonsäurederivates mit einem entsprechenden Alkohol oder Amin wie vorstehend beschrieben durchgeführt.

Die Veresterung oder Amidierung wird vorzugsweise in einem Lösungsmittel wie Methylenchlorid, Diethylether, Tetrahydrofuran, Toluol, Dioxan, Acetonitril, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt. Hierbei wird die Umsetzung mit einer entsprechenden Säure vorzugsweise in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1 H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, und die Acylierung mit einer entsprechenden reaktionsfähigen Verbindung wie deren, Anhydrid, Ester, Imidazolide oder Halogenide gegebenenfalls in Gegenwart einer tertiären organischen Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.

Die anschließende Reduktion einer Nitrogruppe erfolgt vorzugsweise hydrogenolytisch, z.B. mit Wasserstoff in Gegenwart eines Katalysators wie Palladium/Kohle oder Raney-Nickel in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die anschließende Hydrierung einer Cyanogruppe erfolgt vorzugsweise hydrogenolytisch, z.B. mit Wasserstoff in Gegenwart eines Katalysators wie Palladium/Kohle oder Raney-Nickel in einem Lösungsmittel wie Methanol, Ethanol, Essigsäureethylester, Methylenchlorid, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die anschließende Herstellung einer entsprechenden Guanidinoverbindung der allgemeinen Formel I wird zweckmäßigerweise durch Umsetzung mit einer die Amidinogruppe übertragenden Verbindung wie 3,5-Dimethylpyrazol-1-carbonsäureamidin vorzugsweise in einem Lösungsmittel wie Dimethylformamid und gegebenenfalls in Gegenwart einer tertiären organischen Base wie Triethylamin bei Temperaturen zwischen 0 und 50°C, vorzugsweise bei der Raumtemperatur, durchgeführt.

Bei den vorstehend beschriebenen Umsetzungen können gegebenenfalls vorhandene reaktive Gruppen wie Carboxy-, Hydroxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Carboxygruppe die Trimethylsilyl-, Methyl-, Ethyl-, tert.Butyl-, Benzyl- oder Tetrahydropyranylgruppe und
als Schutzrest für eine Hydroxy-, Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan, Essigester oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können erhaltene chirale Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen Gemisches diastereomerer Salze oder Derivate, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure, Dibenzoylweinsäure, Di-o-Tolylweinsäure, Apfelsäure, Mandelsäure, Camphersulfonsäure, Glutaminsäure, N-Acetylglutaminsäure, Asparaginsäure, N-Acetyl-asparaginsäure oder Chinasäure. Als optisch aktiver Alkohol kommt beispielsweise (+)- oder-(-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure, Maleinsäure, Methansulfonsäure, Ethansulfonsäure, para-Toluolsulfonsäure, Phenylsulfonsäure oder L-(+)-Mandelsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Für Verbindungen der allgemeinen Formel I, die 2 oder mehr saure oder basische Gruppen enthalten, kommen auch Salze mit 2 oder mehr anorganischen oder organischen Basen oder Säuren in Betracht (sog. Disalze etc.).

Die als Ausgangsprodukte verwendeten Verbindungen der allgemeinen Formeln V bis XI sind teilweise literaturbekannt oder man erhält diese nach literaturbekannten Verfahren oder können nach den vorstehend und in den Beispielen beschriebenen Verfahren erhalten werden. Beispielsweise werden die Verbindungen der allgemeinen Formel IX in der deutschen Patentanmeldung 198 44 003 beschrieben.

Wie bereits eingangs erwähnt, weisen die neuen Verbindungen der allgemeinen Formel I wertvolle pharmakologische Eigenschaften auf, insbesondere eine inhibierende Wirkung auf verschiedene Kinasen, vor allem auf Rezeptor-Tyrosinkinasen wie VEGFR1, VEGFR2, VEGFR3, PDGFRα, PDGFRβ, FGFR1, FGFR3, EGFR, HER2, c-Kit, IGF1 R und HGFR, Flt-3, und auf die Proliferation kultivierter humaner Zellen, insbesondere die von Endothelzellen, z.B. bei der Angiogenese, aber auch auf die Proliferation anderer Zellen, insbesondere von Tumorzellen.

Die biologischen Eigenschaften der neuen Verbindungen wurde nach folgendem Standardverfahren wie folgt geprüft:
Humane Nabelschnur Endothelzellen (HUVEC) wurden in IMDM (Gibco BRL), supplementiert mit 10 % foetalem Rinderserum (FBS) (Sigma), 50 *µ*M ß-Mercaptoeethanol (Fluka), Standardantibiotika, 15 µg/ml Endothelzellwachstumsfaktor (ECGS, Collaborative Biomedical Products) und 100 *µ*g/ml Heparin (Sigma) auf Gelatine-beschichteten Kulturflaschen (0.2 % Gelatine, Sigma) bei 37°C, 5 % CO₂ in wassergesättigter Atmosphäre kultiviert.

Zur Untersuchung der inhibitorischen Aktivität der erfindungsgemäßen Verbindungen wurden die Zellen für 16 Stunden "gehungert", d.h. in Kulturmedium ohne Wachstumsfaktoren (ECGS + Heparin) gehalten. Die Zellen wurden mittels Trypsin/EDTA von den Kulturflaschen abgelöst und einmal in serumhaltigem Medium gewaschen. Anschließend wurden 2,5 x 10³ Zellen pro well ausgesät.

Die Proliferation der Zellen wurde mit 5 ng/ml VEGF₁₆₅ (vascular endothelial growth factor; H. Weich, GBF Braunschweig) und 10 *µ*g/ml Heparin stimuliert. Pro Platte wurden jeweils 6 wells als Kontrollwert nicht stimuliert.

Die erfindungsgemäßen Verbindungen wurden in 100 % Dimethylsulfoxid gelöst und in verschiedenen Verdünnungen als Dreifachbestimmungen den Kulturen zugefügt, wobei die maximale Dimethylsulfoxid-Konzentration 0.3 % betrug.

Die Zellen wurden für 76 Stunden bei 37°C inkubiert, dann wurde für weitere 16 Stunden ³H-Thymidin (0.1 *µ* Ci/well, Amersham) zugegeben, um die DNA Synthese zu bestimmen. Anschließend wurden die radioaktiv markierten Zellen auf Filtermatten immobilisiert und die eingebaute Radioaktivität in einem ß-counter bestimmt. Zur Bestimmung der inhibitorischen Aktivität der erfindungsgemäßen Verbindungen wurde der Mittelwert der nicht-stimulierten Zellen vom Mittelwert der Faktor-stimulierten Zellen (in Anwesenheit oder Abwesenheit der erfindungsgemäßen Verbindungen) subtrahiert.

Die relative Zellproliferation wurde in Prozent der Kontrolle (HUVEC ohne Inhibitor) berechnet und die Wirkstoffkonzentration, die die Proliferation der Zellen zu 50 % hemmt (IC₅₀), abgeleitet.

Die erfindungsgemäßen Verbindungen der allgemeinen Formel I weisen einen IC₅₀ zwischen 50 µM und 1 nm auf.

Auf Grund ihrer Hemmwirkung auf die Proliferation von Zellen, insbesondere von Endothelzellen und von Tumorzellen, eignen sich die Verbindungen der allgemeinen Formel I zur Behandlung von Krankheiten, in denen die Proliferation von Zellen, insbesondere die von Endothelzellen, eine Rolle spielt.

So stellt beispielsweise die Proliferation von Endothelzellen und die damit verbundene Neovaskularisierung einen entscheidenden Schritt bei der Tumorprogression dar (Folkman J. et al., Nature 339, 58-61, (1989); Hanahan D. und Folkman J., Cell 86, 353-365, (1996)). Weiterhin ist die Proliferation von Endothelzellen auch bei Hämangiomen, bei der Metastasierung, der rheumatischen Arthritis, der Psoriasis und der okularen Neovaskularisierung von Bedeutung (Folkman J., Nature Med. 1, 27-31, (1995); Carmeliet P & Rakeh J., Nature 407, 249-257, (2000)). Der therapeutische Nutzen von Inhibitoren der Endothelzellproliferation wurde im Tiermodell beispielsweise von O'Reilly et al. und Parangi et al. gezeigt (O'Reilly M.S. et al., Cell 88, 277-285, (1997); Parangi S. et al., Proc Natl Acad Sci USA 93, 2002-2007, (1996)).

Die Verbindungen der allgemeinen Formel I, deren Tautomeren, deren Stereoisomere oder deren physiologisch verträglichen Salze eignen sich somit beispielsweise zur Behandlung von Tumoren (z.B. Plattenepithelkarzinom, Astrozytom, Kaposi's Sarkom, Glioblastom, Lungenkrebs, Blasenkrebs, Hals- und Nackenkarzimom, Oesophaguskarzinom, Melanom, Ovarkarzinom, Prostatakarzinom, Brustkrebs, kleinzelliges Lungenkarzinom, Gliom, Colorektalkarzinom, Pankreaskarzinom, urogenital Krebs und gastrointestinal Karzinom sowie hämatologischer Krebserkrankungen, wie z.B. multiples Myelom und akut myeloische Leukämie), Psoriasis, Arthritis (z. B. rheumatoide Arthritis), Hämangioma, Angiofibroma, Augenerkrankungen (z.B. diabetische Retinopathie), neovaskulares Glaukom, Nierenerkrankungen (z.B. Glomerulonephritis), diabetische Nephropathie, maligne Nephrosklerose, thrombische mikroangiopathische Syndrome, Transplantationsabstossungen und Glomerulopathie, fibrotische Erkrankungen (z. B. Leberzirrhose), mesangialzellproliferative Erkrankungen, Artheriosklerose, Verletzungen des Nervengewebes und zur Hemmung der Reocclusion von Gefässen nach Ballonkatheterbehandlung, bei der Gefässprothetik oder nach dem Einsetzen von mechanischen Vorrichtungen zum Offenhalten von Gefässen (z.B. Stents), oder anderen Erkrankungen, bei denen Zellproliferation oder Angiogenese eine Rolle spielen.

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin, Taxol), mit Nukleinsäuren interagierenden Verbindungen (z.B. cis-Platin, Cyclophosphamid, Adriamycin), Hormon-Antagonisten (z.B. Tamoxifen), Steroiden und deren Analoga (z.B. Dexamethason), Inhibitoren metabolischer Prozesse (z.B. 5-FU etc.), Zytokinen (z.B. Interferonen), Kinase-Inhibitoren (z.B. EGFR-Kinase-Inhibitoren wie z.B. Iressa; Gleevec), allosterisch wirkenden Rezeptortyrosinkinase-Inhibitoren, Antikörpern (z.B. Herceptin), COX-2-Inhibitoren oder auch in Kombination mit Strahlentherapie etc. Diese Kombinationen können entweder simultan oder sequentiell verabreicht werden.

Nur Beispeile, welche unter Formel (I) fallen sind erfindungsgemäβ.

Die nachfolgenden Beispiele sollen die Erfindung näher erläutern:

| Beispiel | Name |
|---|---|
| 1.0 | 3-Z-[1-(4-(*N*-Methyl-*N*-methylsulfonyl-amino)-anilino)-1-(3-iod-phenyl)-methylen]-6-chlor-2-indolinon |
| 1.1 | 3-Z-[1-(4-(Dimethylamino-methyl)-anilino)-1-(3-iod-phenyl)-methylen]-6-chlor-2-indolinon |
| 1.2 | 3-Z-[1-(4-(N-(Dimethylamino-methylcarbonyl)-N-methyl-amino)-anilino)-1-(4-chlor-phenyl)-methylen]-6-chlor-2-indolinon |
| 1.3 | 3-Z-[1-(4-(N-(2-Dimethylamino-ethyl)-N-acetyl-amino)-anilino)-1-(4-chlor-phenyl)-methylen]-6-chlor-2-indolinon |
| 1.4 | 3-Z-[1-(4-(N-(4-Methyl-piperazin-1-yl-methylcarbonyl)-N-methyl-amino)-anilino)-1-(4-chlor-phenyl)-methylen]-6-chlor-2-indolinon |
| 1.5 | 3-Z-[1-(4-(N-(3-Dimethylamino-propyl)-N-acetyl-amino)-anilino)-1-(4-chlor-phenyl)-methylen]-6-chlor-2-indolinon |
| 1.6 | 3-Z-[1-(4-(Dimethylamino-methyl)-anilino)-1-(4-chlor-phenyl)-methylen]-6-chlor-2-indolinon |
| 1.7 | 3-Z-[1-(4-(N-(2-Dimethylamino-ethyl)-N-acetyl-amino)-anilino)-1-(3,4-dimethoxy-phenyl)-methylen]-6-chlor-2-indolinon |
| 1.8 | 3-Z-[1-(4-(N-(4-Methyl-piperazin-1-yl-methylcarbonyl)-N-methyl-amino)-anilino)-1-(3,4-dimethoxy-phenyl)-methylen]-6-chlor-2-indolinon |
| 1.9 | 3-Z-[1-(4-(N-(2-Dimethylamino-ethyl)-N-methylsulfonyl-amino)-anilino)-1-(3,4-dimethoxy-phenyl)-methylen]-6-chlor-2-indolinon |
| 1.10 | 3-Z-[1-(4-(Dimethylamino-methyl)-anilino)-1-(3,4-dimethoxy-phenyl)-methylen]-6-chlor-2-indolinon |
| 1.11 | 3-Z-[1-(4-(N-(2-Dimethylamino-ethyl)-N-methyl-carbamoyl)-anilino)-1-(3,4-dimethoxy-phenyl)-methylen]-6-chlor-2-indolinon |
| 2.0 | 3-Z-[1-(4-(Dimethylamino-methyl)-anilino)-1-(4-cyano-phenyl)-methylen]-6-chlor-2-indolinon |
| 3.0 | 3-Z-[1-(4-(Dimethylaminomethyl)-anilino)-1-(4-iod-phenyl)-methylen]-6-fluor-2-indolinon |
| 3.1 | 3-Z-[1-(4-(Dimethylaminomethyl)-anilino)-1-(3-fluor-phenyl)-methylen]-6-fluor-2-indolinon |
| 3.2 | 3-Z-[1-(4-(N-(3-Dimethylamino-propyl)-N-acetyl-amino)-anilino)-1-(3-fluor-phenyl)-methylen]-6-fluor-2-indolinon |
| 3.3 | 3-Z-[1-(4-(N-(4-Methyl-piperazin-1-yl-methylcarbonyl)-N-methyl-amino)-anilino)-1-(3-fluor-phenyl)-methylen]-6-fluor-2-indolinon |
| 3.4 | 3-Z-[1-(4-(Dimethylaminomethyl)-anilino)-1-(4-(2-acetylamino-ethyl)-phenyl)-methylen]-6-fiuor-2-indolinon |
| 3.5 | 3-Z-[1-(4-(N-(3-Dimethylamino-propyl)-N-acetyl-amino)-anilino)-1-(4-(2-acetylamino-ethyl)-phenyl)-methylen]-6-fluor-2-indolinon |
| 3.6 | 3-Z-[1-(4-(N-(4-Methyl-piperazin-1-yl-methylcarbonyl)-N-methyl-amino)-anilino)-1-(4-(2-acetylamino-ethyl)-phenyl)-methylen]-6-fluor-2-indolinon |
| 3.7 | 3-Z-[1-(4-(Dimethylaminomethyl)-anilino)-1-(4-methoxycarbonylmethyl-phenyl)-methylen]-6-fluor-2-indolinon |
| 3.8 | 3-Z-[1-(4-(Dimethylaminomethyl)-anilino)-1-(3-iod-phenyl)-methylen]-6-fluor-2-indolinon |
| 3.9 | 3-Z-[1-(4-(Dimethylaminomethyl)-anilino)-1-(3-methoxycarbonylmethyl-phenyl)-methylen]-6-fluor-2-indolinon |
| 3.10 | 3-Z-[1-(4-(Dimethylaminomethyl)-anilino)-1-(3-(N-tert.butoxycarbonyl-aminomethyl)-phenyl)-methylen]-6-fluor-2-indolinon |
| 3.11 | 3-Z-[1-(4-(*N*-(2-Dimethylamino-ethyl)-N-methylsulfonyl-amino)-anilino)-1-(4-methoxycarbonylmethyl-phenyl)-methylen]-6-fluor-2-indolinon |
| 3.12 | 3-Z-[1-(4-(N-(4-Methyl-piperazin-1-yl-methylcarbonyl)-N-methyl-amino)-anilino)-1-(4-methoxycarbonylmethyl-phenyl)-methylen]-6-fluor-2-indolinon |
| 3.13 | 3-Z-[1-(4-(Dimethylaminomethyl)-anilino)-1-(3-cyanomethyl-phenyl)-methylen]-6-fluor-2-indolinon |
| 3.14 | 3-Z-[1-(4-(N-(4-Methyl-piperazin-1-yl-methylcarbonyl)-N-methyl-amino)-anilino)-1-(4-(N-tert.butoxycarbonyl-aminomethyl)-phenyl)-methylen]-6-fluor-2-indolinon |
| 3.15 | 3-Z-[1-(4-(Dimethylaminomethyl)-anilino)-1-(4-(N-tert.butoxycarbonyl-aminomethyl)-phenyl)-methylen]-6-fluor-2-indolinon |
| 3.16 | 3-Z-[1-(4-(Dimethylaminomethyl)-anilino)-1-(3-(N-tert.butoxycarbonyl-2-amino-ethyl)-phenyl)-methylen]-6-fluor-2-indolinon |
| 3.17 | 3-Z-[1-(4-(N-Acetyl-N-methyl-amino)-anilino)-1-(4-(2-methoxycarbonyl-ethyl)-phenyl)-methylen]-6-fluor-2-indolinon |
| 3.18 | 3-Z-[1-(4-(N-(4-Methyl-piperazin-1-yl-methylcarbonyl)-N-methyl-amino)-anilino)-1-(4-(2-methoxycarbonyl-ethyl)-phenyl)-methylen]-6-fluor-2-indolinon |
| 3.19 | 3-Z-[1-(4-(N-(2-Dimethylamino-ethyl)-N-methylsulfonyl-amino)-anilino)-1-(4-methoxycarbonylmethyl-phenyl)-methylen]-6-fluor-2-indolinon |
| 3.20 | 3-Z-[1-(4-(N-(3-Dimethylamino-propyl)-N-acetyl-amino)-anilino)-1-(4-(2-methoxycarbonyl-ethyl)-phenyl)-methylen]-6-fluor-2-indolinon |
| 3.21 | 3-Z-[1-(4-(N-tert.Butoxycarbonyl-methylamino-methyl)-anilino)-1-(4-(2-methoxycarbonyl-ethyl)-phenyl)-methylen]-6-fluor-2-indolinon |
| 3.22 | 3-Z-[1-(4-(4-Methyl-piperazin-1-yl-carbonyl)-anili no)-1-(4-(2-methoxycarbonyl-ethyl)-phenyl)-methylen]-6-fluor-2-indolinon |
| 3.23 | 3-Z-[1-(4-(1-Methyl-imidazol-2-yl)-anilino)-1-(4-(2-methoxycarbonyl-ethyl)-phenyl)-methylen]-6-fluor-2-indolinon |
| 3.24 | 3-Z-[1-(4-Methylsulfonyl-anilino)-1-(4-(2-methoxycarbonyl-ethyl)-phenyl)-methylen]-6-fluor-2-indolinon |
| 3.25 | 3-Z-[1-(4-(N-(4-Methyl-piperazin-1-yl-methylcarbonyl)-N-methyl-amino)-anilino)-1-(3-methoxycarbonylmethyl-phenyl)-methylen]-6-fluor-2-indolinon |
| 3.26 | 3-Z-[1-(4-(N-(2-Dimethylamino-ethyl)-N-methylsulfonyl-amino)-anilino)-1-(3-methoxycarbonylmethyl-phenyl)-methylen]-6-fluor-2-indolinon |
| 3.27 | 3-Z-[1-(4-(4-Methyl-piperazin-1-yl-carbonyl)-anifino)-1-(3-methoxycarbonylmethyl-phenyl)-methylen]-6-fluor-2-indolinon |
| 3.28 | 3-Z-[1-(4-(N-(Dimethylamino-methylcarbonyl)-N-methyl-amino)-anilino)-1-(3-methoxycarbonylmethyl-phenyl)-methylen]-6-fluor-2-indolinon |
| 3.29 | 3-Z-[1-(4-(N-(3-Dimethylamino-propyl)-N-acetyl-amino)-anilino)-1-(3-methoxycarbonylmethyl-phenyl)-methylen]-6-fluor-2-indolinon |
| 3.30 | 3-Z-[1-(4-(N-(2-Dimethylamino-ethyl)-N-acetyl-amino)-anilino)-1-(3-methoxycarbonylmethyl-phenyl)-methylen]-6-fluor-2-indolinon |
| 3.31 | 3-Z-[1-(4-(N-(4-Dimethylamino-butylcarbonyl)-N-methyl-amino)-anilino)-1-(3-methoxycarbonylmethyl-phenyl)-methylen]-6-fluor-2-indolinon |
| 3.32 | 3-Z-[1-Anilino-1-(4-methoxycarbonylmethyl-phenyl)-methylen]-6-fluor-2-indolinon |
| 3.33 | 3-Z-[1-(4-(1-Methyl-imidazol-2-yl)-anilino)-1-(4-methoxycarbonylmethyl-phenyl)-methylen]-6-fluor-2-indolinon |
| 3.34 | 3-Z-[1-(4-(4-Methyl-piperazin-1-yl-carbonyl)-anilino)-1-(4-methoxycarbonylmethyl-phenyl)-methylen]-6-fluor-2-indolinon |
| 3.35 | 3-Z-[1-(4-(N-(Dimethylamino-carbonylmethyl)-N-methylsulfonyl-amino)-anilino)-1-(4-methoxycarbonylmethyl-phenyl)-methylen]-6-fluor-2-indolinon |
| 3.36 | 3-Z-[1-(4-(N-(Dimethylamino-methylcarbonyl)-N-methyl-amino)-anilino)-1-(4-methoxycarbonylmethyl-phenyl)-methylen]-6-fluor-2-indolinon |
| 3.37 | 3-Z-[1-(4-(N-Methyl-N-acetyl-amino)-anilino)-1-(4-methoxycarbonylmethyl-phenyl)-methylen]-6-fluor-2-indolinon |
| 3.38 | 3-Z-[1-(4-(N-(2-Dimethylamino-ethylcarbonyl)-N-methyl-amino)-anilino)-1-(4-methoxycarbonylmethyl-phenyl)-methylen]-6-fluor-2-indolinon |
| 3.39 | 3-Z-[1-(4-Methylsulfonyl-anilino)-1-(4-methoxycarbonylmethyl-phenyl)-methylen]-6-fluor-2-indolinon |
| 3.40 | 3-Z-[1-(4-(N-(3-Dimethylamino-propylcarbonyl)-N-methyl-amino)-anilino)-1-(4-methoxycarbonylmethyl-phenyl)-methylen]-6-fluor-2-indolinon |
| 3.41 | 3-Z-[1-(4-(N-(3-Dimethylamino-propyl)-N-acetyl-amino)-anilino)-1-(4-methoxycarbonylmethyl-phenyl)-methylen]-6-fluor-2-indolinon |
| 3.42 | 3-Z-[1-Anilino-1-(3-methoxycarbonylmethyl-phenyl)-methylen]-6-fluor-2-indolinon |
| 3.43 | 3-Z-[1-(4-Methylsulfonyl-anilino)-1-(3-methoxycarbonylmethy)-phenyl)-methylen]-6-fluor-2-indolinon |
| 3.44 | 3-Z-[1-(4-(1-Methyl-imidazol-2-yl)-anilino)-1-(3-methoxycarbonylmethyl-phenyl)-methylen]-6-fluor-2-indolinon |
| 3.45 | 3-Z-[1-(4-(N-(Dimethylamino-carbonylmethyl)-N-methylsulfonyl-amino)-anilino)-1-(3-methoxycarbony)methyl-phenyl)-methylen]-6-fluor-2-indolinon |
| 3.46 | 3-Z-[1-(4-(N-(3-Dimethylamino-propylcarbonyl)-N-methyl-amino)-anilino)-1-(3-methoxycarbonylmethyl-phenyl)-methylen]-6-fluor-2-indolinon |
| 3.47 | 3-Z-[1-(4-(N-(2-Dimethylamino-ethylcarbonyl)-N-methyl-amino)-anilino)-1-(3-methoxycarbonylmethyl-phenyl)-methylen]-6-fluor-2-indolinon |
| 3.48 | 3-Z-[1-(4-(N-(4-Methyl-piperazin-1-yl-methylcarbonyl)-N-methyl-amino)-anilino)-1-(3-(2-methoxycarbonyl-ethyl)-phenyl)-methylen]-6-fluor-2-indolinon |
| 3.49 | 3-Z-[1-(4-(N-(2-Dimethylamino-ethyl)-N-methylsulfonyl-amino)-anilino)-1-(3-(2-methoxycarbonyl-ethyl)-phenyl)-methylenJ-6-fluor-2-indolinon |
| 3.50 | 3-Z-[1-(4-(N-(Dimethylamino-methylcarbonyl)-N-methyl-amino)-anilino)-1-(3-(2-methoxycarbonyl-ethyl)-phenyl)-methylen]-6-fluor-2-indolinon |
| 3.51 | 3-Z-[1-(4-(N-(3-Dimethylamino-propyl)-N-acetyl-amino)-anilino)-1-(3-(2-methoxycarbonyl-ethyl)-phenyl)-methylen]-6-fluor-2-indolinon |
| 3.52 | 3-Z-[1-(4-(N-(4-Methyl-piperazin-1-yl-methylcarbonyl)-N-methyl-amino)-anilino)-1-(3-(N-tert.butoxycarbonyl-aminomethyl)-phenyl)-methylen]-6-fluor-2-indolinon |
| 3.53 | 3-Z-[1-(4-(N-Methyl-N-acetyl-amino)-anilino)-1-(3-acetylaminomethyl-phenyl)-methylen]-6-chlor-2-indolinon |
| 3.54 | 3-Z-[1-(4-(N-(3-Dimethylamino-propyl)-N-acetyl-amino)-anilino)-1-(3-acetylaminomethyl-phenyl)-methylen]-6-chlor-2-indolinon |
| 3.55 | 3-Z-[1-(4-(N-(2-Dimethylamino-ethyl)-N-methylsulfonyl-amino)-anilino)-1-(3-acetylaminomethyl-phenyl)-methylen]-6-chlor-2-indolinon |
| 3.56 | 3-Z-[1-(4-(N-(Dimethylamino-methylcarbonyl)-N-methyl-amino)-anilino)-1-(3-acetylaminomethyl-phenyl)-methylen]-6-chlor-2-indolinon |
| 3.57 | 3-Z-[1-(4-(2-Dimethylamino-ethyl)-anilino)-1-(4-(2-methoxycarbonyl-ethyl)-phenyl)-methylen]-6-fluor-2-indolinon |
| 3.58 | 3-Z-[1-(4-(N-(2-Dimethylamino-ethylcarbonyl)-N-methyl-amino)-anilino)-1-(4-(2-methoxycarbonyl-ethyl)-phenyl)-methylen]-6-fluor-2-indolinon |
| 3.59 | 3-Z-[1-(4-(N-(Dimethylamino-methylcarbonyl)-N-methyl-amino)-anilino)-1-(4-(2-methoxycarbonyl-ethyl)-phenyl)-methylen]-6-fluor-2-indolinon |
| 3.60 | 3-Z-[1-(4-(2-Dimethylamino-ethyl)-anilino)-1-(3-(2-ethoxycarbonyl-ethyl)-phenyl)-methylen]-6-fluor-2-indolinon |
| 3.61 | 3-Z-[1-(4-(N-(2-Dimethylamino-ethyl)-N-aceiyl-amino)-anilino)-1-(4-(2-methoxycarbonyl-ethyl)-phenyl)-methylen]-6-fluor-2-indolinon |
| 3.62 | 3-Z-[1-(4-(N-(3-Dimethylamino-propylcarbonyl)-N-methyl-amino)-anilino)-1-(3-(2-ethoxycarbonyl-ethyl)-phenyl)-methylen]-6-fluor-2-indolinon |
| 3.63 | 3-Z-[1-(4-(N-(4-Dimethylamino-butylcarbonyl)-N-methyl-amino)-anilino)-1-(3-(2-ethoxycarbonyl-ethyl)-phenyl)-methylen]-6-fluor-2-indolinon |
| 3.64 | 3-Z-[1-(4-(1-Methyl-imidazol-2-yl)-anilino)-1-(3-(2-ethoxycarbonyl-ethyl)-phenyl)-methylen]-6-fluor-2-indolinon |
| 3.65 | 3-Z-[1-(4-(N-(4-Dimethylamino-butylcarbonyl)-N-methyl-amino)-anilino)-1-(4-(2-methoxycarbonyl-ethyl)-phenyl)-methylen]-6-fluor-2-indolinon |
| 3.66 | 3-Z-[1-Anilino-1-(4-(2-methoxycarbonyl-ethyl)-phenyl)-methylen]-6-fluor-2-indolinon |
| 3.67 | 3-Z-[1-(4-(Pyrrolidin-1-yl-methyl)-anilino)-1-(4-(2-methoxycarbonyl-ethyl)-phenyl)-methylen]-6-fluor-2-indolinon |
| 3.68 | 3-Z-[1-(4-(Diethylaminomethyl)-anilino)-1-(4-(2-methoxycarbonyl-ethyl)-phenyl)-methylen]-6-fluor-2-indolinon |
| 3.69 | 3-Z-[1-(4-(N-tert.Butoxycarbonyl-amino-methyl)-anilino)-1-(4-(2-methoxycarbonyl-ethyl)-phenyl)-methylen]-6-fluor-2-indolinon |
| 3.70 | 3-Z-[1-(4-(2-Dimethylamino-ethyl)-anilino)-1-(3-methoxycarbonylmethyl-phenyl)-methylen]-6-fluor-2-indolinon |
| 3.71 | 3-Z-[1-(4-(2-Dimethylamino-ethyl)-anilino)-1-(4-methoxycarbonylmethyl-phenyl)-methylen]-6-fluor-2-indolinon |
| 3.72 | 3-Z-[1-(4-(Dimethylaminomethyl)-anilino)-1-(3-(2-ethoxycarbonyl-ethyl)-phenyl)-methylen]-6-fluor-2-indolinon |
| 3.73 | 3-Z-[1-(4-(2-Dimethylamino-ethyl)-anilino)-1-(4-(2-methoxycarbonyl-ethyl)-phenyl)-methylen]-6-chlor-2-indolinon |
| 3.74 | 3-Z-[1-(4-(1-Methyl-imidazof-2-yl)-anilino)-1-(4-(2-ethoxycarbonyl-ethyl)-phenyl)-methylen]-6-chlor-2-indolinon |
| 3.75 | 3-Z-[1-(4-Dimethylaminomethyl-anilino)-1-(4-(2-methoxycarbonyl-ethyl)-phenyl)-methylen]-6-chlor-2-indolinon |
| 3.76 | 3-Z-[1-(4-Dimethylaminomethyl-anilino)-1-(4-(2-ethoxycarbonyl-ethyl)-phenyl)-methylen]-6-fluor-2-indolinon |
| 3.77 | 3-Z-[1-(4-((4-Methyl-piperazin-1-yl)-methyl)-anilino)-1-(3-(2-methoxycarbonyl-ethyl)-phenyl)-methylen]-6-fluor-2-indolinon |
| 3.78 | 3-Z-[1-(4-(Imidazol-1-yl-methyl)-anilino)-1-(3-(2-methoxycarbonyl-ethyl)-phenyl)-methylen]-6-fluor-2-indolinon |
| 3.79 | 3-Z-[1-(4-((4-Methyl-piperazin-1-yl)-methyl)-anilino)-1-(4-(2-methoxycarbonyl-ethyl)-phenyl)-methylen]-6-fluor-2-indolinon |
| 3.80 | 3-Z-[1-(4-(Imidazol-1-yl-methyl)-anilino)-1-(4-(2-methoxycarbonyl-ethyl)-phenyl)-methylen]-6-fluor-2-indolinon |
| 3.81 | 3-Z-[1-(4-(N-(2-Dimethylamino-ethyl)-N-methyl-aminomethyl)-anilino)-1-(4-(2-methoxycarbonyl-ethyl)-phenyl)-methylen]-6-fluor-2-indolinon |
| 3.82 | 3-Z-[1-(4-(N-(2-Dimethylamino-ethyl)-N-methyl-aminomethyl)-anilino)-1-(3-(2-ethoxycarbonyl-ethyl)-phenyl)-methylen]-6-fluor-2-indolinon |
| 3.83 | 3-Z-[1-(4-(Pyrrolidin-1-yl-methyl)-anilino)-1-(4-(2-methoxycarbonyl-ethyl)-phenyl)-methylen]-6-chlor-2-indolinon |
| 3.84 | 3-Z-[1-Anilino-1-(3-(2-ethoxycarbonyl-ethyl)-phenyl)-methylen]-6-fluor-2-indolinon |
| 3.85 | 3-Z-[1-(4-(N-tert.Butoxycarbonyl-amino-methyl)-anilino)-1-(3-(2-ethoxycarbonyl-ethyl)-phenyl)-methylen]-6-fluor-2-indolinon |
| 3.86 | 3-Z-[1-(4-(N-tert.Butoxycarbonyl-methylamino-methyl)-anilino)-1-(3-(2-ethoxycarbonyl-ethyl)-phenyl)-methylen]-6-fluor-2-indolinon |
| 3.87 | 3-Z-[1-(4-Dimethylaminomethyl-anilino)-1-(3-methoxycarbonylmethoxy-phenyl)-methylen]-6-fluor-2-indolinon |
| 3.88 | 3-Z-[1-(4-Dimethylaminomethyl-anilino)-1-(4-methoxycarbonylmethoxy-phenyl)-methylen]-6-fluor-2-indolinon |
| 3.89 | 3-Z-[1-(4-Dimethylaminomethyl-anilino)-1-(3-(2-ethoxycarbonyl-ethoxy)-phenyl)-methylen]-6-fluor-2-indolinon |
| 3.90 | 3-Z-[1-(4-(Pyrrolidin-1-yl-methyl)-anilino)-1-(4-(2-methoxycarbonyl-ethyl)-phenyl)-methylen]-6-brom-2-indolinon |
| 3.91 | 3-Z-[1-(4-(Dimethylaminomethyl)-anilino)-1-(4-(2-methoxycarbonyl-ethyl)-phenyl)-methylen]-6-brom-2-indolinon |
| 3.92 | 3-Z-[1-(4-(Diethylaminomethyl)-anilino)-1-(4-(2-methoxycarbonyl-ethyl)-phenyl)-methylen]-6-brom-2-indolinon |
| 3.93 | 3-Z-[1-(3-Dimethylaminomethyl-anilino)-1-(4-(2-methoxycarbonyl-ethyl)-phenyl)-methylen]-6-fluor-2-indolinon |
| 3.94 | 3-Z-[1-(3-Dimethylaminomethyl-anilino)-1-(3-(2-ethoxycarbonyl-ethyl)-phenyl)-methylen]-6-fluor-2-indolinon |
| 3.95 | 3-Z-[1-(3-Dimethylaminomethyl-anilino)-1-(4-(2-methoxycarbonyl-ethyl)-phenyl)-methylen]-6-chlor-2-indolinon |
| 4.0 | 3-Z-[1-(4-(Dimethylaminomethyl)-anilino)-1-(3,4-dimethoxy-phenyl)-methylen]-6-cyano-2-indolinon |
| 5.0 | 3-Z-[1-(4-(*N*-Methyl-*N*-methylsulfonyl-amino)-anilino)-1-(3-(2-methoxycarbonyl-vinyl)-phenyl)-methylen]-6-chlor-2-indolinon |
| 5.1 | 3-Z-[1-(4-(Dimethylaminomethyl)-anilino)-1-(4-(2-methoxycarbonyl-vinyl)-phenyl)-methylen]-6-chlor-2-indolinon |
| 5.2 | 3-Z-[1-(4-(Dimethylaminomethyl)-anilino)-1-(4-(2-carbamoyl-vinyl)-phenyl)-methylen]-6-fluor-2-indolinon |
| 5.3 | 3-Z-[1-(4-(Dimethylaminomethyl)-anilino)-1-(4-(2-methoxycarbonyl-vinyl)-phenyl)-methylen]-6-fluor-2-indolinon |
| 5.4 | 3-Z-[1-(4-(Dimethylaminomethyl)-anilino)-1-(3-(2-methoxycarbonyl-vinyl)-phenyl)-methylen]-6-fluor-2-indolinon |
| 6.0 | 3-Z-[1-(4-Dimethylaminomethyl-anilino)-1-(3-(2-methoxycarbonyl-ethyl)-phenyl)-methylen]-6-chlor-2-indolinon |
| 6.1 | 3-Z-[1-(4-(N-Methyl-N-methylsulfonyl-amino)-anilino)-1-(3-(2-methoxycarbonyl-ethyl)-phenyl)-methylen]-6-chlor-2-indolinon |
| 6.2 | 3-Z-[1-(4-Dimethylaminomethyl-anilino)-1-(4-(2-carbamoyl-ethyl)-phenyl)-methylen]-6-fluor-2-indolinon |
| 6.3 | 3-Z-[1-(4-Dimethylaminomethyl-anilino)-1-(4-(2-methoxycarbonyl-ethyl)-phenyl)-methylen]-6-fluor-2-indolinon |
| 6.4 | 3-Z-[1-(4-Dimethylaminomethyl-anil ino)-1-(3-(2-methoxycarbonyl-ethyl)-phenyl)-methylen]-6-fluor-2-indolinon |
| 7.0 | 3-Z-[1-(4-Dimethylaminomethyl-anilino)-1-(4-aminomethyl-phenyl)-methylen]-6-chlor-2-indolinon |
| 8.0 | 3-Z-[1-(4-(N-((4-Methyl-piperazin-1-yl)-methylcarbonyl)-N-methyl-amino)-anilino)-1-(4-aminomethyl-phenyl)-methylen]-6-chlor-2-indolinon |
| 9.0 | 3-Z-[1-(4-(Dimethylaminomethyl)-anilino)-1-(3-aminomethyl-phenyl)-methylen]-6-fluor-2-indolinon |
| 9.1 | 3-Z-[1-(4-(Dimethylaminomethyl)-anilino)-1-(3-(2-amino-ethyl)-phenyl)-methylen]-6-fluor-2-indolinon |
| 9.2 | 3-Z-[1-(4-(Dimethylaminomethyl)-anilino)-1-(4-aminomethyl-phenyl)-methylen]-6-fluor-2-indolinon |
| 9.3 | 3-Z-[1-(4-(N-(4-Methyl-piperazin-1-yl-methylcarbonyl)-N-methyl-amino)-anilino)-1-(4-aminomethyl-phenyl)-methylen]-6-fluor-2-indolinon |
| 9.4 | 3-Z-[1-(4-(Methylamino-methyl)-anilino)-1-(4-(2-carboxy-ethyl)-phenyl)-methylen]-6-fluor-2-indolinon |
| 9.5 | 3-Z-[1-(4-(Methylamino-methyl)-anilino)-1-(4-(2-methylcarbamoyl-ethyl)-phenyl)-methylen]-6-fluor-2-indolinon |
| 9.6 | 3-Z-[1-(4-(N-(4-Methyl-piperazin-1-yl-methylcarbonyl)-N-methyl-amino)-anilino)-1-(3-aminomethyl-phenyl)-methylen]-6-fluor-2-indolinon |
| 9.7 | 3-Z-[1-(4-(Amino-methyl)-anilino)-1-(4-(2-methoxycarbonyl-ethyl)-phenyl)-methylen]-6-fluor-2-indolinon |
| 9.8 | 3-Z-[1-(4-(Amino-methyl)-anilino)-1-(3-(2-ethoxycarbonyl-ethyl)-phenyl)-methylen]-6-fluor-2-indolinon |
| 9.9 | 3-Z-[1-(4-(Methylamino-methyl)-anilino)-1-(3-(2-ethoxycarbonyl-ethyl)-phenyl)-methylen]-6-fluor-2-indolinon |
| 10.0 | 3-Z-[1-(4-Dimethylaminomethyl-anilino)-1-(3-(2-carboxy-ethyl)-phenyl)-methylen]-6-chlor-2-indolinon |
| 10.1 | 3-Z-[1-(4-Dimethylaminomethyl-anilino)-1-(4-(2-carboxy-ethyl)-phenyl)-methylen]-6-fluor-2-indolinon |
| 10.2 | 3-Z-[1-(4-Dimethylaminomethyl-anilino)-1-(3-carboxymethyl-phenyl)-methylen]-6-fluor-2-indolinon |
| 10.3 | 3-Z-[1-(4-Dimethylaminomethyl-anilino)-1-(3-(2-carboxy-ethyl)-phenyl)-methylen]-6-fluor-2-indolinon |
| 10.4 | 3-Z-[1-(4-Dimethylaminomethyl-anilino)-1-(4-carboxymethyl-phenyl)-methylen]-6-fluor-2-indolinon |
| 10.5 | 3-Z-[1-(4-(N-(4-Methyl-piperazin-1-yl-methylcarbonyl)-N-methyl-amino)-anilino)-1-(4-carboxymethyl-phenyl)-methylen]-6-fluor-2-indolinon |
| 10.6 | 3-Z-[1-(4-(N-(2-Dimethylamino-ethyl)-N-methylsulfonyl-amino)-anilino)-1-(4-carboxymethyl-phenyl)-methylen]-6-fluor-2-indolinon |
| 10.7 | 3-Z-[1-(4-(N-Methyl-N-acetyl-amino)-anilino)-1-(4-(2-carboxy-ethyl)-phenyl)-methylen]-6-fluor-2-indolinon |
| 10.8 | 3-Z-[1-(4-(N-(4-Methyl-piperazin-1-yl-methylcarbonyl)-N-methyl-amino)-anilino)-1-(4-(2-carboxy-ethyl)-phenyl)-methylen]-6-fluor-2-indolinon |
| 10.9 | 3-Z-[1-(4-(N-(2-Dimethylamino-ethyl)-N-methylsulfonyl-amino)-anilino)-1-(4-(2-carboxy-ethyl)-phenyl)-methylen]-6-fluor-2-indolinon |
| 10.10 | 3-Z-[1-(4-(N-(3-Dimethylamino-propyl)-N-acetyl-amino)-anilino)-1-(4-(2-carboxy-ethyl)-phenyl)-methylen]-6-fluor-2-indolinon |
| 10.11 | 3-Z-[1-(4-(N-tert.Butoxycarbonyl-methylamino-methyl)-anilino)-1-(4-(2-carboxy-ethyl)-phenyl)-methylen]-6-fluor-2-indolinon |
| 10.12 | 3-Z-[1-(4-(4-Methyl-piperazin-1-yl-carbonyl)-anilino)-1-(4-(2-carboxy-ethyl)-phenyl)-methylen]-6-fluor-2-indolinon |
| 10.13 | 3-Z-[1-(4-(1-Methyl-imidazol-2-yl)-anilino)-1-(4-(2-carboxy-ethyl)-phenyl)-methylen]-6-fluor-2-indolinon |
| 10.14 | 3-Z-[1-(4-Methylsulfonyl-anilino)-1-(4-(2-carboxy-ethyl)-phenyl)-methylen]-6-fluor-2-indolinon |
| 10.15 | 3-Z-[1-(4-(4-Methyl-piperazin-1-yl-carbonyl)-anilino)-1-(3-carboxymethyl-phenyl)-methylen]-6-fluor-2-indolinon |
| 10.16 | 3-Z-[1-(4-(N-(4-Methyl-piperazin-1-yl-methylcarbonyl)-N-methyl-amino)-anilino)-1-(3-carboxymethyl-phenyl)-methylen]-6-fluor-2-indolinon |
| 10.17 | 3-Z-[1-(4-(N-(Dimethylamino-methylcarbonyl)-N-methyl-amino)-anilino)-1-(3-carboxymethyl-phenyl)-methylen]-6-fluor-2-indolinon |
| 10.18 | 3-Z-[1-(4-(N-(4-Dimethylamino-butylcarbonyl)-N-methyl-amino)-anilino)-1-(3-carboxymethyl-phenyl)-methylen]-6-fluor-2-indolinon |
| 10.19 | 3-Z-[1-Anilino-1-(3-carboxymethyl-phenyl)-methylen]-6-fluor-2-indolinon |
| 10.20 | 3-Z-[1-(4-Methylsulfonyl-anili no)-1-(3-carboxymethyl-phenyl)-methylen]-6-fluor-2-indolinon |
| 10.21 | 3-Z-[1-(4-(1-Methyl-imidazol-2-yl)-anilino)-1-(3-carboxymethyl-phenyl)-methylen]-6-fluor-2-indolinon |
| 10.22 | 3-Z-[1-(4-(N-(Dimethylamino-carbonylmethyl)-N-methylsulfonyl-amino)-anilino)-1-(3-carboxymethyl-phenyl)-methylen]-6-fluor-2-indolinon |
| 10.23 | 3-Z-[1-Anilino-1-(4-carboxymethyl-phenyl)-methylen]-6-fluor-2-indolinon |
| 10.24 | 3-Z-[1-(4-(1-Methyl-imidazol-2-yl)-anilino)-1-(4-carboxymethyl-phenyl)-methylen]-6-fluor-2-indolinon |
| 10.25 | 3-Z-[1-(4-(N-(3-Dimethylamino-propylcarbonyl)-N-methyl-amino)-anilino)-1-(4-carboxymethyl-phenyl)-methylen]-6-fluor-2-indolinon |
| 10.26 | 3-Z-[1-(4-(N-(Dimethylamino-methylcarbonyl)-N-methyl-amino)-anilino)-1-(4-carboxymethyl-phenyl)-methylen]-6-fluor-2-indolinon |
| 10.27 | 3-Z-[1-(4-(4-Methyl-piperazin-1-yl-carbonyl)-anilino)-1-(4-carboxymethyl-phenyl)-methylen]-6-fluor-2-indolinon |
| 10.28 | 3-Z-[1-(4-Methylsulfonyl-anilino)-1-(4-carboxymethyl-phenyl)-methylen]-6-fluor-2-indolinon |
| 10.29 | 3-Z-[1-(4-(N-Methyl-N-acetyl-amino)-anilino)-1-(4-carboxymethyl-phenyl)-methylen]-6-fluor-2-indolinon |
| 10.30 | 3-Z-[1-(4-(N-(Dimethylamino-carbonylmethyl)-N-methylsulfonyl-amino)-anilino)-1-(4-carboxymethyl-phenyl)-methylen]-6-fluor-2-indolinon |
| 10.31 | 3-Z-[1-(4-(N-(2-Dimethylamino-ethylcarbonyl)-N-methyl-amino)-anilino)-1-(4-carboxymethyl-phenyl)-methylen]-6-fluor-2-indolinon |
| 10.32 | 3-Z-[1-(4-(N-(3-Dimethylamino-propylcarbonyl)-N-methyl-amino)-anilino)-1-(4-carboxymethyl-phenyl)-methylen]-6-fluor-2-indolinon |
| 10.33 | 3-Z-[1-(4-(N-(4-Methyl-piperazin-1-yl-methylcarbonyl)-N-methyl-amino)-anilino)-1-(3-(2-carboxy-ethyl)-phenyl)-methylen]-6-fluor-2-indolinon |
| 10.34 | 3-Z-[1-(4-(N-(2-Dimethylamino-ethyl)-N-methylsulfonyl-amino)-anilino)-1-(3-carboxymethyl-phenyl)-methylen]-6-fluor-2-indolinon |
| 10.35 | 3-Z-[1-(4-(N-(2-Dimethylamino-ethyl)-N-methylsulfonyl-amino)-anilino)-1-(3-(2-carboxy-ethyl)-phenyl)-methylen]-6-fluor-2-indolinon |
| 10.36 | 3-Z-[1-(4-(N-(Dimethylamino-methylcarbonyl)-N-methyl-amino)-anilino)-1-(3-(2-carboxy-ethyl)-phenyl)-methylen]-6-fluor-2-indolinon |
| 10.37 | 3-Z-[1-(4-(N-(3-Dimethylamino-propyl)-N-acetyl-ami no)-anilino)-1-(3-(2-carboxy-ethyl)-phenyl)-methylen]-6-fluor-2-indolinon |
| 10.38 | 3-Z-[1-(4-(N-(Dimethylamino-methylcarbonyl)-N-methyl-amino)-anilino)-1-(4-(2-carboxy-ethyl)-phenyl)-methylen]-6-fluor-2-indolinon |
| 10.39 | 3-Z-[1-(4-(2-Dimethylamino-ethyl)-anilino)-1-(4-(2-carboxy-ethyl)-phenyl)-methylen]-6-fluor-2-indolinon |
| 10.40 | 3-Z-[1-(4-(N-(2-Dimethylamino-ethylcarbonyl)-N-methyl-amino)-anilino)-1-(4-(2-carboxy-ethyl)-phenyl)-methylen]-6-fluor-2-indolinon |
| 10.41 | 3-Z-[1-(4-(2-Dimethylamino-ethyl)-anilino)-1-(3-(2-carboxy-ethyl)-phenyl)-methylen]-6-fluor-2-indolinon |
| 10.42 | 3-Z-[1-(4-(N-(2-Dimethylamino-ethyl)-N-acetyl-amino)-anilino)-1-(4-(2-carboxy-ethyl)-phenyl)-methylen]-6-fluor-2-indolinon |
| 10.43 | 3-Z-[1-(4-(N-(3-Dimethylamino-propylcarbonyl)-N-methyl-amino)-anilino)-1-(3-(2-carboxy-ethyl)-phenyl)-methylen]-6-fluor-2-indolinon |
| 10.44 | 3-Z-[1-(4-(N-(4-Dimethylamino-butylcarbonyl)-N-methyl-amino)-anilino)-1-(3-(2-carboxy-ethyl)-phenyl)-methylen]-6-fluor-2-indolinon |
| 10.45 | 3-Z-[1-(4-(1-Methyl-imidazol-2-yl)-anilino)-1-(3-(2-carboxy-ethyl)-phenyl)-methylen]-6-fluor-2-indolinon |
| 10.46 | 3-Z-[1-(4-(N-(4-Dimethylamino-butylcarbonyl)-N-methyl-amino)-anilino)-1-(4-(2-carboxy-ethyl)-phenyl)-methylen]-6-fluor-2-indolinon |
| 10.47 | 3-Z-[1-Anilino-1-(4-(2-carboxy-ethyl)-phenyl)-methylen]-6-fluor-2-indolinon |
| 10.48 | 3-Z-[1-(4-(Pyrrolidin-1-yl-methyl)-anilino)-1-(4-(2-carboxy-ethyl)-phenyl)-methylen]-6-fluor-2-indolinon |
| 10.49 | 3-Z-[1-(4-(Diethylaminomethyl)-anilino)-1-(4-(2-carboxy-ethyl)-phenyl)-methylen]-6-fluor-2-indolinon |
| 10.50 | 3-Z-[1-(4-Aminomethyl-anilino)-1-(4-(2-carboxy-ethyl)-phenyl)-methylen]-6-fluor-2-indolinon |
| 10.51 | 3-Z-[1-(4-(2-Dimethylamino-ethyl)-anilino)-1-(3-carboxymethyl-phenyl)-methylen]-6-fluor-2-indolinon |
| 10.52 | 3-Z-[1-(4-(2-Dimethylamino-ethyl)-anilino)-1-(4-carboxymethyl-phenyl)-methylen]-6-fluor-2-indolinon |
| 10.53 | 3-Z-[1-(4-(2-Dimethylamino-ethyl)-anilino)-1-(4-(2-carboxy-ethyl)-phenyl)-methylen]-6-chlor-2-indolinon |
| 10.54 | 3-Z-[1-(4-(1-Methyl-imidazol-2-yl)-anilino)-1-(4-(2-carboxy-ethyl)-phenyl)-methylen]-6-chlor-2-indolinon |
| 10.55 | 3-Z-[1-(4-Dimethylaminomethyl-anilino)-1-(4-(2-carboxy-ethyl)-phenyl)-methylen]-6-chlor-2-indolinon |
| 10.56 | 3-2-[1-(4-((4-Methyl-piperazin-1-yl)-methyl)-anilino)-1-(3-(2-carboxy-ethyl)-phenyl)-methylen]-6-fluor-2-indolinon |
| 10.57 | 3-Z-[1-(4-(Imidazol-1-yl-methyl)-anilino)-1-(3-(2-carboxy-ethyl)-phenyl)-methylen]-6-fluor-2-indolinon |
| 10.58 | 3-Z-[1-(4-(N-(2-Dimethylamino-ethyl)-N-methyl-aminomethyl)-anilino)-1-(3-(2-carboxy-ethyl)-phenyl)-methylen]-6-fluor-2-indolinon |
| 10.59 | 3-Z-[1-(4-((4-Methyl-piperazin-1-yl)-methyl)-anilino)-1-(4-(2-carboxy-ethyl)-phenyl)-methylen]-6-fluor-2-indolinon |
| 10.60 | 3-Z-[1-(4-(Imidazol-1-yl-methyl)-anilino)-1-(4-(2-carboxy-ethyl)-phenyl)-methylen]-6-fluor-2-indolinon |
| 10.61 | 3-Z-[1-(4-(Pyrrolidin-1-yl-methyl)-anilino)-1-(4-(2-carboxy-ethyl)-phenyl)-methylen]-6-chlor-2-indolinon |
| 10.62 | 3-Z-[1-(4-(N-(2-Dimethylamino-ethyl)-N-methyl-aminomethyl)-anilino)-1-(4-(2-carboxy-ethyl)-phenyl)-methylen]-6-fluor-2-indolinon |
| 10.63 | 3-Z-[1-Anilino-1-(3-(2-carboxy-ethyl)-phenyl)-methylen]-6-fluor-2-indolinon |
| 10.64 | 3-Z-[1-(4-Aminomethyl-anilino)-1-(3-(2-carboxy-ethyl)-phenyl)-methylen]-6-fluor-2-indolinon |
| 10.65 | 3-Z-[1-(4-Methylaminomethyl-anilino)-1-(3-(2-carboxy-ethyl)-phenyl)-methylen]-6-fluor-2-indolinon |
| 10.66 | 3-Z-[1-(4-Dimethylaminomethyl-anilino)-1-(3-carboxymethoxy-phenyl)-methylen]-6-fluor-2-indolinon |
| 10.67 | 3-Z-[1-(4-Dimethylaminomethyl-anilino)-1-(4-carboxymethoxy-phenyl)-phenyl)-methylen]-6-fluor-2-indolinon |
| 10.68 | 3-Z-[1-(4-(Pyrrolidin-1-yl-methyl)-anilino)-1-(4-(2-carboxy-ethyl)-phenyl)-methylen]-6-brom-2-indolinon |
| 10.69 | 3-Z-[1-(4-(Dimethylaminomethyl)-anilino)-1-(4-(2-carboxy-ethyl)-phenyl)-methylen]-6-brom-2-indolinon |
| 10.70 | 3-Z-[1-(4-(Diethylaminomethyl)-anilino)-1-(4-(2-carboxy-ethyl)-methylen]-6-brom-2-indolinon |
| 10.71 | 3-Z-[1-(3-Dimethylaminomethyl-anilino)-1-(4-(2-carboxy-ethyl)-phenyl)-methylen]-6-fluor-2-indolinon |
| 10.72 | 3-Z-[1-(3-Dimethylaminomethyl-anilino)-1-(3-(2-carboxy-ethyl)-phenyl)-methylen]-6-fluor-2-indolinon |
| 10.73 | 3-Z-[1-(3-Dimethylaminomethyl-anilino)-1-(4-(2-carboxy-ethyl)-phenyl)-methylen]-6-chlor-2-indolinon |
| 11.0 | 3-Z-[1-(4-Dimethylaminomethyl-anilino)-1-(3-(2-carbamoyl-ethyl)-phenyl)-methylen]-6-chlor-2-indolinon |
| 11.1 | 3-Z-[1-(4-Dimethylaminomethyl-anilino)-1-(3-(2-methylcarbamoyl-ethyl)-phenyl)-methylen]-6-chlor-2-indolinon |
| 11.2 | 3-Z-[1-(4-Dimethylaminomethyl-anilino)-1-(4-(2-methylcarbamoyl-ethyl)-phenyl)-methylen]-6-fluor-2-indolinon |
| 11.3 | 3-Z-[1-(4-Dimethylaminomethyl-anilino)-1-(3-dimethylcarbamoylmethyl-phenyl)-methylen]-6-fluor-2-indolinon |
| 11.4 | 3-Z-[1-(4-Dimethylaminomethyl-anilino)-1-(3-(2-carbamoyl-ethyl)-phenyl)-methylen]-6-fluor-2-indolinon |
| 11.5 | 3-Z-[1-(4-Dimethylaminomethyl-anilino)-1-(3-(2-methylcarbamoyl-ethyl)-phenyl)-methylen]-6-fluor-2-indolinon |
| 11.6 | 3-Z-[1-(4-Dimethylaminomethyl-anilino)-1-(3-(2-dimethylcarbamoyl-ethyl)-phenyl)-methylen]-6-fluor-2-indolinon |
| 11.7 | 3-Z-[1-(4-Dimethylaminomethyl-anilino)-1-(3-carbamoylmethyl-phenyl)-methylen]-6-fluor-2-indolinon |
| 11.8 | 3-Z-[1-(4-Dimethylaminomethyl-anilino)-1-(3-methylcarbamoylmethyl-phenyl)-methylen]-6-fluor-2-indolinon |
| 11.9 | 3-Z-[1-(4-Dimethylaminomethyl-anilino)-1-(4-carbamoylmethyl-phenyl)-methylen]-6-fluor-2-indolinon |
| 11.10 | 3-2-[1-(4-Dimethylaminomethyl-anilino)-1-(4-(2-dimethylcarbamoyl-ethyl)-phenyl)-methylen]-6-fluor-2-indolinon |
| 11.11 | 3-Z-[1-(4-Dimethylaminomethyl-anilino)-1-(4-(2-(4-methyl-piperazin-1-yl-carbonyl)-ethyl)-phenyl)-methylen]-6-fluor-2-indolinon |
| 11.12 | 3-Z-[1-(4-(N-(4-Methyl-piperazin-1-yl-methylcarbonyl)-N-methyl-amino)-anilino)-1-(4-carbamoylmethyl-phenyl)-methylen]-6-fluor-2-indolinon |
| 11.13 | 3-Z-[1-(4-(N-(2-Dimethylamino-ethyl)-N-methylsulfonyl-amino)-anilino)-1-(4-carbamoylmethyl-phenyl)-methylen]-6-fluor-2-indolinon |
| 11.14 | 3-Z-[1-(4-Dimethylaminomethyl-anilino)-1-(4-dimethylcarbamoylmethyl-phenyl)-methylen]-6-fluor-2-indolinon |
| 11.15 | 3-Z-[1-(4-Dimethylaminomethyl-anilino)-1-(4-methylcarbamoylmethyl-phenyl)-methylen]-6-fluor-2-indolinon |
| 11.16 | 3-Z-[1-(4-(N-(2-Dimethylamino-ethyl)-N-methylsulfonyl-amino)-anilino)-1-(4-methylcarbamoylmethyl-phenyl)-methylen]-6-fluor-2-indolinon |
| 11.17 | 3-Z-[1-(4-(N-(2-Dimethylamino-ethyl)-N-methylsulfonyl-amino)-anilino)-1-(4-dimethylcarbamoylmethyl-phenyl)-methylen]-6-fluor-2-indolinon |
| 11.18 | 3-Z-[1-(4-(N-(4-Methyl-piperazin-1-yl-methylcarbonyl)-N-methyl-amino)-anilino)-1-(4-methylcarbamoylmethyl-phenyl)-methylen]-6-fluor-2-indolinon |
| 11.19 | 3-Z-[1-(4-(N-Methyl-N-acetyl-amino)-anilino)-1-(4-(2-methylcarbamoyl-ethyl)-phenyl)-methylen]-6-fluor-2-indolinon |
| 11.20 | 3-Z-[1-(4-(N-(4-Methyl-piperazin-1-yl-methylcarbonyl)-N-methyl-amino)-anilino)-1-(4-(2-methylcarbamoyl-ethyl)-phenyl)-methylen]-6-fluor-2-indolinon |
| 11.21 | 3-Z-[1-(4-(N-(2-Dimethylamino-ethyl)-N-methylsulfonyl-amino)-anilino)-1-(4-(2-methylcarbamoyl-ethyl)-phenyl)-methylen]-6-fluor-2-indolinon |
| 11.22 | 3-Z-[1-(4-(N-tert.Butoxycarbonyl-methylamino-methyl)-anilino)-1-(4-(2-methylcarbamoyl-ethyl)-phenyl)-methylen]-6-fluor-2-indolinon |
| 11.23 | 3-Z-[1-(4-(1-Methyl-imidazol-2-yl)-anilino)-1-(4-(2-methylcarbamoyl-ethyl)-phenyl)-methylen]-6-fluor-2-indolinon |
| 11.24 | 3-Z-[1-(4-Methylsulfonyl-anilino)-1-(4-(2-methylcarbamoyl-ethyl)-phenyl)-methylen]-6-fluor-2-indolinon |
| 11.25 | 3-Z-[1-(4-(4-Methyl-piperazin-1-yl-carbonyl)-anilino)-1-(4-(2-methylcarbamoyl-ethyl)-phenyl)-methylen]-6-fluor-2-indolinon |
| 11.26 | 3-Z-[1-(4-(4-Methyl-piperazin-1-yl-carbonyl)-anilino)-1-(3-methylcarbamoylmethyl-phenyl)-methylen]-6-fluor-2-indolinon |
| 11.27 | 3-Z-[1-(4-(N-(4-Methyl-piperazin-1-yl-methylcarbonyl)-N-methyl-amino)-anilino)-1-(3-methylcarbamoylmethyl-phenyl)-methylen]-6-fluor-2-indolinon |
| 12.0 | 3-Z-[1-(4-Dimethylaminomethyl-anilino)-1-(4-acetylaminomethyl-phenyl)-methylen]-6-chlor-2-indolinon |
| 12.1 | 3-Z-[1-(4-(N-(4-Methyl-piperazin-1-yl-methylcarbonyl)-N-methyl-amino)-anilino)-1-(4-acetylaminomethyl-phenyl)-methylen]-6-chlor-2-indolinon |
| 12.2 | 3-Z-[1-(4-Dimethylaminomethyl-anilino)-1-(4-benzoylamino-phenyl)-methylen]-6-chlor-2-indolinon |
| 12.3 | 3-Z-[1-(4-(N-(4-Methyl-piperazin-1-yl-methylcarbonyl)-N-methyl-amino)-anilino)-1-(4-benzoylaminomethyl-phenyl)-methylen]-6-chlor-2-indolinon |
| 12.4 | 3-Z-[1-(4-Dimethylaminomethyl-anilino)-1-(3-acetylaminomethyl-phenyl)-methylen]-6-fluor-2-indolinon |
| 12.5 | 3-Z-[1-(4-Dimethylaminomethyl-anilino)-1-(3-propionylaminomethyl-phenyl)-methylen]-6-fluor-2-indolinon |
| 12.6 | 3-Z-[1-(4-Dimethylaminomethyl-anilino)-1-(3-benzoylaminomethyl-phenyl)-methylen]-6-fluor-2-indolinon |
| 12.7 | 3-Z-[1-(4-Dimethylaminomethyl-anilino)-1-(3-phenylacetylaminomethyl-phenyl)-methylen]-6-fluor-2-indolinon |
| 12.8 | 3-Z-[1-(4-Dimethylaminomethyl-anilino)-1-(3-(2-acetylamino-ethyl)-phenyl)-methylen]-6-fluor-2-indolinon |
| 12.9 | 3-Z-[1-(4-Dimethylaminomethyl-anilino)-1-(3-(2-benzoylamino-ethyl)-phenyl)-methylen]-6-fluor-2-indolinon |
| 12.10 | 3-Z-[1-(4-Dimethylaminomethyl-anilino)-1-(3-(2-propionylamino-ethyl)-phenyl)-methylen]-6-fluor-2-indolinon |
| 12.11 | 3-Z-[1-(4-Dimethylaminomethyl-anilino)-1-(3-(2-phenylacetylamino-ethyl)-phenyl)-methylen]-6-fluor-2-indolinon |
| 12.12 | 3-Z-[1-(4-Dimethylaminomethyl-anilino)-1-(4-acetylaminomethyl-phenyl)-methylen]-6-fluor-2-indolinon |
| 12.13 | 3-Z-[1-(4-Dimethylaminomethyl-anilino)-1-(4-propionylaminomethyl-phenyl)-methylen]-6-fluor-2-indolinon |
| 12.14 | 3-Z-[1-(4-Dimethylaminomethyl-anilino)-1-(4-phenylacetylaminomethyl-phenyl)-methylen]-6-fluor-2-indolinon |
| 12.15 | 3-Z-[1-(4-(N-(4-Methyl-piperazin-1-yl-methylcarbonyl)-N-methyl-amino)-anilino)-1-(4-acetylaminomethyl-phenyl)-methylen]-6-fluor-2-indolinon |
| 12.16 | 3-Z-[1-(4-(N-(4-Methyl-piperazin-1-yl-methylcarbonyl)-N-methyl-amino)-anilino)-1-(4-propionylaminomethyl-phenyl)-methylen]-6-fluor-2-indolinon |
| 12.17 | 3-Z-[1-(4-(N-(4-Methyl-piperazin-1-yl-methylcarbonyl)-N-methyl-amino)-anilino)-1-(4-phenylacetylaminomethyl-phenyl)-methylen]-6-fluor-2-indolinon |
| 12.18 | 3-Z-[1-(4-(N-(4-Methyl-piperazin-1-yl-methylcarbonyl)-N-methyl-amino)-anilino)-1-(3-cyclopropylcarbonylaminomethyl-phenyl)-methylen]-6-fluor-2-indolinon |
| 12.19 | 3-Z-[1-(4-(N-(4-Methyl-piperazin-1-yl-methylcarbonyl)-N-methyl-amino)-anilino)-1-(3-cyclobutylcarbonylaminomethyl-phenyl)-methylen]-6-fluor-2-indolinon |
| 12.20 | 3-Z-[1-(4-(N-(4-Methyl-piperazin-1-yl-methylcarbonyl)-N-methyl-amino)-anilino)-1-(3-(pyridin-2-yl-carbonylaminomethyl)-phenyl)-methylen]-6-fluor-2-indolinon |
| 12.21 | 3-Z-[1-(4-(N-(4-Methyl-piperazin-1-yl-methylcarbonyl)-N-methyl-amino)-anilino)-1-(3-cyclohexylcarbonylaminomethyl-phenyl)-methylen]-6-fluor-2-indolinon |
| 12.22 | 3-Z-[1-(4-(N-(4-Methyl-piperazin-1-yl-methylcarbonyl)-N-methyl-amino)-anilino)-1-(3-(pyridin-3-yl-carbonylaminomethyl)-phenyl)-methylen]-6-fluor-2-indolinon |
| 12.23 | 3-Z-[1-(4-(N-(4-Methyl-piperazin-1-yl-methylcarbonyl)-N-methyl-amino)-anilino)-1-(3-isobutyrylaminomethyl-phenyl)-methylen]-6-fluor-2-indoli-non |
| 12.24 | 3-Z-[1-(4-(N-(4-Methyl-piperazin-1-yl-methylcarbonyl)-N-methyl-amino)-anilino)-1-(3-(3-methylbutyryl-aminomethyl-phenyl)-methylen]-6-fluor-2-indolinon |
| 12.25 | 3-Z-[1-(4-(N-(4-Methyl-piperazin-1-yl-methylcarbonyl)-N-methyl-amino)-anilino)-1-(3-cyclohexylmethylcarbonylaminomethyl-phenyl)-methylen]-6-fluor-2-indolinon |
| 12.26 | 3-Z-[1-(4-(N-(4-Methyl-piperazin-1-yl-methylcarbonyl)-N-methyl-amino)-anilino)-1-(3-methoxyacetylaminomethyl-phenyl)-methylen]-6-fluor-2-indolinon |
| 12.27 | 3-Z-[1-(4-(N-(4-Methyl-piperazin-1-yl-methylcarbonyl)-N-methyl-amino)-anilino)-1-(3-(2-methoxybenzoyl-aminomethyl)-phenyl)-methylen]-6-fluor-2-indolinon |
| 12.28 | 3-Z-[1-(4-(N-(4-Methyl-piperazin-1-yl-methylcarbonyl)-N-methyl-amino)-anilino)-1-(3-tert.butylacetylaminomethyl-phenyl)-methylen]-6-fluor-2-indolinon |
| 12.29 | 3-Z-[1-(4-(N-(4-Methyl-piperazin-1-yl-methylcarbonyl)-N-methyl-amino)-anilino)-1-(3-(2-thiophen-carbonylaminomethyl)-phenyl)-methylen]-6-fluor-2-indolinon |
| 12.30 | 3-Z-[1-(4-(N-(4-Methyl-piperazin-1-yl-methylcarbonyl)-N-methyl-amino)-anilino)-1-(3-pivaloylaminomethyl-phenyl)-methylen]-6-fluor-2-indolinon |
| 12.31 | 3-Z-[1-(4-(N-(4-Methyl-piperazin-1-yl-methylcarbonyl)-N-methyl-amino)-anilino)-1-(3-(2-furoylaminomethyl)-phenyl)-methylen]-6-fluor-2-indolinon |
| 12.32 | 3-Z-[1-(4-(N-(4-Methyl-piperazin-1-yl-methylcarbonyl)-N-methyl-amino)-anilino)-1-(3-acetylaminomethyl-phenyl)-methylen]-6-fluor-2-indolinon |
| 12.33 | 3-Z-[1-(4-(N-(4-Methyl-piperazin-1-yl-methylcarbonyl)-N-methyl-amino)-anilino)-1-(3-propionylaminomethyl-phenyl)-methylen]-6-fluor-2-indolinon |
| 12.34 | 3-Z-[1-(4-(N-(4-Methyl-piperazin-1-yl-methylcarbonyl)-N-methyl-amino)-anilino)-1-(3-benzoylaminomethyl-phenyl)-methylen]-6-fluor-2-indolinon |
| 12.35 | 3-Z-[1-(4-(N-(4-Methyl-piperazin-1-yl-methylcarbonyl)-N-methyl-amino)-anilino)-1-(3-phenylacetylaminomethyl-phenyl)-methylen]-6-fluor-2-indolinon |
| 12.36 | 3-Z-[1-(4-Dimethylaminomethyl-anilino)-1-(3-cyclopropylcarbonylaminomethyl-phenyl)-methylen]-6-fluor-2-indolinon |
| 12.37 | 3-Z-[1-(4-Dimethylaminomethyl-anilino)-1-(3-cyclobutylcarbonylaminomethyl-phenyl)-methylen]-6-fluor-2-indolinon |
| 12.38 | 3-Z-[1-(4-Dimethylaminomethyl-anilino)-1-(3-(pyridin-2-yl-carbonylaminomethyl)-phenyl)-methylen]-6-fluor-2-indolinon |
| 12.39 | 3-Z-[1-(4-Dimethylaminomethyl-anilino)-1-(3-cyclohexylcarbonylaminomethyl-phenyl)-methylen]-6-fluor-2-indolinon |
| 12.40 | 3-Z-[1-(4-Dimethylaminomethyl-anilino)-1-(3-(pyridin-3-yl-carbonylaminomethyl)-phenyl)-methylen]-6-fluor-2-indolinon |
| 12.41 | 3-Z-[1-(4-Dimethylaminomethyl-anilino)-1-(3-isobutyrylaminomethyl-phenyl)-methylen]-6-fluor-2-indolinon |
| 12.42 | 3-Z-[1-(4-Dimethylaminomethyl-anilino)-1-(3-(3-methylbutyryl-aminomethyl-phenyl)-methylen]-6-fluor-2-indolinon |
| 12.43 | 3-Z-[1-(4-Dimethylaminomethyl-anilino)-1-(3-cyclohexylmethylcarbonylaminomethyl-phenyl)-methylen]-6-fluor-2-indolinon |
| 12.44 | 3-Z-[1-(4-Dimethylaminomethyl-anilino)-1-(3-methoxyacetylaminomethyl-phenyl)-methylen]-6-fluor-2-indolinon |
| 12.45 | 3-Z-[1-(4-Dimethylaminomethyl-anilino)-1-(3-(2-methoxybenzoyl-aminomethyl)-phenyl)-methylen]-6-fluor-2-indolinon |
| 12.46 | 3-Z-[1-(4-Dimethylaminomethyl-anilino)-1-(3-tert.butylacetylaminomethyl-phenyl)-methylen]-6-fluor-2-indolinon |
| 12.47 | 3-Z-[1-(4-Dimethylaminomethyl-anilino)-1-(3-(2-thiophen-carbonylaminomethyl)-phenyl)-methylen]-6-fluor-2-indolinon |
| 12.48 | 3-Z-(1-(4-Dimethylaminomethyl-anilino)-1-(3-pivaloylaminomethyl-phenyl)-methylen]-6-fluor-2-indolinon |
| 12.49 | 3-Z-[1-(4-Dimethylaminomethyl-anilino)-1-(3-(2-furoylaminomethyl)-phenyl)-methylen]-6-fluor-2-indolinon |
| 12.50 | 3-Z-[1-(4-Dimethylaminomethyl-anilino)-1-(3-(pyridin-4-yl-carbonylaminomethyl)-phenyl)-methylen]-6-fluor-2-indolinon |
| 13.0 | 3-Z-[1-(4-Trimethylammoniummethyl-anilino)-1-(4-(2-carboxy-ethyl)-phenyl)-methylen]-6-fluor-2-indolinon-iodid |
| 13.1 | 3-Z-[1-(4-Trimethylammoniummethyl-anilino)-1-(3-(2-carboxy-ethyl)-phenyl)-methylen]-6-fluor-2-indolinon-iodid |
| 14.0 | 3-Z-[1-(4-Guanidinomethyl-anilino)-1-(4-(2-carboxy-ethyl)-phenyl)-methylen]-6-fluor-2-indolinon |
| 14.1 | 3-Z-[1-(4-Guanidinomethyl-anilino)-1-(3-(2-carboxy-ethyl)-phenyl)-methylen]-6-fluor-2-indolinon |

Verwendete Abkürzungen:

| | |
|---|---|
| HOBt = | 1-Hydroxy-1H-benzotriazol |
| TBTU = | O-Benzotriazol-1-yl-N,N,N',N'-tetramethyluronium-tetrafluoroborat |

### Herstellung der Ausgangsverbindungen:

### Beispiel I:

### 2-(4-Fluor-2-nitrophenyl)-malonsäuredimethylester

Zu einer Lösung von 188 ml Malonsäuredimethylester in 970 ml N-Methylpyrrolidon werden unter Eiskühlung 185 g Kalium-*tert*-butylat gegeben und der Ansatz 2 Stunden nachgerührt. Der entstandene Brei wird im Laufe von 30 Minuten tropfenweise mit 150 ml 2,5-Difluornitrobenzol versetzt und anschließend 6 Stunden bei 85 °C nachgerührt. Die Mischung wird auf 4 Liter Eiswasser und 250 ml konzentrierte Salzsäure gegossen und mit 2 Liter Ethylacatat extrahiert. Die organische Phase wird mit Natriumsulfat getrocknet und eingeengt. Der ölige Rückstand wird zweimal mit Wasser ausgerührt und anschließend in 600 ml Ethylacetat aufgenommen. Die Lösung wird mit Natriumsulfat getrocknet und zur Trockne eingeengt. Das kristallisierte Rohprodukt wird aus 600 ml Ethylacetat/Hexan = 2:8 umkristallisiert und getrocknet.
Ausbeute: 222 g (59 % der Theorie)
R_{f}-Wert: 0.40 (Kieselgel, Cyclohexan/Ethylacetat = 5:1)
C₁₁H₁₀FNO₆
Massenspektrum: m/z = 270 [M-H]⁻

Analog Beispiel I werden folgende Verbindungen hergestellt:
(I.1) 2-(4-Brom-2-nitrophenyl)-malonsäurediethylester
   aus 2,5-Dibromnitrobenzol und Malonsäurediethylester
   R_{f}-Wert: 0.40 (Kieselgel, Petrolether/Ethylacetat = 5:1)
   C₁₃H₁₄BrNO₆
   Massenspektrum: m/z = 359/361 [M]⁺
(1.2) 2-(4-Cyano-2-nitrophenyl)-malonsäuredimethylester
   aus 4-Chlor-3-nitro-benzonitril und Malonsäuredimethylester
   R_{f}-Wert: 0.50 (Kieselgel, Methylenchlorid/Methanol = 50:1)
   C₁₂H₁₀N₂O₆
   Massenspektrum: m/z = 277 [M-H]⁻

### Beispiel II:

### 4-Cyano-2-nitrophenylessigsäuremethylester

14.2 g 2-(4-Cyano-2-nitrophenyl)-malonsäuredimethylester (Edukt I.2) werden in 200 ml Dimethylsulfoxid gelöst und 4.5 g Lithiumchlorid und 1.0 ml Wasser zugesetzt. Die Lösung wird 3.5 Stunden bei 100 °C gerührt, anschließend mit 300 ml Eiswasser versetzt und für 12 Stunden stehen gelassen. Der entstandene Niederschlag wird abgesaugt, in Methylenchlorid aufgenommen und mit Wasser gewaschen. Die organische Phase wird über Natriumsulfat getrocknet, einrotiert und getrocknet.
Ausbeute: 7.7 g (68 % der Theorie)
R_{f}-Wert: 0.40 (Kieselgel, Methylenchlorid/Methanol) = 50:1
C₁₀H₈N₂O₄
Massenspektrum: m/z = 219 [M-H]⁻

### Beispiel III:

### 4-Fluor-2-nitrophenylessigsäure

50.0 g 2-(4-Fluor-2-nitrophenyl)-malonsäuredimethylester (Edukt I) werden in 400 ml 6 molarer Salzsäure 20 Stunden bei 100 °C gerührt, anschließend mit 400 ml Wasser versetzt und auf 0 °C abgekühlt. Der entstandene Niederschlag wird abgesaugt, mit Wasser und 100 ml Petrolether gewaschen und getrocknet.
Ausbeute: 34.5 g (94 % der Theorie)
R_{f}-Wert: 0.30 (Kieselgel, Cyclohexan/Ethylacetat) = 5:2
C₈H₆FNO₄
Massenspektrum: m/z = 154 [M-COO-H]⁻

### Beispiel IV:

### 6-Fluor-2-indolinon

119 g 4-Fluor-2-nitrophenylessigsäure (Edukt III) werden in 600 ml Essigsäure unter Zusatz von 20 g Palladium auf Aktivkohle (10%) unter 50 psi Wasserstoffdruck hydriert. Der Katalysator wird abgesaugt, das Lösungsmittel abdestilliert. Das Rohprodukt wird mit 500 ml Petrolether ausgerührt, abgesaugt, mit Wasser gewaschen und getrocknet.
Ausbeute: 82.5 g (91 % der Theorie)
R_{f}-Wert: 0.30 (Kieselgel, Petrolether/Ethylacetat = 1:1)
C₈H₆FNO
Massenspektrum: m/z =150 [M-H]⁻

Analog Beispiel IV werden folgende Verbindungen hergestellt:
(IV.1) 6-Brom-2-indolinon
   aus 2-(4-Brom-2-nitrophenyl)-malonsäurediethylester (Edukt I.1) mit Raney-Nickel als Hydrierkatalysator
   R_{f}-Wert: 0.45 (Kieselgel, Petrolether/Ethylacetat = 1:1)
   C₈H₆BrNO Massenspektrum: m/z = 210/212 [M-H]⁻
(IV.2) 6-Cyano-2-indolinon
   aus 4-Cyano-2-nitrophenylessigsäuremethylester (Edukt II) mit Palladium/Calciumcarbonat als Hydrierkatalysator
   R_{f}-Wert: 0.45 (Kieselgel, Methylenchlorid/Methanol = 9:1)
   C₉H₆N₂O Massenspektrum: m/z = 157 [M-H]⁻

### Beispiel V:

### 1-Acetyl-6-fluor-2-indolinon

82.5 g 6-Fluor-2-indolinon (Edukt IV) werden in 180 ml Essigsäureanhydrid 3 Stunden bei 130 °C gerührt. Nach Abkühlen auf Raumtemperatur wird der Niederschlag abgesaugt, mit 100 ml Petrolether gewaschen und getrocknet.
Ausbeute: 64.8 g (61 % der Theorie)
R_{f}-Wert: 0.75 (Kieselgel, Petrolether/Ethylacetat = 1:1)
C₁₀H₈FNO₂
Massenspektrum: m/z = 192 [M-H]⁻

Analog Beispiel V werden folgende Verbindungen hergestellt:
(V.1) 1-Acetyl-6-chlor-2-indolinon
   aus 6-Chlor-2-indolinon und Essigsäureanhydrid
   R_{f}-Wert: 0.55 (Kieselgel, Petrolether/Ethylacetat = 2:3)
   C₁₁H₁₀CINO₆
   Massenspektrum: m/z = 208/210 [M-H]⁻
(V.2) 1-Acetyl-6-brom-2-indolinon
   aus 6-Brom-2-indolinon (Edukt IV.1) und Essigsäureanhydrid
   R_{f}-Wert: 0.60 (Kieselgel, Petrolether/Ethylacetat = 2:1)
   C₁₀H₈BrNO₂
   Massenspektrum: m/z = 253/255 [M]⁺
(V.3) 1-Acetyl-6-cyano-2-indolinon
   aus 6-Cyano-2-indolinon (Edukt IV.2) und Essigsäureanhydrid
   R_{f}-Wert: 0.60 (Kieselgel, Methylenchlorid/Methanol = 50:1)
   C₁₁H₈N₂O₂
   Massenspektrum: m/z = 199 [M-H]⁻

### Beispiel VI:

### 1-Acetyl-3-[1-hydroxy-1-(3-iod-phenyl)-methylen]-6-chlor-2-indolinon

10.5 g 1-Acetyl-6-chlor-2-indolinon (Edukt V.1), 13.6 g 3-lodbenzoesäure und 17.7 g TBTU werden in 100 ml Dimethylformamid vorgelegt, 35 ml Triethylamin zugegeben und das Gemisch für 12 Stunden bei Raumtemperatur gerührt. Nach dieser Zeit wird das Lösungsmittel abgezogen, der Rückstand mit Wasser versetzt, abgesaugt und mit wenig Wasser, Methanol und Ether gewaschen und im Vakuum bei 100°C getrocknet.
Ausbeute: 12.9 g (59 % der Theorie)
R_{f}-Wert: 0.80 (Kieselgel, Methylenchlorid/Methanol = 9:1)
C₁₇H₁₁ClINO₃
Massenspektrum: m/z = 438/440 [M-H]⁻

Analog Beispiel VI werden folgende Verbindungen hergestellt:
(VI.1) 1-Acetyl-3-[1-hydroxy-1-(4-methoxycarbonylmethyl-phenyl)-methylen]-6-fluor-2-indolinon
   aus 1-Acetyl-6-fluor-2-indolinon (Edukt V) und (4-Carboxyphenyl)-essigsäuremethylester (Darstellung nach Tetrahedron 1997, 53, 7335-7340)
(VI.2) 1-Acetyl-3-[1-hydroxy-1-(4-chlor-phenyl)-methylen]-6-chlor-2-indolinon
   aus 1-Acetyl-6-chlor-2-indolinon (Edukt V.1) und 4-Chlor-benzoesäure
(VI.3) 1-Acetyl-3-[1-hydroxy-1-(3,4-dimethoxy-phenyl)-methylen]-6-chlor-2-indolinon
   aus 1-Acetyl-6-chlor-2-indolinon (Edukt V.1) und 3,4-Dimethoxy-benzoesäure
(VI.4) 1-Acetyl-3-[1-hydroxy-1-(3,4-dimethoxy-phenyl)-methylen]-6-cyano-2-indolinon
   aus 1-Acetyl-6-cyano-2-indolinon (Edukt V.3) und 3,4-Dimethoxy-benzoesäure
(VI.5) 1-Acetyl-3-[1-hydroxy-1-(3-fluor-phenyl)-methylen]-6-fluor-2-indolinon
   aus 1-Acetyl-6-fluor-2-indolinon (Edukt V) und 3-Fluor-benzoesäure
(VI.6) 1-Acetyl-3-[1-hydroxy-1-(4-(2-acetylamino-ethyl)-phenyl)-methylen]-6-fluor-2-indolinon
   aus 1-Acetyl-6-fluor-2-indolinon (Edukt V) und 4-(2-Acetylamino-ethyl)-benzoesäure (Darstellung nach J. Am. Chem. Soc. 1943, 65, 2377)
(VI.7) 1-Acetyl-3-[1-hydroxy-1-(3-methoxycarbonylmethyl-phenyl)-methylen]-6-fluor-2-indolinon
   aus 1-Acetyl-6-fluor-2-indolinon (Edukt V) und (3-Carboxyphenyl)-essigsäuremethylester (Darstellung analog zu Tetrahedron 1997, 53, 7335-7340)
(VI.8) 1-Acetyl-3-[1-hydroxy-1-(3-(N-tert.butoxycarbonyl-aminomethyl)-phenyl)-methylen]-6-fluor-2-indolinon
   aus 1-Acetyl-6-fluor-2-indolinon (Edukt V) und 3-(N-tert.Butoxycarbonylaminomethyl)-benzoesäure (Darstellung nach Tetrahedron 1997, 53, 7335-7340)
(VI.9) 1-Acetyl-3-[1-hydroxy-1-(3-cyanomethyl-phenyl)-methylen]-6-fluor-2-indolinon
   aus 1-Acetyl-6-fluor-2-indolinon (Edukt V) und (3-Carboxy-phenyl)-acetonitril (Darstellung nach J. Prakt. Chem. 1998, 340, 367-374)
(VI.10) 1-Acetyl-3-[1-hydroxy-1-(4-(N-tert.butoxycarbonyl-aminomethyl)-phenyl)-methylen]-6-fluor-2-indolinon
   aus 1-Acetyl-6-fluor-2-indolinon (Edukt V) und 4-(N-tert.Butoxycarbonylaminomethyl)-benzoesäure (Darstellung nach Bioorg. Med. Chem. Lett 2000, 10, 553-557)
(VI.11) 1-Acetyl-3-[1-hydroxy-1-(4-iod-phenyl)-methylen]-6-fluor-2-indolinon
   aus 1-Acetyl-6-fluor-2-indolinon (Edukt V) und 4-lod-benzoesäure
(VI.12) 1-Acetyl-3-[1-hydroxy-1-(4-iod-phenyl)-methylen]-6-chlor-2-indolinon
   aus 1-Acetyl-6-chlor-2-indolinon (Edukt V.1) und 4-lod-benzoesäure
(VI.13) 1-Acetyl-3-[1-hydroxy-1-(3-iod-phenyl)-methylen]-6-fluor-2-indolinon
   aus 1-Acetyl-6-fluor-2-indolinon (Edukt V) und 3-lod-benzoesäure
(VI.14) 1-Acetyl-3-[1-hydroxy-1-(4-(2-methoxycarbonylethyl)-phenyl)-methylen]-6-fluor-2-indolinon
   aus 1-Acetyl-6-fluor-2-indolinon (Edukt V) und 4-(2-Methoxycarbonylethyl)-benzoesäure (Darstellung analog zu Tetrahedron 1997, 53, 7335-7340)
(VI.15) 1-Acetyl-3-[1-hydroxy-1-(3-(2-methoxycarbonylethyl)-phenyl)-methylen]-6-fluor-2-indolinon
   aus 1-Acetyl-6-fluor-2-indolinon (Edukt V) und 3-(2-Methoxycarbonylethyl)-benzoesäure (Darstellung analog zu Tetrahedron 1997, 53, 7335-7340)
(VI.16) 1-Acetyl-3-[1-hydroxy-1-(3-(N-tert.butoxycarbonyl-2-aminoethyl)-phenyl)-methylen]-6-fluor-2-indolinon
   aus 1-Acetyl-6-fluor-2-indolinon (Edukt V) und 3-(N-tert.Butoxycarbonyl-2-aminoethyl)-benzoesäure (Darstellung analog zu Bioorg. Med. Chem. Lett 2000, 10, 553-557)
(VI.17) 1-Acetyl-3-[1-hydroxy-1-(4-(N-tert.butoxycarbonyl-2-aminoethyl)-phenyl)-methylen]-6-fluor-2-indolinon
   aus 1-Acetyl-6-fluor-2-indolinon (Edukt V) und 4-(N-tert.Butoxycarbonyl-2-aminoethyl)-benzoesäure (Darstellung analog zu Bioorg. Med. Chem. Lett 2000, 10, 553-557)
(VI.18) 1-Acetyl-3-[1-hydroxy-1-(4-cyano-phenyl)-methylen]-6-chlor-2-indolinon
   aus 1-Acetyl-6-chlor-2-indolinon (Edukt V.1) und 4-Cyano-benzoesäure
(VI.19) 1-Acetyl-3-[1-hydroxy-1-(3-acetylaminomethyl-phenyl)-methylen]-6-fluor-2-indolinon
   aus 1-Acetyl-6-fluor-2-indolinon (Edukt V) und 3-Acetylaminomethyl-benzoesäure (dargestellt nach J. Med. Chem. 1997, 40, 4030-4052)
(VI.20) 1-Acetyl-3-[1-hydroxy-1-(3-(2-ethoxycarbonylethyl)-phenyl)-methylen]-6-fluor-2-indolinon
   aus 1-Acetyl-6-fluor-2-indolinon (Edukt V) und 3-(2-Ethoxycarbonylethyl)-benzoesäure (Darstellung analog zu Tetrahedron 1997, 53, 7335-7340)
(VI.21) 1-Acetyl-3-[1-hydroxy-1-(4-(2-methoxycarbonylethyl)-phenyl)-methylen]-6-chlor-2-indolinon
   aus 1-Acetyl-6-chlor-2-indolinon (Edukt V.1) und 4-(2-Methoxycarbonylethyl)-benzoesäure (Darstellung analog zu Tetrahedron 1997, 53, 7335-7340)
(VI.22) 1-Acetyl-3-[1-hydroxy-1-(4-(2-ethoxycarbonylethyl)-phenyl)-methylen]-6-fluor-2-indolinon
   aus 1-Acetyl-6-fluor-2-indolinon (Edukt V) und 4-(2-Ethoxycarbonylethyl)-benzoesäure (Darstellung analog zu Tetrahedron 1997, 53, 7335-7340)
(VI.23) 1-Acetyl-3-[1-hydroxy-1-(3-methoxycarbonylmethyloxy-phenyl)-methylen]-6-fluor-2-indolinon
   aus 1-Acetyl-6-fluor-2-indolinon (Edukt V) und 3-Methoxycarbonylmethyloxybenzoesäure (Darstellung siehe Tetrahedron Letters 1998, 39, 8563-8566)
(VI.24) 1-Acetyl-3-[1-hydroxy-1-(4-methoxycarbonylmethyloxy-phenyl)-methylen]-6-fluor-2-indolinon
   aus 1-Acetyl-6-fluor-2-indolinon (Edukt V) und 4-Methoxycarbonylmethyloxybenzoesäure (Darstellung analog zu Tetrahedron Letters 1998, 39, 8563-8566)
(VI.25) 1-Acetyl-3-[1-hydroxy-1-(3-(2-ethoxycarbonyl-ethyloxy)-phenyl)-methylen]-6-fluor-2-indolinon
   aus 1-Acetyl-6-fluor-2-indolinon (Edukt V) und 3-(2-Ethoxycarbonyl-ethyloxy)-benzoesäure (Darstellung siehe PCT Int. Appl. WO9620173, 60)
(VI.26) 1-Acetyl-3-[1-hydroxy-1-(4-(2-ethoxycarbonyl-ethyloxy)-phenyl)-methylen]-6-fluor-2-indolinon
   aus 1-Acetyl-6-fluor-2-indolinon (Edukt V) und 4-(2-Ethoxycarbonyl-ethyloxy)-benzoesäure (Darstellung siehe PCT Int. Appl. WO9620173, 58)
(VI.27) 1-Acetyl-3-[1-hydroxy-1-(4-(2-methoxycarbonylethyl)-phenyl)-methylen]-6-brom-2-indolinon
   aus 1-Acetyl-6-brom-2-indolinon (Edukt V.2) und 4-(2-Methoxycarbonylethyl)-benzoesäure (Darstellung analog zu Tetrahedron 1997, 53, 7335-7340)

### Beispiel VII:

### 1-Acetyl-3-[1-methoxy-1-(3-iod-phenyl)-methylen]-6-chlor-2-indolinon

Eine Lösung von 3.52 g 1-Acetyl-3-[1-hydroxy-1-(3-iod-phenyl)-methylen]-6-chlor-2-indolinon (Edukt VI) und 2.72 ml Ethyldiisopropylamin in 80 ml Dichlormethan wird portionsweise mit 2.36 g Trimethyloxoniumtetrafluoroborat versetzt und eine Stunde bei Raumtemperatur gerührt. Dann werden nochmals 1.4 ml Ethyldiisopropylamin und 1.2 g Trimethyloxoniumtetrafluoroborat zugegeben und weitere zwei Stunden bei Raumtemperatur gerührt. Anschließend wird mit Wasser extrahiert, die organische Phase über Magnesiumsulfat getrocknet und zur Trockne eingeengt. Der Rückstand wird aus Ether umkristallisiert und bei 80 °C im Vakuum getrocknet.
Ausbeute: 2.40 g (66 % der Theorie)
R_{f}-Wert: 0.60 (Kieselgel, Petrolether/Dichlormethan/Ethylacetat = 5:4:1)
C₁₈H₁₃ClINO₃
Massenspektrum: m/z = 438/440 [M-H]⁻
Fp. 185 - 187 °C

Analog Beispiel VII werden folgende Verbindungen hergestellt:
(VII.1) 1-Acetyl-3-[1-methoxy-1-(4-methoxycarbonylmethyl-phenyl)-methylen]-6-fluor-2-indolinon
   aus 1-Acetyl-3-[1-hydroxy-1-(4-methoxycarbonylmethyl-phenyl)-methylen]-6-fluor-2-indolinon (Edukt VI.1)
(VII.2) 1-Acetyl-3-[1-methoxy-1-(4-chlor-phenyl)-methylen]-6-chlor-2-indolinon
   aus 1-Acetyl-3-[1-hydroxy-1-(4-chlor-phenyl)-methylen]-6-chlor-2-indolinon (Edukt VI.2)
(VII.3) 1-Acetyl-3-[1-methoxy-1-(3,4-dimethoxy-phenyl)-methylen]-6-chlor-2-indolinon
   aus 1-Acetyl-3-[1-hydroxy-1-(3,4-dimethoxy-phenyl)-methylen]-6-chlor-2-indolinon (Edukt VI.3)
(VII.4) 1-Acetyl-3-[1-methoxy-1-(3,4-dimethoxy-phenyl)-methylen]-6-cyano-2-indolinon
   aus 1-Acetyl-3-[1-hydroxy-1-(3,4-dimethoxy-phenyl)-methylen]-6-cyano-2-indolinon (Edukt VI.4)
(VII.5) 1-Acetyl-3-[1-methoxy-1-(3-fluor-phenyl)-methylen]-6-fluor-2-indolinon
   aus 1-Acetyl-3-[1-hydroxy-1-(3-fluor-phenyl)-methylen]-6-fluor-2-indolinon (Edukt VI.5)
(VII.6) 1-Acetyl-3-[1-methoxy-1-(4-(2-acetylamino-ethyl)-phenyl)-methylen]-6-fluor-2-indolinon
   aus 1-Acetyl-3-[1-hydroxy-1-(4-(2-acetylamino-ethyl)-phenyl)-methylen]-6-fluor-2-indolinon (Edukt VI.6)
(VII.7) 1-Acetyl-3-[1-methoxy-1-(3-methoxycarbonylmethyl-phenyl)-methylen]-6-fluor-2-indolinon
   aus 1-Acetyl-3-[1-hydroxy-1-(3-methoxycarbonylmethyl-phenyl)-methylen]-6-fluor-2-indolinon (Edukt VI.7)
(VII.8) 1-Acetyl-3-[1-methoxy-1-(3-(N-tert.butoxycarbonyl-aminomethyl)-phenyl)-methylen]-6-fluor-2-indolinon
   aus 1-Acetyl-3-[1-hydroxy-1-(3-(N-tert.butoxycarbonyl-aminomethyl)-phenyl)-methylen]-6-fluor-2-indolinon (Edukt VI.8)
(VII.9) 1-Acetyl-3-[1-methoxy-1-(3-cyanomethyl-phenyl)-methylen]-6-fluor-2-indolinon
   aus 1-Acetyl-3-[1-hydroxy-1-(3-cyanomethyl-phenyl)-methylen]-6-fluor-2-indolinon (Edukt VI.9)
(VII.10) 1-Acetyl-3-[1-methoxy-1-(4-(N-tert.butoxycarbonyl-aminomethyl)-phenyl)-methylen]-6-fluor-2-indolinon
   aus 1-Acetyl-3-[1-hydroxy-1-(4-(N-tert.butoxycarbonyl-aminomethyl)-phenyl)-methylen]-6-fluor-2-indolinon (Edukt VI.10)
(VII.11) 1-Acetyl-3-[1-methoxy-1-(4-iod-phenyl)-methylen]-6-fluor-2-indolinon
   aus 1-Acetyl-3-[1-hydroxy-1-(4-iod-phenyl)-methylen]-6-fluor-2-indolinon (Edukt VI.11)
(VII.12) 1-Acetyl-3-[1-methoxy-1-(4-iod-phenyl)-methylen]-6-chlor-2-indolinon
   aus 1-Acetyl-3-[1-hydroxy-1-(4-iod-phenyl)-methylen]-6-chlor-2-indolinon (Edukt VI.12)
(VII.13) 1-Acetyl-3-[1-methoxy-1-(3-iod-phenyl)-methylen]-6-fluor-2-indolinon
   aus 1-Acetyl-3-[1-hydroxy-1-(3-iod-phenyl)-methylen]-6-fluor-2-indolinon (Edukt VI.13)
(VII.14) 1-Acetyl-3-[1-methoxy-1-(3-(2-methoxycarbonylethyl)-phenyl)-methylen]-6-fluor-2-indolinon
   aus 1-Acetyl-3-[1-hydroxy-1-(3-(2-methoxycarbonylethyl)-phenyl)-methylen]-6-fluor-2-indolinon (Edukt VI.14)
(VII.15) 1-Acetyl-3-[1-methoxy-1-(4- (2-methoxycarbonylethyl)- phenyl)-methylen]-6-fluor-2-indolinon
   aus 1-Acetyl-3-[1-hydroxy-1-(4-(2-methoxycarbonylethyl)-phenyl)-methylen]-6-fluor-2-indolinon (Edukt VI.15)
(VII.16) 1-Acetyl-3-[1-methoxy-1-(4-(N-tert.butoxycarbonyl-2-aminoethyl)-phenyl)-methylen]-6-fluor-2-indolinon
   aus 1-Acetyl-3-[1-hydroxy-1-(4-(N-tert.butoxycarbonyl-2-aminoethyl)-phenyl)-methylen]-6-fluor-2-indolinon (Edukt VI.17)
(VII.17) 1-Acetyl-3-[1-methoxy-1-(3-(N-tert.butoxycarbonyl-2-aminoethyl)-phenyl)-methylen]-6-fluor-2-indolinon
   aus 1-Acetyl-3-[1-hydroxy-l-(3-(N-tert.butoxycarbonyl-2-aminoethyl)-phenyl)-methylen]-6-fluor-2-indolinon (Edukt VI.16)
(VII.18) 1-Acetyl-3-[1-methoxy-1-(3-acetylaminomethyl-phenyl)-methylen]-6-fluor-2-indolinon
   aus 1-Acetyl-3-[1-hydroxy-1-(3-acetylaminomethyl-phenyl)-methylen]-6-fluor-2-indolinon (Edukt VI.19)
(VII.19) 1-Acetyl-3-[1-methoxy-1-(3-(2-ethoxycarbonylethyl)-phenyl)-methylen]-6-fluor-2-indolinon
   aus 1-Acetyl-3-[1-hydroxy-1-(3-(2-ethoxycarbonylethyl)-phenyl)-methylen]-6-fluor-2-indolinon (Edukt VI.20)
(VII.20) 1-Acetyl-3-[1-methoxy-1-(4-(2-methoxycarbonylethyl)-phenyl)-methylen]-6-chlor-2-indolinon
   aus 1-Acetyl-3-[1-hydroxy-1-(4-(2-methoxycarbonylethyl)-phenyl)-methylen]-6-chlor-2-indolinon (Edukt VI.21)
(VII.21) 1-Acetyl-3-[1-methoxy-1-(4-(2-ethoxycarbonylethyl)-phenyl)-methylen]-6-fluor-2-indolinon
   aus 1-Acetyl-3-[1-hydroxy-1-(4-(2-ethoxycarbonylethyl)-phenyl)-methylen]-6-fluor-2-indolinon (Edukt VI.22)
(VII.22) 1-Acetyl-3-[1-methoxy-1-(4-methoxycarbonylmethyloxy-phenyl)-methylen]-6-fluor-2-indolinon
   aus 1-Acetyl-3-[1-hydroxy-1-(4-methoxycarbonylmethyloxy-phenyl)-methylen]-6-fluor-2-indolinon (Edukt VI.23)
(VII.23) 1-Acetyl-3-[1-methoxy-1-(3-methoxycarbonylmethyloxy-phenyl)-methylen]-6-fluor-2-indolinon
   aus 1-Acetyl-3-[1-hydroxy-1-(3-methoxycarbonylmethyloxy-phenyl)-methylen]-6-fluor-2-indolinon (Edukt VI.24)
(VII.24) 1-Acetyl-3-[1-methoxy-1-(3-(2-ethoxycarbonyl-ethyloxy)-phenyl)-methylen]-6-fluor-2-indolinon
   aus 1-Acetyl-3-[1-hydroxy-1-(3-(2-ethoxycarbonyl-ethyloxy)-phenyl)-methylen]-6-fluor-2-indolinon (Edukt VI.25)
(VII.25) 1-Acetyl-3-[1-methoxy-1-(4-(2-ethoxycarbonyl-ethyloxy)-phenyl)-methylen]-6-fluor-2-indolinon
   aus 1-Acetyl-3-[1-hydroxy-1-(4-(2-ethoxycarbonyl-ethyloxy)-phenyl)-methylen]-6-fluor-2-indolinon (Edukt VI.26)
(VII.26) 1-Acetyl-3-[1-methoxy-1-(4-(2-methoxycarbonylethyl)-phenyl)-methylen]-6-brom-2-indolinon
   aus 1-Acetyl-3-[1-hydroxy-1-(4-(2-methoxycarbonylethyl)-phenyl)-methylen]-6-brom-2-indolinon (Edukt VI.27)

### Beispiel VIII:

### 1-Acetyl-3-[1-chlor-1-(4-cyano-phenyl)-methylen]-6-chlor-2-indolinon

Eine Suspension von 7.0 g 1-Acetyl-3-[1-chlor-1-(4-cyano-methyl)-phenyl)-methylen]-6-chlor-2-indolinon (Edukt VI.18) und 6.39 g Phosphorpentachlorid in 150 ml Dioxan wird 6 Stunden bei 100 °C gerührt. Nach Zugabe von weiteren 1.0 g
Phosphorpentachlorid wird weitere 4 Stunden bei 110 °C gerührt. Anschließend wird das Lösungsmittel abdestilliert und der Rückstand mit Ethylacetat gewaschen.
Ausbeute: 4.5 g (61 % der Theorie)
R_{f}-Wert: 0.70 (Kieselgel, Methylenchlorid/Methanol = 50:1)
C₁₈H₁₀Cl₂N₂O₂

### Beispiel IX:

Die Synthesen folgender Verbindungen sind bereits in der internationalen Anmeldung WO 01/27081 beschrieben:
(IX.1) 4-(Diethylamino-methyl)-anilin
(IX.2) N-(2-Dimethylamino-ethyl)-N-methylsulfonyl-p-phenylendiamin
(IX.3) 3-(Dimethylaminomethyl)-anilin
(IX.4) 4-(Dimethylaminomethyl)-anilin
(IX.5) 4-(2-Dimethylamino-ethyl)-anilin
(IX.6) 4-[N-(2-Dimethylamino-ethyl)-N-acetyl-amino]-anilin
(IX.7) 4-[N-(3-Dimethylamino-propyl)-N-acetyl-amino]-anilin
(IX.8) 4-[(N-Dimethylaminocarbonylmethyl-N-methylsulfonyl)-amino]-anilin
(IX.9) N-(4-Aminophenyl)-N-methyl-methansulfonamid
(IX.10) N-(Dimethylamino-methylcarbonyl)-N-methyl-p-phenylendiamin
(IX.11) N-[(2-Dimethylamino-ethyl)-carbonyl]-N-methyl-p-phenylendiamin
(IX.12) 4-(N-tert.Butoxycarbonyl-aminomethyl)-anilin
(IX.13) 4-(N-Ethyl-N-tert.butoxycarbonyl-aminomethyl)-anilin
(IX.14) 4-[(4-Methyl-piperazin-1-yl)-methyl]-anilin
(IX.15) 4-(Imidazol-1-yl-methyl)-anilin
(IX.16) 4-(1-Methyl-imidazol-2-yl)-anilin
(IX.17) 4-[(N-(2-Dimethylamino-ethyl)-N-methyl-amino)-methyl]-anilin
(IX.18) 4-(N-Methyl-N-tert.butoxycarbonyl-aminomethyl)-anilin
(IX.19) N-[(4-Methyl-piperazin-1-yl)-methylcarbonyl]-N-methyl-p-phenylendiamin
(IX.20) 4-(4-tert.Butoxycarbonyl-piperazin-1-yl-methyl)-anilin
(IX.21) 4-(Thiomorpholin-4-yl-methyl)-anilin
(IX.22) 4-(Pyrrolidin-1-yl-methyl)-anilin
(IX.23) 4-(Morpholin-4-yl-methyl)-anilin
(IX.24) 4-(N-Benzyl-N-methyl-aminomethyl)-anilin
(IX.25) 4-(N-Ethyl-N-methyl-aminomethyl)-anilin
(IX.26) 4-[N-(2-Dimethylamino-ethyl)-N-methyl-amino]-anilin
(IX.27) 4-[(N-Propyl-N-methyl-amino)-methyl]-anilin
Analog Beispiel IX werden folgende Verbindungen hergestellt:
(IX.28) 4-[N-(2-(N-Benzyl-N-methyl-amino)-ethyl)-N-acetyl-amino]-anilin
(IX.29) 4-Amino-N-(2-dimethylamino-ethyl)-N-methyl-benzamid
(IX.30) 4-(4-Methyl-piperazin-1-yl-carbonyl)-anilin
(IX.31) 4-(2-Dimethylamino-ethoxy)-anilin
(IX.32) N-(4-Dimethylaminobutylcarbonyl)-N-methyl-p-phenylendiamin
(IX.33) N-[(3-Dimethylamino-propyl)-carbonyl]-N-methyl-p-phenylendiamin

Herstellung der Endverbindungen (nur Beispiele, welche unter Formel (I) fallen sind erfindungsgemäß).

### Beispiel 1.0

### 3-Z-[1-(4-(N-Methyl-N-methylsulfonyl-amino)-anilino)-1-(3-iod-phenyl)-methylen]-6-chlor-2-indolinon

0.9 g 1-Acetyl-3-(1-methoxy-1-(3-iod-phenyl)-methylen)-6-chlor-2-indolinon (Edukt VII) und 0.5 g N-Methyl-N-methylsulfonyl-p-phenylendiamin (Edukt IX.9) werden in 10 ml Dimethylformamid gelöst und 3 Stunden bei 120°C gerührt. Nach dem Abkühlen werden 1.5 ml Piperidin zugegeben und eine weitere Stunde bei Raumtemperatur gerührt. Man gibt Wasser zu, saugt den erhaltenen Niederschlag ab, wäscht ihn mit wenig Wasser, Methanol und Ether und trocknet ihn schließlich im Vakuum bei 100°C.
Ausbeute: 0.9 g (74% der Theorie),
R_{f}-Wert: 0.6 (Kieselgel, Methylenchlorid/Methanol = 9:1)
Fp. 292-294 °C
C₂₃H₁₉ClIN₃O₃S Massenspektrum: m/z = 578/580 [M-H]⁻

Analog Beispiel 1.0 werden folgende Verbindungen der allgemeinen Formel I-1 hergestellt:

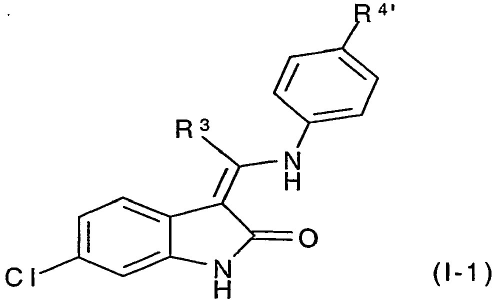

| Beispiel | R³ | R⁴ | Edukte | Summenformel | Massenspektrum | Fp. [°C] | R_{f}-Wert* |
|---|---|---|---|---|---|---|---|
| 1.1 | 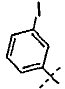 | -CH₂-NMe₂ | VII | C₂₄H₂₁ClIN₃O | 529/531 [M+H]⁺ | 238-240 | 0.30 (A) |
| | | | IX.4 | | | | |
| 1.2 | 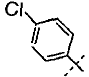 | -N(Me)-(CO)-CH₂-NMe₂ | VII.2 | C₂₆H₂₄Cl₂N₄O₂ | 495/497 [M+H]⁺ | 277-279 | 0.20 (B) |
| | | | IX.10 | | | | |
| 1.3 | 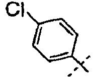 | -N(COMe)-(CH₂)₂-NMe₂ | VII.2 | C₂₇H₂₆Cl₂N₄O₂ | 507/509 [M-H]⁻ | 241-243 | 0.10 (B) |
| | | | IX.6 | | | | |
| 1.4 | 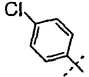 | 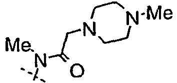 | VII.2 | C₂₉H₂₉Cl₂N₅O₂ | 548/550 [M-H]⁻ | 266-268 | 0.10 (B) |
| | | | IX.19 | | | | |
| 1.5 | 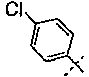 | -N(COMe)-(CH₂)₃-NMe₂ | VII.2 | C₂₈H₂₈Cl₂N₄O₂ | 521/523 [M-H]⁻ | 241-242 | 0.10 (B) |
| | | | IX.7 | | | | |
| 1.6 | 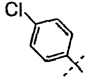 | -CH₂-NMe₂ | VII.2 | C₂₄H₂₁Cl₂N₃O | 438/440 [M+H]⁺ | 243-244 | 0.10 (B) |
| | | | IX.4 | | | | |
| 1.7 | 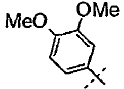 | -N(COMe)-(CH₂)₂-NMe₂ | VII.3 | C₂₉H₃₁ClN₄O₄ | 533/535 [M-H]⁻ | 128-130 | 0.75 (C) |
| | | | IX.6 | | | | |
| 1.8 | 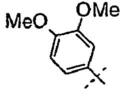 | 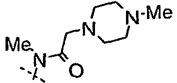 | VII.3 | C₃₁H₃₄ClN₅O₄ | 574/576 [M-H]⁻ | 208-210 | 0.65 (C) |
| | | | IX.19 | | | | |
| 1.9 | 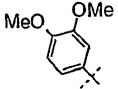 | -N(SO₂Me)-(CH₂)₂-NMe₂ | VII.3 | C₂₈H₃₁ClN₄O₅S | 569/571 [M-H]⁻ | 198-200 | 0.75 (C) |
| | | | IX.2 | | | | |
| 1.10 | 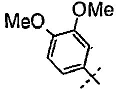 | -CH₂-NMe₂ | VII.3 | C₂₆H₂₆ClN₃O₃ | 462/464 [M-H]⁻ | 239-240 | 0.70 (C) |
| | | | IX.4 | | | | |
| 1.11 | 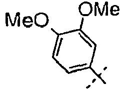 | 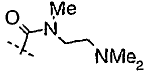 | VII.3 | C₂₉H₃₁CIN₄O₄ | 533/535 [M-H]⁻ | 147-149 | 0.70 (C) |
| | | | IX.29 | | | | |

| | | | | | | | |
|---|---|---|---|---|---|---|---|
| *Fließmittelgemische: (A): Kieselgel, Methylenchlorid/Methanol 9:1 (B): Kieselgel, Methylenchlorid/Ethanol 10:1 (C): Kieselgel, Methylenchlorid/Methanol 4:1 | | | | | | | |

### Beispiel 2.0

### 3-Z-[1-(4-(Dimethylamino-methyl)-anilino)-1-(4-cyano-phenyl)-methylen]-6-chlor-2-indolinon

1.07 g 1-Acetyl-3-[1-chlor-1-(4-cyano-phenyl)-methylen]-6-chlor-2-indolinon (Edukt VII) und 0.54 g 4-(Dimethylaminomethyl)-anilin (Edukt IX.4) werden in 10 ml Dimethylformamid gelöst und 3 Stunden bei 80°C gerührt. Nach dem Abkühlen wird 1 ml 6N Natronlauge zugegeben und 30 Minuten bei Raumtemperatur gerührt. Man gibt Wasser zu und extrahiert dreimal mit Methylenchlorid. Die vereinigten organischen Phasen werden zweimal mit Wasser gewaschen, über Natriumsulfat getrocknet, einrotiert und das Produkt aus Diethylether umkristallisiert.
Ausbeute: 0.92 g (72% der Theorie),
R_{f}-Wert: 0.1 (Kieselgel, Methylenchlorid/Methanol = 9:1)
C₂₅H₂₁ClN₄O
Massenspektrum: m/z = 427/429 [M-H]⁻

### Beispiel 3.0

### 3-Z-[1-(4-(Dimethylaminomethyl)-anilino)-1-(4-iod-phenyl)-methylen]-6-fluor-2-indolinon

3.5 g 1-Acetyl-3-(1-methoxy-1-(4-iod-phenyl)-methylen)-6-fluor-2-indolinon (Edukt VII.11) und 1.6 g 4-(Dimethylaminomethyl)-anilin (Edukt IX.4) werden in 30 ml Dimethylformamid gelöst und 2 Stunden bei 120°C gerührt. Nach dem Abkühlen wird das Lösungsmittel abgezogen, der Rückstand in 30 ml Methanol aufgenommen und 2 Spatelspitzen Natriummethylat zugegeben. Nach Auftreten eines gelben Niederschlags saugt man vom Lösungsmittel ab, wäscht den Rückstand mit wenig Methanol und Ether und trocknet ihn schließlich im Vakuum bei 100°C.
Ausbeute: 1.9 g (46% der Theorie),
R_{f}-Wert: 0.3 (Kieselgel, Methylenchlorid/Methanol = 9:1)
Fp. 243-246 °C
C₂₄H₂₁FIN₃O
Massenspektrum: m/z = 514 [M+H]⁺

Analog Beispiel 3.0 werden folgende Verbindungen der allgemeinen Formel I-3a hergestellt:

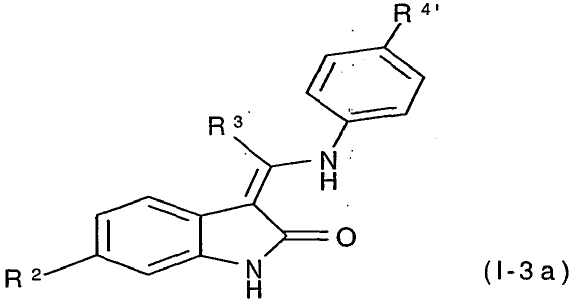

| Beispiel | R² | R³ | R⁴' | Edukte | Summenformel | Massenspektrum | Fp. [°C] | R_{f}-Wert* |
|---|---|---|---|---|---|---|---|---|
| 3.1 | -F | 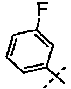 | -CH₂-NMe₂ | VII.5 | C₂₄H₂₁F₂N₃O | 404 [M-H]⁻ | 225-227 | 0.20 (A) |
| | | | | IX.4 | | | | |
| 3.2 | -F | 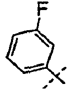 | -N(COMe)-(CH₂)₃-NMe₂ | VII.5 | C₂₈H₂₈F₂N₄O₂ | 491 [M+H]⁺ | 160-163 | 0.20 (A) |
| | | | | IX.7 | | | | |
| 3.3 | -F | 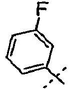 | 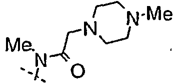 | VII.5 | C₂₉H₂₉F₂N₅O₂ | 518 [M+H]⁺ | 218-220 | 0.40 (A) |
| | | | | IX.19 | | | | |
| 3.4 | -F | 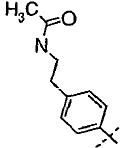 | -CH₂-NMe₂ | VII.6 | C₂₈H₂₉FN₄O₂ | 471 [M-H]⁻ | 106-110 | 0.25 (A) |
| | | | | IX.4 | | | | |
| 3.5 | -F | 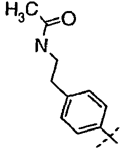 | -N(COMe)-(CH₂)₃-NMe₂ | VII.6 | C₃₂H₃₆FN₅O₃ | 558 [M+H]⁺ | 194-196 | 0.25 (A) |
| | | | | IX.7 | | | | |
| 3.6 | -F | 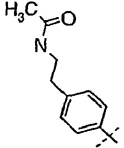 | 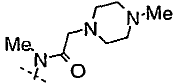 | VII.6 | C₃₃H₃₇FN₆O₃ | 583 [M-H]⁻ | 238-240 | 0.25 (A) |
| | | | | IX.19 | | | | |
| 3.7 | -F | 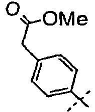 | -CH₂-NMe₂ | VII.1 | C₂₇H₂₆FN₃O₃ | 460 [M+H]⁺ | 173-176 | 0.30 (A) |
| | | | | IX.4 | | | | |
| 3.8 | -F | 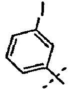 | -CH₂-NMe₂ | VII.13 | C₂₄H₂₁FIN₃O | 514 [M+H]⁺ | 198-200 | 0.30 (B) |
| | | | | IX.4 | | | | |
| 3.9 | -F | 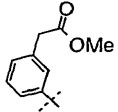 | -CH₂-NMe₂ | VII.7 | C₂₇H₂₆FN₃O₃ | 458 [M-H]⁻ | 195-198 | 0.25 (A) |
| | | | | IX.4 | | | | |
| 3.10 | -F | 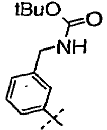 | -CH₂-NMe₂ | VII.8 | C₃₀H₃₃FN₄O₃ | 517 [M+H]⁺ | 230-240 | 0.30 (A) |
| | | | | IX.4 | | | | |
| 3.11 | -F | 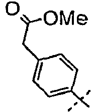 | -N(SO₂Me)-(CH₂)₂-NMe₂ | VII.1 | C₂₉H₃₁FN₄O₅S | 567 [M+H]⁺ | 188-189 | 0.40 (A) |
| | | | | IX.2 | | | | |
| 3.12 | -F | 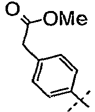 | 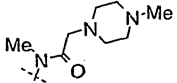 | VII.1 | C₃₂H₃₄FN₅O₄ | 572 [M+H]⁺ | 200-203 | 0.35 (C) |
| | | | | IX.19 | | | | |
| 3.13 | -F | 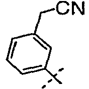 | -CH₂-NMe₂ | VII.9 | C₂₆H₂₃FN₄O | 427 [M+H]⁺ | 130-135 | 0.25 (A) |
| | | | | IX.4 | | | | |
| 3.14 | -F | 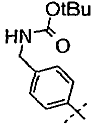 | 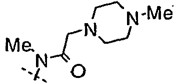 | VII.10 | C₃₅H₄₁FN₆O₄ | 629 [M+H]⁺ | 215-220 | 0.35 (A) |
| | | | | IX.19 | | | | |
| 3.15 | -F | 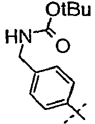 | -CH₂-NMe₂ | VII.10 | C₃₀H₃₃FN₄O₃ | 517 [M+H]⁺ | 186-190 | 0.35 (A) |
| | | | | IX.4 | | | | |
| 3.16 | -F | 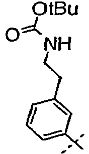 | -CH₂-NMe₂ | VII.17 | C₃₁H₃₅FN₄O₃ | 531 [M+H]⁺ | n. b. | 0.40 (A) |
| | | | | IX.4 | | | | |
| 3.17 | -F | 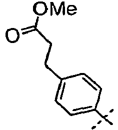 | -NMe-(COMe) | VII.15 | C₂₈H₂₆FN₃O₄ | 488 [M+H]⁺ | 166-170 | 0.40 (A) |
| | | | | - | | | | |
| 3.18 | -F | 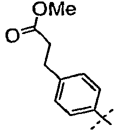 | 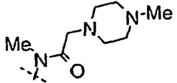 | VII.15 | C₃₃H₃₆FN₅O₄ | 586 [M+H]⁺ | 176-180 | 0.30 (A) |
| | | | | IX.19 | | | | |
| 3.19 | -F | 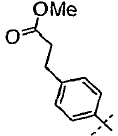 | -N(SO₂Me)-(CH₂)₂-NMe₂ | VII.15 | C₃₀H₃₃FN₄O₅S | 581 [M+H]⁺ | 195-198 | 0.45 (A) |
| | | | | IX.2 | | | | |
| 3.20 | -F | 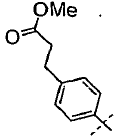 | -N(COMe)- | VII.15 IX.7 | C₃₂H₃₅FN₄O₄ | 559 [M+H]⁺ | 100-104 | 0.50 (A) |
| | | | (CH₂)₃-NMe₂ | | | | | |
| 3.21 | -F | 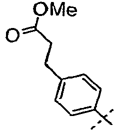 | 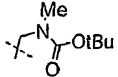 | VII.15 | C₃₂H₃₄FN₃O₅ | 558 [M-H]⁻ | 132-137 | 0.80 (D) |
| | | | | IX.18 | | | | |
| 3.22 | -F | 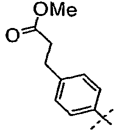 | 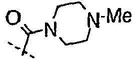 | VII.15 | C₃₁H₃₁FN₄O₄ | 543 [M+H]⁺ | 234-236 | 0.60 (A) |
| | | | | IX.30 | | | | |
| 3.23 | -F | 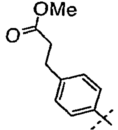 | 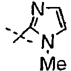 | VII.15 | C₂₉H₂₅FN₄O₃ | 497 [M+H]⁺ | 110-115 | 0.40 (A) |
| | | | | IX.16 | | | | |
| 3.24 | -F | 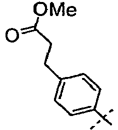 | -SO₂Me | VII.15 | C₂₆H₂₃FN₂O₅S | 495 [M+H]⁺ | 130-137 | 0.60 (A) |
| | | | | - | | | | |
| 3.25 | -F | 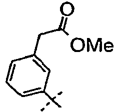 | 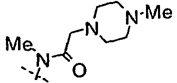 | VII.7 | C₃₂H₃₄FN₅O₄ | 572 [M+H]⁺ | 189 | 0.60 (B) |
| | | | | IX.19 | | | | |
| 3.26 | -F | 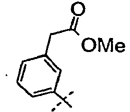 | -N(SO₂Me)-(CH₂)₂-NMe₂ | VII.7 | C₂₉H₃₁FN₄O₅S | 567 [M+H]⁺ | n. b. | 0.60 (B) |
| | | | | IX.2 | | | | |
| 3.27 | -F | 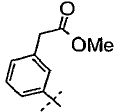 | 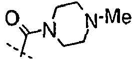 | VII.7 | C₃₀H₂₉FN₄O₄ | 529 [M+H]⁺ | 201-203 | 0.60 (B) |
| | | | | IX.30 | | | | |
| 3.28 | -F | 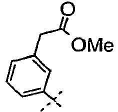 | -N(Me)-(CO)-CH₂-NMe₂ | VII.7 | C₂₉H₂₉FN₄O₄ | 517 [M+H]⁺ | 126 | 0.60 (B) |
| | | | | IX.10 | | | | |
| 3.29 | -F | 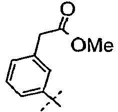 | -N(COMe)-(CH₂)₂-NMe₂ | VII.7 | C₃₀H₃₁FN₄O₄ | 531 [M+H]⁺ | 179 | 0.50 (B) |
| | | | | IX.6 | | | | |
| 3.30 | -F | 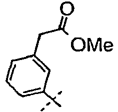 | -N(COMe)-(CH₂)₃-NMe₂ | VII.7 | C₃₁H₃₃FN₄O₄ | 545 [M+H]⁺ | 123 | 0.20 (B) |
| | | | | IX.7 | | | | |
| 3.31 | -F | 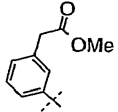 | -N(Me)-(CO)-(CH₂)₄-NMe₂ | VII.7 | C₃₂H₃₅FN₄O₄ | 559 [M+H]⁺ | 201 | 0.20 (B) |
| | | | | IX.32 | | | | |
| 3.32 | -F | 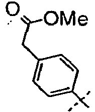 | -H | VII.1 | C₂₄H₁₉FN₂O₃ | 403 [M+H]⁺ | 198-206 | 0.80 (A) |
| | | | | - | | | | |
| 3.33 | -F | 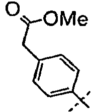 | 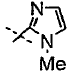 | VII.1 | C₂₈H₂₃FN₄O₃ | 483 [M+H]⁺ | 223-226 | 0.75 (A) |
| | | | | IX.16 | | | | |
| 3.34 | -F | 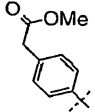 | 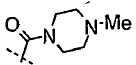 | VII.1 | C₃₀H₂₉FN₄O₄ | 529 [M+H]⁺ | 215-220 | 0.30 (A) |
| | | | | IX.30 | | | | |
| 3.35 | -F | 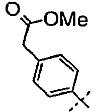 | -N(SO₂Me)-(CH₂)-(CO)-NMe₂ | VII.1 | C₂₉H₂₉FN₄O₆S | 581 [M+H]⁺ | 227-230 | 0.65 (A) |
| | | | | IX.8 | | | | |
| 3.36 | -F | 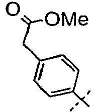 | -N(Me)-(CO)-CH₂-NMe₂ | VII.1 | C₂₉H₂₉FN₄O₄ | 517 [M+H]⁺ | 128-130 | 0.45 (A) |
| | | | | IX.10 | | | | |
| 3.37 | -F | 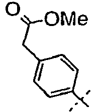 | -N(COMe)-CH₃ | VII.1 | C₂₇H₂₄FN₃O₄ | 474 [M+H]⁺ | 218-223 | 0.40 (A) |
| | | | | - | | | | |
| 3.38 | -F | 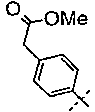 | -N(Me)-(CO)-(CH₂)₂-NMe₂ | VII.1 | C₃₀H₃₁FN₄O₄ | 531 [M+H]⁺ | 192-194 | 0.40 (A) |
| | | | | IX.11 | | | | |
| 3.39 | -F | 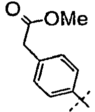 | -SO₂Me | VII.1 | C₂₅H₂₁FN₂O₅S | 481 [M+H]⁺ | 205-214 | 0.65 (A) |
| | | | | - | | | | |
| 3.40 | -F | 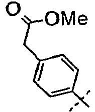 | -N(Me)-(CO)-(CH₂)₃-NMe₂ | VII.1 | C₃₁H₃₃FN₄O₄ | 545 [M+H]⁺ | 190-193 | 0.15 (A) |
| | | | | IX.33 | | | | |
| 3.41 | -F | 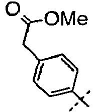 | -N(COMe)-(CH₂)₃-NMe₂ | VII.1 | C₃₁H₃₃FN₄O₄ | 545 [M+H]⁺ | 184-188 | 0.50 (A) |
| | | | | IX.7 | | | | |
| 3.42 | -F | 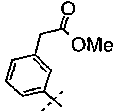 | -H | VII.7 | C₂₄H₁₉FN₂O₃ | 403 [M+H]⁺ | 114 | 0.70 (B) |
| | | | | - | | | | |
| 3.43 | -F | 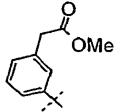 | -SO₂Me | VII.7 | C₂₅H₂₁FN₂O₅S | 481 [M+H]⁺ | 129 | 0.60 (B) |
| | | | | - | | | | |
| 3.44 | -F | 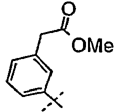 | 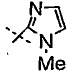 | VII.7 | C₂₈H₂₃FN₄O₃ | 483 [M+H]⁺ | 125 | 0.60 (B) |
| | | | | IX.16 | | | | |
| 3.45 | -F | 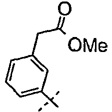 | -N(SO₂Me)-(CH₂)-(CO)-NMe₂ | VII.7 | C₂₉H₂₉FN₄O₆S | 581 [M+H]⁺ | 163 | 0.60 (B) |
| | | | | IX.8 | | | | |
| 3.46 | -F | 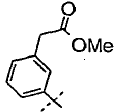 | -N(Me)-(CO)-(CH₂)₃-NMe₂ | VII.7 | C₃₁H₃₃FN₄O₄ | 545 [M+H]⁺ | 101 | 0.10 (B) |
| | | | | IX.33 | | | | |
| 3.47 | -F | 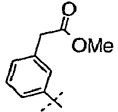 | -N(Me)-(CO)-(CH₂)₂-NMe₂ | VII.7 | C₃₀H₃₁FN₄O₄ | 531 [M+H]⁺ | 161 | 0.20 (B) |
| | | | | IX.11 | | | | |
| 3.48 | -F | 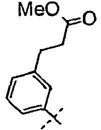 | 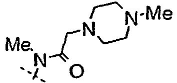 | VII.14 | C₃₀H₃₁FN₄O₄ | 586 [M+H]⁺ | 181-183 | 0.20 (B) |
| | | | | IX.19 | | | | |
| 3.49 | -F | 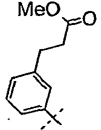 | -N(SO₂Me)-(CH₂)₂-NMe₂ | VII.14 | C₃₀H₃₃FN₄O₅S | 581 [M+H]⁺ | 158-160 | 0.35 (B) |
| | | | | IX.2 | | | | |
| 3.50 | -F | 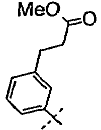 | -N(Me)-(CO)-CH₂-NMe₂ | VII.14 | C₃₀H₃₁FN₄O₄ | 531 [M+H]⁺ | n. b. | 0.40 (B) |
| | | | | IX.10 | | | | |
| 3.51 | -F | 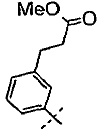 | -N(COMe)-(CH₂)₃-NMe₂ | VII.14 | C₃₂H₃₅FN₄O₄ | 559 [M+H]⁺ | n. b. | 0.50 (E) |
| | | | | IX.7 | | | | |
| 3.52 | -F | 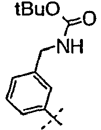 | 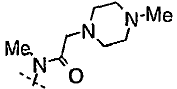 | VII.8 | C₃₅H₄₁FN₆O₄ | 629 [M+H]⁺ | n. b. | 0.35 (A) |
| | | | | IX.19 | | | | |
| 3.53 | -F | 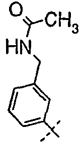 | -NMe-(CO)-CH₃ | VII.26 | C₂₇H₂₅FN₄O₃ | 473 [M+H]⁺ | 122-126 | 0.50 (F) |
| | | | | - | | | | |
| 3.54 | -F | 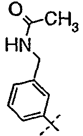 | -N(COMe)-(CH₂)₃-NMe₂ | VII.26 | C₃₁H₃₄FN₅O₃ | 544 [M+H]⁺ | 80-83 | 0.25 (A) |
| | | | | IX.7 | | | | |
| 3.55 | -F | 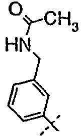 | -N(SO₂Me)-(CH₂)₂-NMe₂ | VII.18 | C₂₉H₃₂FN₅O₄S | 566 [M+H]⁺ | 190-195 | 0.30 (A) |
| | | | | IX.2 | | | | |
| 3.56 | -F | 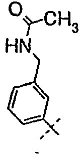 | -N(Me)-(CO)-CH₂-NMe₂ | VII.18 | C₂₉H₃₀FN₅O₃ | 516 [M+H]⁺ | 238-241 | 0.30 (G) |
| | | | | IX.10 | | | | |
| 3.57 | -F | 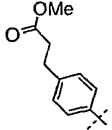 | -(CH₂)₂-NMe₂ | VII.15 | C₂₉H₃₀FN₃O₃ | 488 [M+H]⁺ | 205-208 | 0.55 (G) |
| | | | | IX.5 | | | | |
| 3.58 | -F | 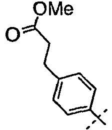 | -N(Me)-(CO)-(CH₂)₂-NMe₂ | VII.15 | C₃₁H₃₁FN₄O₄ | 543 [M-H]⁻ | 196-202 | 0.20 (A) |
| | | | | IX.11 | | | | |
| 3.59 | -F | 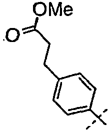 | -N(Me)-(CO)-CH₂-NMe₂ | VII.15 | C₃₀H₃₁FN₄O₄ | 531 [M+H]⁺ | 177-182 | 0.30 (A) |
| | | | | IX.10 | | | | |
| 3.60 | -F | 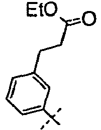 | -(CH₂)₂-NMe₂ | VII.19 | C₃₀H₃₂FN₃O₃ | 500 [M-H]⁻ | 100-105 | 0.35 (B) |
| | | | | IX.5 | | | | |
| 3.61 | -F | 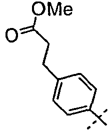 | -N(COMe)-(CH₂)₂-NMe₂ | VII.15 | C₃₁H₃₃FN₄O₄ | 545 [M+H]⁺ | 167-169 | 0.40 (A) |
| | | | | IX.6 | | | | |
| 3.62 | -F | 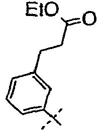 | -N(Me)-(CO)-(CH₂)₃-NMe₂ | VII.19 | C₃₃H₃₇FN₄O₄ | 571 [M-H]⁻ | n.b. | 0.35 (A) |
| | | | | IX.33 | | | | |
| 3.63 | -F | 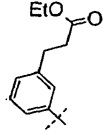 | -N(Me)-(CO)-(CH₂)₄-NMe₂ | VII.19 | C₃₄H₃₉FN₄O₄ | 585 [M-H]⁻ | n.b. | 0.40 (A) |
| | | | | IX.32 | | | | |
| 3.64 | -F | 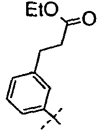 | 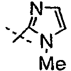 | VII.19 | C₃₀H₂₇FN₄O₃ | 511 [M+H]⁺ | 95-105 | 0.25 (B) |
| | | | | IX.16 | | | | |
| 3.65 | -F | 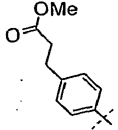 | -N(Me)-(CO)-(CH₂)₄-NMe₂ | VII.15 | C₃₃H₃₇FN₄O₄ | 573 [M+H]⁺ | 173-175 | 0.20 (A) |
| | | | | IX.32 | | | | |
| 3.66 | -F | 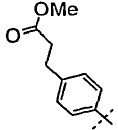 | -H | VII.15 | C₂₅H₂₁FN₂O₃ | 417 [M+H]⁺ | 168-174 | 0.65 (A) |
| | | | | - | | | | |
| 3.67 | -F | 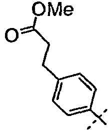 | 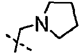 | VII.15 | C₃₀H₃₀FN₃O₃ | 500 [M+H]⁺ | 168-173 | 0.40 (B) |
| | | | | IX.22 | | | | |
| 3.68 | -F | 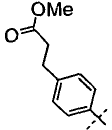 | -CH₂-NEt₂ | VII.15 | C₃₀H₃₂FN₃O₃ | 502 [M+H]⁺ | n.b. | 0.45 (B) |
| | | | | IX.1 | | | | |
| 3.69 | -F | 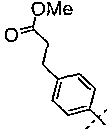 | 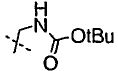 | VII.15 | C₃₁H₃₂FN₃O₅ | 544 [M-H]⁻ | n.b. | 0.30 (G) |
| | | | | IX.12 | | | | |
| 3.70 | -F | 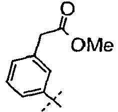 | -(CH₂)₂-NMe₂ | VII.7 | C₂₈H₂₈FN₃O₃ | 472 [M-H]⁻ | 165-170 | 0.25 (B) |
| | | | | IX.5 | | | | |
| 3.71 | -F | 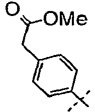 | -(CH₂)₂-NMe₂ | VII.1 | C₂₈H₂₈FN₃O₃ | 472 [M-H]⁻ | 193-197 | 0.25 (B) |
| | | | | IX.5 | | | | |
| 3.72 | -F | 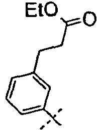 | -CH₂-NMe₂ | VII.19 | C₂₉H₃₀FN₃O₃ | 488 [M+H]⁺ | 48-52 | 0.45 (B) |
| | | | | IX.4 | | | | |
| 3.73 | -Cl | 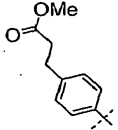 | -(CH₂)₂-NMe₂ | VII.20 | C₂₉H₃₀ClN₃O₃ | 504/506 [M+H]⁺ | 156-160 | 0.30 (H) |
| | | | | IX.5 | | | | |
| 3.74 | -Cl | 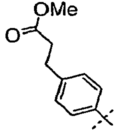 | 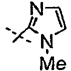 | VII.20 | C₂₉H₂₅ClN₄O₃ | 513/515 [M+H]⁺ | 110 | 0.40 (H) |
| | | | | IX.16 | | | | |
| 3.75 | -Cl | 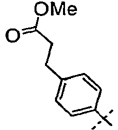 | -CH₂-NMe₂ | VII.20 | C₂₈H₂₈ClN₃O₃ | 490/492 [M+H]⁺ | 173-175 | 0.70 (I) |
| | | | | IX.4 | | | | |
| 3.76 | -F | 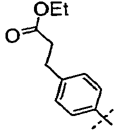 | -CH₂-NMe₂ | VII.21 | C₂₉H₃₀FN₃O₃ | 488 [M+H]⁺ | 158-161 | 0.35 (B) |
| | | | | IX.4 | | | | |
| 3.77 | -F | 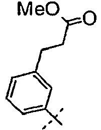 | 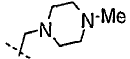 | VII.14 | C₃₁H₃₃FN₄O₃ | 529 [M+H]⁺ | 147-150 | 0.50 (I) |
| | | | | IX.14 | | | | |
| 3.78 | -F | 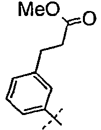 | 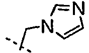 | VII.14 | C₂₉H₂₅FN₄O₃ | 497 [M+H]⁺ | 182-185 | 0.60 (K) |
| | | | | IX.15 | | | | |
| 3.79 | -F | 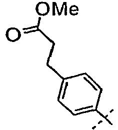 | 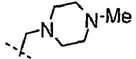 | VII.15 | C₃₁H₃₃FN₄O₃ | 529 [M+H]⁺ | 184 | 0.35 (B) |
| | | | | IX.14 | | | | |
| 3.80 | -F | 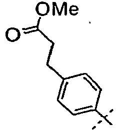 | 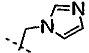 | VII.15 | C₂₉H₂₅FN₄O₃ | 497 [M+H]⁺ | 233 | 0.45 (B) |
| | | | | IX.15 | | | | |
| 3.81 | -F | 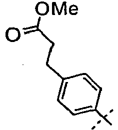 | -CH₂-NMe-(CH₂)₂-NMe₂ | VII.15 | C₃₁H₃₅FN₄O₃ | 531 [M+H]⁺ | 120 | 0.40 (B) |
| | | | | IX.17 | | | | |
| 3.82 | -F | 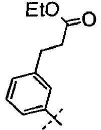 | -CH₂-NMe-(CH₂)₂-NMe₂ | VII.19 | C₃₂H₃₇FN₄O₃ | 545 [M+H]⁺ | n.b. | 0.40 (K) |
| | | | | IX.17 | | | | |
| 3.83 | -Cl | 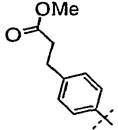 | 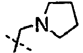 | VII.20 | C₃₀H₃₀ClN₃O₃ | 516/518 [M+H]⁺ | 195-197 | 0.30 (H) |
| | | | | IX.22 | | | | |
| 3.84 | -F | 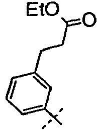 | -H | VII.19 | C₂₆H₂₃FN₂O₃ | 431 [M+H]⁺ | 156-160 | 0.80 (M) |
| | | | | - | | | | |
| 3.85 | -F | 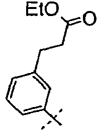 | 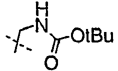 | VII.19 | C₃₂H₃₄FN₃O₅ | 560 [M+H]⁺ | n.b. | 0.50 (L) |
| | | | | IX.12 | | | | |
| 3.86 | -F | 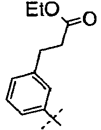 | 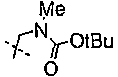 | VII.19 | C₃₃H₃₆FN₃O₅ | 574 [M+H]⁺ | n.b. | 0.60 (L) |
| | | | | IX.18 | | | | |
| 3.87 | -F | 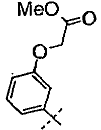 | -CH₂-NMe₂ | VII.22 | C₂₇H₂₆FN₃O₄ | 476 [M+H]⁺ | 129 | 0.25 (B) |
| | | | | IX.4 | | | | |
| 3.88 | -F | 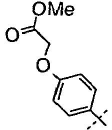 | -CH₂-NMe₂ | VII.23 | C₂₇H₂₆FN₃O₄ | 476 [M+H]⁺ | 155 | 0.25 (B) |
| | | | | IX.4 | | | | |
| 3.89 | -F | 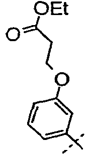 | -CH₂-NMe₂ | VII.24 | C₂₉H₃₀FN₃O₄ | 504 [M+H]⁺ | n.b. | 0.20 (B) |
| | | | | IX.4 | | | | |
| 3.90 | -Br | 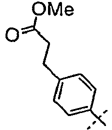 | 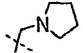 | VII.26 | C₃₀H₃₀BrN₃O₃ | 560/562 [M+H]⁺ | 230-235 | 0.45 (B) |
| | | | | IX.22 | | | | |
| 3.91 | -Br | 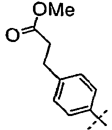 | -CH₂-NMe₂ | VII.26 | C₂₈H₂₈BrN₃O₃ | 534/536 [M+H]⁺ | 178-180 | 0.35 (B) |
| | | | | IX.4 | | | | |
| 3.92 | -Br | 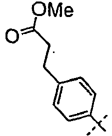 | -CH₂-NEt₂ | VII.26 | C₃₀H₃₂BrN₃O₃ | 562/564 [M+H]⁺ | 173-176 | 0.40 (B) |
| | | | | IX.1 | | | | |

| | | | | | | | | |
|---|---|---|---|---|---|---|---|---|
| *Fließmittelgemische: (A): Kieselgel, Methylenchlorid/Methanol/Ammoniak 9:1:0.1 (B): Kieselgel, Methylenchlorid/Methanol 9:1 (C): Kieselgel, Methylenchlorid/Methanol/Ammoniak 8:1:0.1 (D): Kieselgel, Methylenchlorid/Methanol/Ammoniak 10:1:0.1 (E): Kieselgel, Methylenchlorid/Methanol/Ammoniak 5:1:0.01 (F): Kieselgel, Essigester/Methanol/Ammoniak = 9:1:0,1 (G): Aluminiumoxid, Methylenchlorid/Methanol = 19:1 (H): Kieselgel, Methylenchlorid/Methanol/Ammoniak 9:1:0.01 (I): Kieselgel, Methylenchlorid/Methanol 5:1 (K): Aluminiumoxid, Methylenchlorid/Ethanol = 20:1 (L): Kieselgel, Petrolether/Essigester 1:1 (M): Kieselgel, Petrolether/Essigester 1:2 | | | | | | | | |

Analog Beispiel 3.0 werden folgende Verbindungen der allgemeinen Formel I-3b hergestellt:

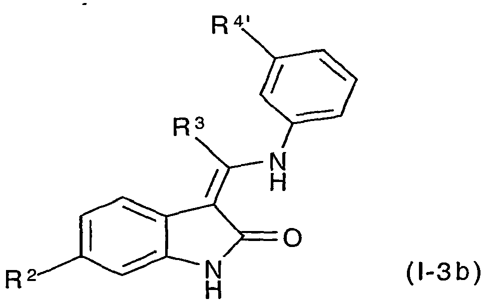

| Beispiel | R² | R³ | R⁴' | Edukte | Summenformel | Massenspektrum | Fp. [°C] | R_{f}-Wert* |
|---|---|---|---|---|---|---|---|---|
| 3.93 | -F | 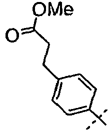 | -CH₂-NMe₂ | VII.15 | C₂₈H₂₈FN₃O₃ | 474 [M+H]⁺ | 176-179 | 0.40 (A) |
| | | | | IX.3 | | | | |
| 3.94 | -F | 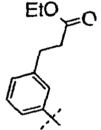 | -CH₂-NMe₂ | VII.19 | C₂₉H₃₀FN₃O₃ | 486 [M-H]⁻ | n.b. | 0.45 (B) |
| | | | | IX.3 | | | | |
| 3.95 | -Cl | 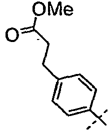 | -CH₂-NMe₂ | VII.20 | C₂₈H₂₈ClN₃O₃ | 490/492 [M+H]⁺ | 163-165 | 0.40 (A) |
| | | | | IX.3 | | | | |

| | | | | | | | | |
|---|---|---|---|---|---|---|---|---|
| *Fließmittelgemische: (A): Kieselgel, Methylenchlorid/Methanol 9:1 (B): Kieselgel, Methylenchlorid/Methanol/Ammoniak 9:1:0.1 | | | | | | | | |

### Beispiel 4.0

### 3-Z-[1-(4-(Dimethylaminomethyl)-anilino)-1-(3,4-dimethoxy-phenyl)-methylen]-6-cyano-2-indolinon

130 mg 1-Acetyl-3-(1-methoxy-1-(3,4-dimethoxy-phenyl)-methylen)-6-cyano-2-indolinon (Edukt VII.4) und 58 mg 4-(Dimethylaminomethyl)-anilin (Edukt IX.4) werden in 5 ml Dimethylformamid gelöst und 2 Stunden bei 80°C gerührt. Nach dem Abkühlen wird das Lösungsmittel abgezogen und der Rückstand über eine Kieselgelsäule mit Methylenchlorid/Methanol 9:1 als Laufmittel aufgereinigt.
Ausbeute: 21 mg (12% der Theorie),
R_{f}-Wert: 0.35 (Kieselgel, Methylenchlorid/Methanol = 9:1)
Fp. 265 °C
C₂₇H₂₆N₄O₃

### Beispiel 5.0

### 3-Z-[1-(4-(N-Methyl-N-methylsulfonyl-amino)-anilino)-1-(3-(2-methoxycarbonyl-vinyl)-phenyl)-methylen]-6-chlor-2-indolinon

580 mg 3-Z-[1-(4-(*N*-Methyl-*N*-methylsulfonyl-amino)-anilino)-1-(3-iod-phenyl)-methylen]-6-chlor-2-indolinon (Edukt 1.0) und 140 ml Acrylsäuremethylester werden in 20 ml Acetonitril und 11 ml Dimethylformamid gelöst und 11 mg Palladium(II)-acetat, 2 ml Triethylamin und 30 mg Tri-ortho-tolyl-phosphin zugegeben. Die Lösung wird für 10 Stunden bei 90°C unter Stickstoff als Schutzgas gerührt. Nach dem Abkühlen wird über Celite filtriert, das Lösungsmittel abgezogen und der Rückstand über eine Kieselgelsäule mit Methylenchlorid/Methanol 20:1 als Laufmittel aufgereinigt.
Ausbeute: 450 mg (84% der Theorie),
R_{f}-Wert: 0.30 (Kieselgel, Toluol/Essigester = 1:1)
Fp. 228-232 °C
C₂₇H₂₄ClN₃O₅S
Massenspektrum: m/z = 537/539 [M]⁺

Analog Beispiel 5.0 werden folgende Verbindungen der allgemeinen Formel I-5 hergestellt:

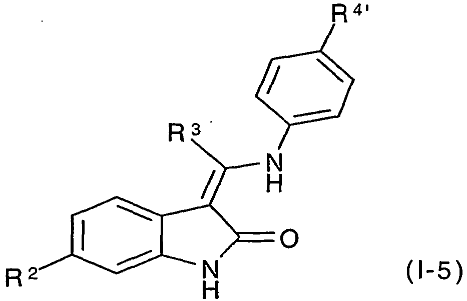

| Beispiel | R² | R³ | R⁴' | Edukt | Summenformel | Massenspektrum | Fp. [°C] | R_{f}-Wert* |
|---|---|---|---|---|---|---|---|---|
| 5.1 | -Cl | 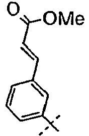 | -CH₂-NMe₂ | 1.1 | C₂₈H₂₆ClN₃O₃ | 486/488 [M-H]⁻ | 150-155 | 0.50 (A) |
| 5.2 | -F | 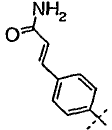 | -CH₂-NMe₂ | 3.0 | C₂₇H₂₅FN₄O₂ | 455 [M-H]⁻ | 269-270 | 0.20 (B) |
| 5.3 | -F | 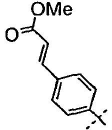 | -CH₂-NMe₂ | 3.0 | C₂₈H₂₆FN₃O₃ | 470 [M-H]⁻ | 205-208 | 0.65 (A) |
| 5.4 | -F | 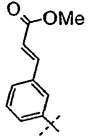 | -CH₂-NMe₂ | 1.1 | C₂₈H₂₆FN₃O₃ | 472 [M+H]⁺ | 138-140 | 0.45 (A) |

| | | | | | | | | |
|---|---|---|---|---|---|---|---|---|
| *Fließmitteigemische: (A): Kieselgel, Methylenchlorid/Methanol 5:1 (B): Kieselgel, Methylenchlorid/Methanol/Ammoniak 9:1:0,01 | | | | | | | | |

### Beispiel 6.0

### 3-Z-[1-(4-Dimethylaminomethyl-anilino)-1-(3-(2-methoxycarbonyl-ethyl)-phenyl)-methylen]-6-chlor-2-indolinon

1.0 g 3-Z-[1-(4-(Dimethylaminomethyl)-anilino)-1-(3-(2-methoxycarbonyl-vinyl)-phenyl)-methylen]-6-chlor-2-indolinon (Edukt 5.1) werden in 100 ml Methanol gelöst und 200 mg 10-prozentiges Palladium/Kohlenstoff als Katalysator zugegeben. Anschließend wird für 6 Stunden bei Raumtemperatur und 50 psi Wasserstoffdruck hydriert. Nach Reaktionsende wird der Katalysator abfiltriert, das Lösungsmittel abgezogen und der Rückstand im Vakuum bei 100°C getrocknet.
Ausbeute: 900 mg (90% der Theorie),
R_{f}-Wert: 0.40 (Kieselgel, Methylenchlorid/Methanol = 9:1)
Fp. 160 °C
C₂₈H₂₈ClN₃O₃
Massenspektrum: m/z = 490/492 [M+H]⁺

Analog Beispiel 6.0 werden folgende Verbindungen der allgemeinen Formel I-6 hergestellt:

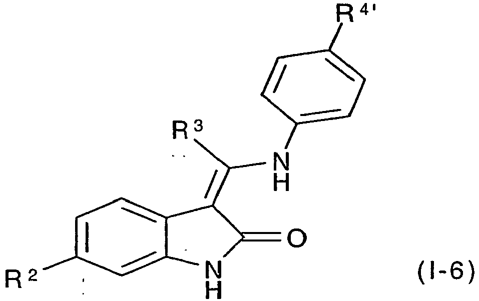

| Beispiel | R² | R³ | R⁴' | Edukt | Summenformel | Massenspektrum | Fp. [°C] | R_{f}Wert* |
|---|---|---|---|---|---|---|---|---|
| 6.1 | -Cl | 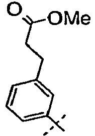 | -N(Me)-SO₂Me | 5.0 | C₂₇H₂₆ClN₃O₅S | 538/540 [M-H]⁻ | 148-150 | 0.50 (A) |
| 6.2 | -F | 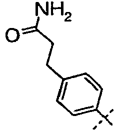 | -CH₂-NMe₂ | 5.2 | C₂₇H₂₇FN₄O₂ | 459 [M+H]⁺ | 150 | 0.70 (B) |
| 6.3 | -F | 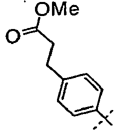 | -CH₂-NMe₂ | 5.3 | C₂₈H₂₈FN₃O₃ | 474 [M+H]⁺ | 140 | 0.35 (A) |
| 6.4 | -F | 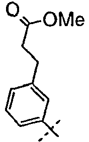 | -CH₂-NMe₂ | 5.4 | C₂₈H₂₈FN₃O₃ | 474 [M+H]⁺ | 140-142 | 0.30 (A) |

| | | | | | | | | |
|---|---|---|---|---|---|---|---|---|
| *Fließmittelgemische: (A): Kieselgel, Methylenchlorid/Methanol 9:1 (B): Kieselgel, Methylenchlorid/Methanol/Ammoniak 5:1:0,01 | | | | | | | | |

### Beispiel 7.0

### 3-Z-[1-(4-Dimethylaminomethyl-anilino)-1-(4-aminomethyl-phenyl)-methylen]-6-chlor-2-indolinon

900 mg 3-Z-[1-(4-Dimethylaminomethyl-anilino)-1-(4-cyano-phenyl)-methylen]-6-chlor-2-indolinon (Edukt 2.0) werden in 20 ml Methylenchlorid gelöst, 30 ml methanolischer Ammoniak zugegeben und 200 mg Raney-Nickel als Katalysator zugesetzt. Anschließend wird für 2 Stunden 15 Minuten bei Raumtemperatur und 50 psi Wasserstoffdruck hydriert. Nach Reaktionsende wird der Katalysator abfiltriert, das Lösungsmittel abgezogen und der Rückstand mit wenig Methanol und Diethylether gewaschen. Zur Freisetzung der Base wird der Rückstand in 1 N Natronlauge aufgenommen und viermal mit Methylenchlorid/Methanol 9:1 extrahiert. Die vereinigten organischen Phasen werden mit Wasser gewaschen und über Natriumsulfat getrocknet. Das Produkt wird mit wenig Diethylether gewaschen und im Vakuum getrocknet.
Ausbeute: 680 mg (75% der Theorie),
R_{f}-Wert: 0.60 (Kieselgel, Methylenchlorid/Methanol/Ammoniak = 9:1:0.1)
Fp. 211-214 °C
C₂₅H₂₅ClN₄O
Massenspektrum: m/z = 433/435 [M+H]⁺

### Beispiel 8.0

### 3-Z-[1-(4-(N-((4-Methyl-piperazin-1-yl)-methylcarbonyl)-N-methyl-amino)-anilino)-1-(4-aminomethyl-phenyl)-methylen]-6-chlor-2-indolinon

1,39 g 1-Acetyl-3-Z-[1-(4-(N-((4-Methyl-piperazin-1-yl)-methylcarbonyl)-N-methyl-amino)-anilino)-1-(4-cyano-phenyl)-methylen]-6-chlor-2-indolinon werden in 20 ml Methylenchlorid gelöst, 30 ml methanolischer Ammoniak zugegeben und 200 mg Raney-Nickel als Katalysator zugesetzt. Anschließend wird für 2 Stunden bei Raumtemperatur und 50 psi Wasserstoffdruck hydriert. Nach Reaktionsende wird der Katalysator abfiltriert, das Lösungsmittel abgezogen und der Rückstand mit wenig Methanol und Diethylether gewaschen. Zur Freisetzung der Base wird der Rückstand in 1 N Natronlauge aufgenommen und viermal mit Methylenchlorid/Methanol 9:1 extrahiert. Die vereinigten organischen Phasen werden mit Wasser gewaschen und über Natriumsulfat getrocknet. Das Produkt wird über eine Kieselgelsäule mit einem Gradienten von Methylenchlorid und Methylenchlorid/Methanol/Ammoniak 8:1:0,1 als Laufmittel aufgereinigt. Das Produkt wird mit wenig Diethylether gewaschen und im Vakuum getrocknet.
Ausbeute: 700 mg (54% der Theorie),
R_{f}-Wert: 0.15.(Kieselgel, Methylenchlorid/Methanol/Ammoniak = 9:1:0.1)
Fp. 232-235 °C
C₃₀H₃₃ClN₆O₂
Massenspektrum: m/z = 544/546 [M]⁺

### Beispiel 9.0

### 3-Z-[1-(4-(Dimethylaminomethyl)-anilino)-1-(3-aminomethyl-phenyl)-methylen]-6-fluor-2-indolinon

2.72 g 3-Z-[1-(4-(Dimethylaminomethyl)-anilino)-1-(3-(N-tert.butoxycarbonylaminomethyl)-phenyl)-methylen]-6-fluor-2-indolinon (Edukt 3.10) werden in 50 ml Methylenchlorid gelöst und 10 ml Trifluoressigsäure zugegeben. Der Ansatz wird für 3 Stunden bei Raumtemperatur gerührt. Nach dieser Zeit wird das Lösungsmittel weitgehend abgezogen, der Rückstand in Essigester aufgenommen und zweimal mit 1 N Natronlauge gewaschen. Die organische Phase wird über Natriumsulfat getrocknet, das Lösungsmittel einrotiert und der Rückstand über eine Kieselgelsäule mit Methylenchlorid/Methanol/Ammoniak 9:1:0,1 als Laufmittel aufgereinigt. Das Produkt wird mit wenig Diethylether gewaschen und im Vakuum getrocknet.
Ausbeute: 1,77 g (81 % der Theorie),
R_{f}-Wert: 0.25 (Kieselgel, Methylenchlorid/Methanol/Ammoniak 9:1:0,1)
Fp. 168-175 °C
C₂₅H₂₅FN₄O
Massenspektrum: m/z = 415 [M-H]⁻

Analog Beispiel 9.0 werden folgende Verbindungen der allgemeinen Formel I-9 hergestellt:

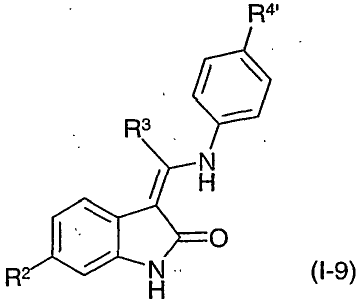

| Beispiel | R² | R³ | R⁴' | Edukt | Summenformel | Massenspektrum | Fp. [°C] | R_{f}-Wert* |
|---|---|---|---|---|---|---|---|---|
| 9.1 | -F | 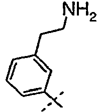 | -CH₂-NMe₂ | 3.16 | C₂₆H₂₇FN₄O | 431 [M+H]⁺ | 155-160 | 0.45 (C) |
| 9.2 | -F | 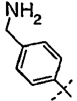 | -CH₂-NMe₂ | 3.15 | C₂₅H₂₅FN₄O | 417 [M+H]⁺ | 203-207 | 0.25 (A) |
| 9.3 | -F | 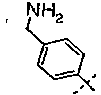 | 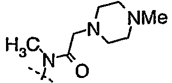 | 3.14 | C₃₀H₃₃FN₆O₂ | 529 [M+H]⁺ | 170-175 | 0.15 (A) |
| 9.4 | -F | 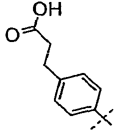 | -CH₂-NHMe | 10.11 | C₂₆H₂₄FN₃O₃ | 446 [M+H]⁺ | 245-251 | 0.20 (D) |
| 9.5 | -F | 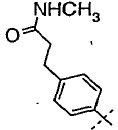 | -CH₂-NHMe | 11.22 | C₂₆H₂₄FN₃O₃ | 459 [M+H]⁺ | 239-243 | 0.30 (A) |
| 9.6 | -F | 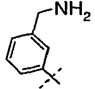 | 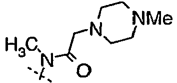 | 3.52 | C₃₀H₃₃FN₆O₂ | 529 [M+H]⁺ | n. b. | n. b. |
| 9.7 | -F | 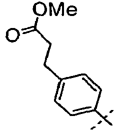 | -CH₂-NH₂ | 3.69 | C₂₆H₂₄FN₃O₃ | 444 [M-H]⁻ | 158-163 | 0.25 (A) |
| 9.8 | -F | 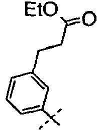 | -CH₂-NH₂ | 3.85 | C₂₇H₂₆FN₃O₃ | 460 [M+H]⁺ | 205-210 | 0.30 (B) |
| 9.9 | -F | 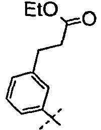 | -CH₂-NHMe | 3.86 | C₂₈H₂₈FN₃O₃ | 474 [M+H]⁺ | 148-150 | 0.30 (B) |

| | | | | | | | | |
|---|---|---|---|---|---|---|---|---|
| *Fließmittelgemische: (A): Kieselgel, Methylenchlorid/Methanol/Ammoniak 9:1:0,1 (B): Kieselgel, Methylenchlorid/Methanol/Ammoniak 9:1:0,01 (C): Kieselgel, Methylenchlorid/Methanol/Ammoniak 8:2:0,2 (D): Reversed Phase RP8, Methanol/Kochsalzlösung(5%) = 3:2 | | | | | | | | |

### Beispiel 10.0

### 3-Z-[1-(4-Dimethylaminomethyl-anilino)-1-(3-(2-carboxy-ethyl)-phenyl)-methylen]-6-chlor-2-indolinon

900 mg 3-Z-[1-(4-Dimethylaminomethyl-anilino)-1-(3-(2-methoxycarbonyl-ethyl)-phenyl)-methylen]-6-chlor-2-indolinon (Edukt 6.0) werden in 10 ml Ethanol gelöst und 5 ml 1 N Natronlauge zugegeben. Der Ansatz wird für 5 Stunden bei Raumtemperatur gerührt. Nach dem Abkühlen werden 5 ml 1 N Salzsäure zugegeben. Der ausgefallene Niederschlag wird abgesaugt und mit Wasser nachgewaschen.
Ausbeute: 830 mg (95% der Theorie),
R_{f}-Wert: 0.50 (Reversed Phase RP8, Methanol/Kochsalzlösung(5%) = 4:1)
Fp. 210-215 °C
C₂₇H₂₆ClN₃O₃
Massenspektrum: m/z = 476/478 [M+H]⁺

Analog Beispiel 10.0 werden folgende Verbindungen der allgemeinen Formel I-10a hergestellt:

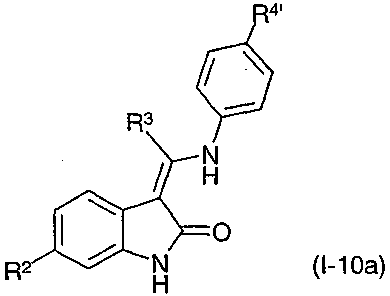

| Beispiel | R² | R³ | R⁴' | Edukt | Summenformel | Massenspektrum | Fp. [°C] | R_{f}-Wert* |
|---|---|---|---|---|---|---|---|---|
| 10.1 | -F | 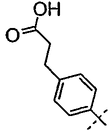 | -CH₂-NMe₂ | 6.3 | C₂₇H₂₆FN₃O₃ | 460 [M+H]⁺ | 250 | 0.65 (A) |
| 10.2 | -F | 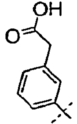 | -CH₂-NMe₂ | 3.9 | C₂₆H₂₄FN₃O₃ | 444 [M-H]⁻ | 278-282 | 0.10 (B) |
| 10.3 | -F | 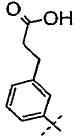 | -CH₂-NMe₂ | 6.4 | C₂₇H₂₆FN₃O₃ | 458 [M-H]⁻ | 198-200 | 0.20 (C) |
| 10.4 | -F | 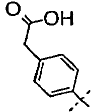 | -CH₂-NMe₂ | 3.7 | C₂₆H₂₄FN₃O₃ | 444 [M-H]⁻ | 212-216 | 0.30 (D) |
| 10.5 | -F | 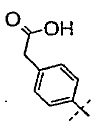 | 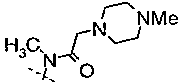 | 3.12 | C₃₁H₃₂FN₅O₄ | 558 [M+H]⁺ | 260-263 | 0.20 (D) |
| 10.6 | -F | 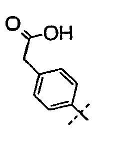 | -N(SO₂Me)-(CH₂)₂-NMe₂ | 3.11 | C₂₈H₂₉FN₄O₅S | 553 [M+H]⁺ | 246-249 | 0.30 (D) |
| 10.7 | -F | 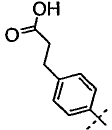 | -NMe-(CO)-CH₃ | 3.17 | C₂₇H₂₄FN₃O₄ | 474 [M+H]⁺ | 286-290 | 0.60 (E) |
| 10.8 | -F | 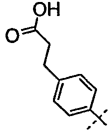 | 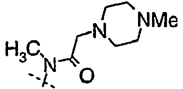 | 3.18 | C₃₂H₃₄FN₅O₄ | 570 [M-H]⁻ | 215-222 | 0.20 (D) |
| 10.9 | -F | 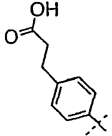 | -N(SO₂Me)-(CH₂)₂-NMe₂ | 3.19 | C₂₉H₃₁FN₄O₅S | 567 [M+H]⁺ | 160-165 | 0.20 (D) |
| 10.10 | -F | 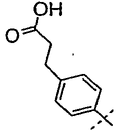 | -N(COMe)-(CH₂)₃-NMe₂ | 3.20 | C₃₁H₃₃FN₄O₄ | 545 [M+H]⁺ | 153-158 | 0.15 (D) |
| 10,11 | -F | 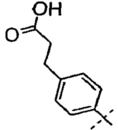 | 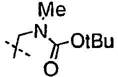 | 3.21 | C₃₁H₃₂FN₃O₅ | 546 [M+H]⁺ | 215-219 | 0.60 (E) |
| 10.12 | -F | 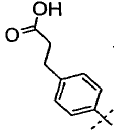 | 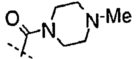 | 3.22 | C₃₀H₂₉FN₄O₄ | 529 [M+H]⁺ | 179-186 | 0.25 (E) |
| 10.13 | -F | 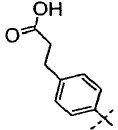 | 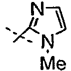 | 3.23 | C₂₈H₂₃FN₄O₃ | 483 [M+H]⁺ | 264-267 | 0.65 (E) |
| 10.14 | -F | 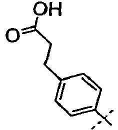 | -SO₂Me | 3.24 | C₂₅H₂₁FN₂O₅S | 481 [M+H]⁺ | 146-155 | 0.70 (E) |
| 10.15 | -F | 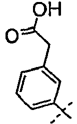 | 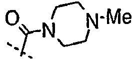 | 3.27 | C₂₉H₂₇FN₄O₄ | 515 [M+H]⁺ | 251 | 0.70 (E) |
| 10.16 | -F | 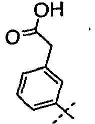 | 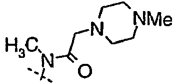 | 3.25 | C₃₁H₃₂FN₅O₄ | 558 [M+H]⁺ | 234 | 0.10 (E) |
| 10.17 | -F | 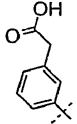 | -N(Me)-(CO)-CH₂-NMe₂ | 3.28 | C₂₈H₂₇FN₄O₄ | 503 [M+H]⁺ | 203 | 0.60 (E) |
| 10.18 | -F | 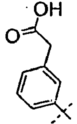 | -N(Me)-(CO)-(CH₂)₄-NMe₂ | 3.31 | C₃₁H₃₃FN₄O₄ | 545 [M+H]⁺ | 251 | n. b. |
| 10.19 | -F | 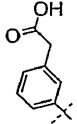 | -H | 3.42 | C₂₃H₁₇FN₂O₃ | 387 [M-H]⁻ | 130 | 0.60 (E) |
| 10.20 | -F | 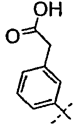 | -SO₂Me | 3.43 | C₂₄H₁₉FN₂O₅S | 467 [M+H]⁺ | 139 | 0.55 (E) |
| 10.21 | -F | 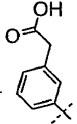 | 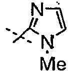 | 3.44 | C₂₇H₂₁FN₄O₃ | 469 [M+H]⁺ | 157 | 0.35 (E) |
| 10.22 | -F | 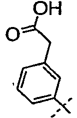 | -N(SO₂Me)-(CH₂)-(CO)-NMe₂ | 3.45 | C₂₈H₂₇FN₄O₆S | 567 [M+H]⁺ | 183 | 0.55 (E) |
| 10.23 | -F | 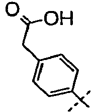 | -H | 3.32 | C₂₃H₁₇FN₂O₃ | 389 [M+H]⁺ | 237-240 | 0.10 (D) |
| 10.24 | -F | 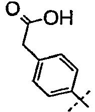 | 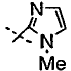 | 3.33 | C₂₇H₂₁FN₄O₃ | 469 [M+H]⁺ | 259-265 | 0.15 (D) |
| 10.25 | -F | 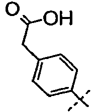 | -N(COMe)-(CH₂)₃-NMe₂ | 3.41 | C₃₀H₃₁FN₄O₄ | 531 [M+H]⁺ | 274-278 | 0.15 (D) |
| 10.26 | -F | 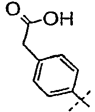 | -N(Me)-(CO)-CH₂-NMe₂ | 3.36 | C₂₈H₂₇FN₄O₄ | 503 [M+H]⁺ | 258-264 | 0.20 (D) |
| 10.27 | -F | 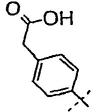 | 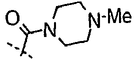 | 3.34 | C₂₉H₂₇FN₄O₄ | 515 [M+H]⁺ | 279-282 | 0.15 (D) |
| 10.28 | -F | 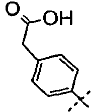 | -SO₂Me | 3.39 | C₂₄H₁₉FN₂O₅S | 467 [M+H]⁺ | 260-266 | 0.35 (F) |
| 10.29 | -F | 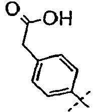 | -N(COMe)-CH₃ | 3.37 | C₂₆H₂₂FN₃O₄ | 460 [M+H]⁺ | 290-294 | 0.30 (F) |
| 10.30 | -F | 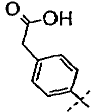 | -N(SO₂Me)-CH₂-(CO)-NMe₂ | 3.35 | C₂₈H₂₇FN₄O₆S | 567 [M+H]⁺ | 238-242 | 0.30 (F) |
| 10.31 | -F | 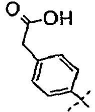 | -N(Me)-(CO)-(CH₂)₂-NMe₂ | 3.38 | C₂₉H₂₉FN₄O₄ | 517 [M+H]⁺ | 250-255 | 0.35 (F) |
| 10.32 | -F | 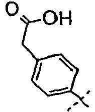 | -N(Me)-(CO)-(CH₂)₃-NMe₂ | 3.40 | C₃₀H₃₁FN₄O₄ | 531 [M+H]⁺ | 184-190 | 0.25 (F) |
| 10.33 | -F | 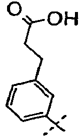 | 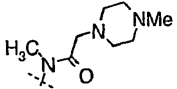 | 3.48 | C₃₂H₃₄FN₅O₄ | 572 [M-H]⁻ | 170-175 | 0.40 (C) |
| 10.34 | -F | 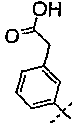 | -N(SO₂Me)-(CH₂)₂-NMe₂ | 3.26 | C₂₈H₂₉FN₄O₅S | 553 [M+H]⁺ | 180 | 0.60 (C) |
| 10.35 | -F | 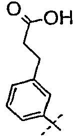 | -N(SO₂Me)-(CH₂)₂-NMe₂ | 3.49 | C₂₉H₃₁FN₄O₅S | 567 [M+H]⁺ | 196-199 | 0.30 (C) |
| 10.36 | -F | 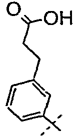 | -N(Me)-(CO)-CH₂-NMe₂ | 3.50 | C₂₉H₂₉FN₄O₄ | 517 [M+H]⁺ | 150 | 0.20 (C) |
| 10.37 | -F | 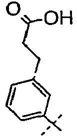 | -N(COMe)-(CH₂)₃-NMe₂ | 3.51 | C₃₁H₃₃FN₄O₄ | 545 [M+H]⁺ | 206-210 | 0.30 (A) |
| 10.38 | -F | 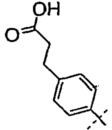 | -N(Me)-(CO)-CH₂-NMe₂ | 3.59 | C₂₉H₂₉FN₄O₄ | 517 [M+H]⁺ | 231-236 | 0.60 (A) |
| 10.39 | -F | 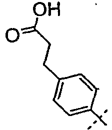 | -(CH₂)₂-NMe₂ | 3.57 | C₂₈H₂₈FN₃O₃ | 474 [M+H]⁺ | 218-222 | 0.50 (A) |
| 10.40 | -F | 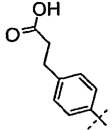 | -N(Me)-(CO)-(CH₂)₂-NMe₂ | 3.58 | C₃₀H₃₁FN₄O₄ | 531 [M+H]⁺ | 215-218 | 0.50 (A) |
| 10.41 | -F | 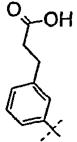 | -(CH₂)₂-NMe₂ | 3.60 | C₂₈H₂₈FN₃O₃ | 474 [M+H]⁺ | 172-177 | 0.15 (G) |
| 10.42 | -F | 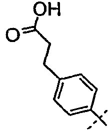 | -N(COMe)-(CH₂)₂-NMe₂ | 3.61 | C₃₀H₃₁FN₄O₄ | 531 [M+H]⁺ | 230-234 | 0.50 (A) |
| 10.43 | -F | 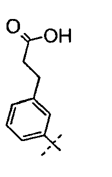 | -N(Me)-(CO)-(CH₂)₃-NMe₂ | 3.62 | C₃₁H₃₃FN₄O₄ | 545 [M+H]⁺ | 170-175 | 0.30 (E) |
| 10.44 | -F | 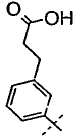 | -N(Me)-(CO)-(CH₂)₄-NMe₂ | 3.63 | C₃₂H₃₅FN₄O₄ | 559 [M+H]⁺ | 142-146 | 0.10 (G) |
| 10.45 | -F | 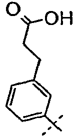 | 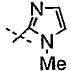 | 3.64 | C₂₈H₂₃FN₄O₃ | 483 [M+H]⁺ | 262-269 | 0.20 (E) |
| 10.46 | -F | 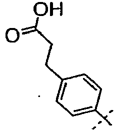 | -N(Me)-(CO)-(CH₂)₄-NMe₂ | 3.65 | C₃₂H₃₅FN₄O₄ | 559 [M+H]⁺ | 234-236 | 0.30 (A) |
| 10.47 | -F | 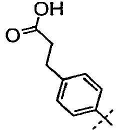 | -H | 3.66 | C₂₄H₁₉FN₂O₃ | 403 [M+H]⁺ | 231-233 | 0.20 (A) |
| 10.48 | -F | 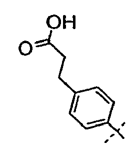 | 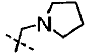 | 3.67 | C₂₉H₂₈FN₃O₃ | 486 [M+H]⁺ | 205-210 | 0.10 (E) |
| 10.49 | -F | 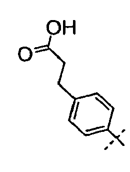 | -CH₂-NEt₂ | 3.68 | C₂₉H₃₀FN₃O₃ | 488 [M+H]⁺ | 145-150 | 0.15 (E) |
| 10.50 | -F | 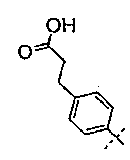 | -CH₂-NH₂ | 9.7 | C₂₅H₂₂FN₃O₃ | 430 [M-H]⁻ | 280-285 | 0.05 (H) |
| 10.51 | -F | 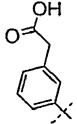 | -(CH₂)₂-NMe₂ | 3.70 | C₂₇H₂₆FN₃O₃ | 460 [M+H]⁺ | 273-276 | 0.15 (E) |
| 10.52 | -F | 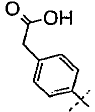 | -(CH₂)₂-NMe₂ | 3.71 | C₂₇H₂₆FN₃O₃ | 460 [M+H]⁺ | 230-235 | 0.05 (E) |
| 10.53 | -Cl | 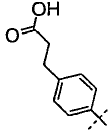 | -(CH₂)₂-NMe₂ | 3.73 | C₂₈H₂₈ClN₃O₃ | 490/492 [M+H]⁺ | 255-258 | 0.50 (A) |
| 10.54 | -Cl | 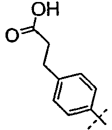 | 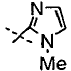 | 3.74 | C₂₈H₂₃ClN₄O₃ | 499/501 [M+H]⁺ | 296-300 | 0.50 (A) |
| 10.55 | -Cl | 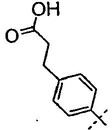 | -CH₂-NMe₂ | 3.75 | C₂₇H₂₆ClN₃O₃ | 476/478 [M+H]⁺ | 228-230 | 0.50 (A) |
| 10.56 | -F | 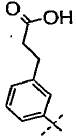 | 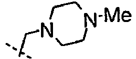 | 3.77 | C₃₀H₃₁FN₄O₃ | 515 [M+H]⁺ | 210-215 | 0.40 (A) |
| 10.57 | -F | 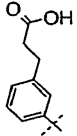 | 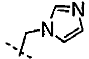 | 3.78 | C₂₈H₂₃FN₄O₃ | 483 [M+H]⁺ | 240-245 | 0.50 (A) |
| 10.58 | -F | 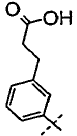 | -CH₂-NMe-(CH₂)₂-NMe₂ | 3.82 | C₃₀H₃₃FN₄O₃ | 517 [M+H]⁺ | n.b. | 0.30 (I) |
| 10.59 | -F | 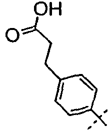 | 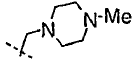 | 3.79 | C₃₀H₃₁FN₄O₃ C₃₀H₃₁FN₄O₃ | 515 [M+H]⁺ | 275 | 0.35 (A) |
| 10.60 | -F | 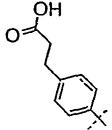 | 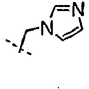 | 3.80 | C₂₈H₂₃FN₄O₃ | 483 [M+H]⁺ | 280 | 0.55 (A) |
| 10.61 | -Cl | 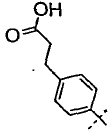 | 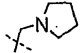 | 3.83 | C₂₉H₂₈ClN₃O₃ | 502/504 [M+H]⁺ | 260-266 | 0.50 (A) |
| 10.62 | -F | 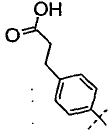 | -CH₂-NMe-(CH₂)₂-NMe₂ | 3.81 | C₃₀H₃₃FN₄O₃ | 517 [M+H]⁺ | n.b. | 0.05 (E) |
| 10.63 | -F | 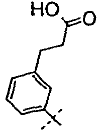 | -H | 3.84 | C₂₄H₁₉FN₂O₃ | 403 [M+H]⁺ | 110-112 | 0.60 (K) |
| 10.64 | -F | 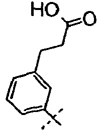 | -CH₂-NH₂ | 9.8 | C₂₅H₂₂FN₃O₃ | 432 [M+H]⁺ | 260-263 | 0.60 (A) |
| 10.65 | -F | 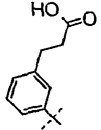 | -CH₂-NHMe | 9.9 | C₂₆H₂₄FN₃O₃ | 446 [M+H]⁺ | 265-270 | 0.60 (A) |
| 10.66 | -F | 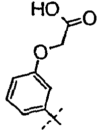 | -CH₂-NMe₂ | 3.87 | C₂₆H₂₄FN₃O₄ | 462 [M+H]⁺ | 250 | 0.10 (M) |
| 10.67 | -F | 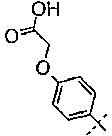 | -CH₂-NMe₂ | 3.88 | C₂₆H₂₄FN₃O₄ | 462 [M+H]⁺ | 247 | 0.15 (M) |
| 10.68 | -Br | 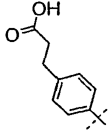 | 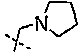 | 3.90 | C₂₉H₂₈BrN₃O₃ | 546/548 [M+H]⁺ | 290-293 | 0.30 (E) |
| 10.69 | -Br | 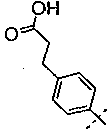 | -CH₂-NMe₂ | 3.91 | C₂₇H₂₆BrN₃O₃ | 520/522 [M+H]⁺ | 243-246 | 0.25 (E) |
| 10.70 | -Br | 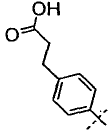 | -CH₂-NEt₂ | 3.92 | C₂₉H₃₀BrN₃O₃ | 548/550 [M+H]⁺ | 252-255 | 0.35 (E) |

| | | | | | | | | |
|---|---|---|---|---|---|---|---|---|
| *Fließmittelgemische: (A): Reversed Phase RP8, Methanol/Kochsalzlösung(5%) = 4:1 (B): Kieselgel, Methylenchlorid/Methanol = 8:2 (C): Kieselgel, Methylenchlorid/Methanol = 5:1 (D): Reversed Phase RP8, Methanol/Kochsalzlösung(5%) = 3:2 (E): Kieselgel, Methylenchlorid/Methanol = 9:1 (F): Reversed Phase RP8, Methanol/Kochsalzlösung(5%) = 7:3 (G): Kieselgel, Methylenchlorid/Methanol/Ammoniak = 9:1:0,1 (H): Aluminiumoxid, Methylenchlorid/Methanol = 19:1 (I): Reversed Phase RP8, Methanol/Kochsalzlösung(5%) = 4:2 (K): Kieselgel, Petrolether/Essigester = 1:1 (M): Kieselgel, Methylenchlorid/Methanol = 4:1 | | | | | | | | |

Analog Beispiel 10.0 werden folgende Verbindungen der allgemeinen Formel I-10b hergestellt:

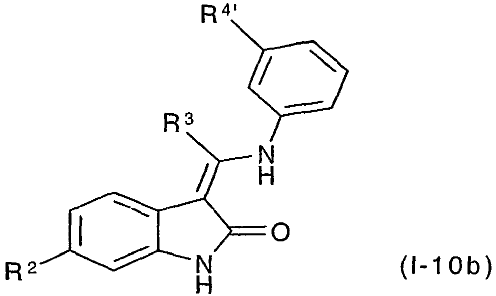

| Beispiel | R² | R³ | R⁴ | Edukt | Summenformel | Massenspektrum | Fp. [°C] | R_{f}-Wert* |
|---|---|---|---|---|---|---|---|---|
| 10.71 | -F | 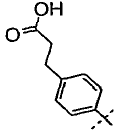 | -CH₂-NMe₂ | 3.93 | C₂₇H₂₆FN₃O₃ | 460 [M+H]⁺ | 150 | 0.20 (A) |
| 10.72 | -F | 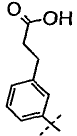 | -CH₂-NMe₂ | 3.94 | C₂₇H₂₆FN₃O₃ | 460 [M+H]⁺ | 105-109 | 0.30 (B) |
| 10.73 | -Cl | 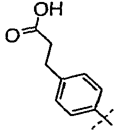 | -CH₂-NMe₂ | 3.95 | C₂₇H₂₆ClN₃O₃ | 476/478 [M+H]⁺ | 230-235 | 0.50 (C) |

| | | | | | | | | |
|---|---|---|---|---|---|---|---|---|
| *Fließmittelgemische: (A): Kieselgel, Methylenchlorid/Methanol = 5:1 (B): Kieselgel, Methylenchlorid/Methanol = 9:1 (C): Reversed Phase RP8, Methanol/Kochsalzlösung(5%) = 4:1 | | | | | | | | |

### Beispiel 11.0

### 3-Z-[1-(4-Dimethylaminomethyl-anilino)-1-(3-(2-carbarnoyl-ethyl)-phenyl)-methylen]-6-chlor-2-indolinon

480 mg 3-Z-[1-(4-Dimethylaminomethyl-anilino)-1-(3-(2-carboxyethyl)-phenyl)-methylen]-6-chlor-2-indolinon (Edukt 10.0), 350 mg TBTU, 150 mg HOBt und 420 ml Triethylamin werden in 10 ml Dimethylformamid gelöst und 620 mg N-Hydroxysuccinimid-Ammoniumsalz zugegeben. Der Ansatz wird für 20 Stunden bei Raumtemperatur gerührt. Nach dem Abziehen des Lösungsmittels wird der Rückstand in wenig Essigester und Wasser suspendiert, abfiltriert und mit Wasser nachgewaschen. Der Rückstand wird über eine Aluminiumoxidsäule (Aktivität 2-3) mit Methylenchlorid/Ethanol 20:1 als Laufmittel aufgereinigt. Das Produkt wird aus Diethylether umkristallisiert und im Vakuum bei 100°C getrocknet.
Ausbeute: 370 mg (78% der Theorie),
R_{f}-Wert: 0.40 (Aluminiumoxid, Methylenchlorid/Ethanol = 20:1)
Fp. 222-225 °C
C₂₇H₂₇ClN₄O₂
Massenspektrum: m/z = 475/477 [M+H]⁺

Analog Beispiel 11.0 werden folgende Verbindungen der allgemeinen Formel I-11 hergestellt:

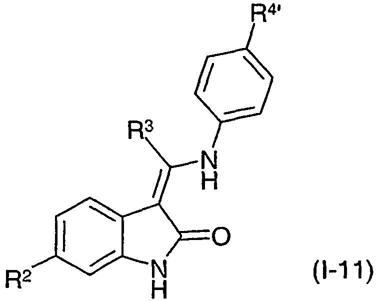

| Beispiel | R² | R³ | R⁴' | Edukt | Summenformel | Massenspektrum | Fp. [°C] | R_{f}-Wert* |
|---|---|---|---|---|---|---|---|---|
| 11.1 | -Cl | 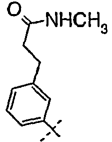 | -CH₂-NMe₂ | 10.0 ** | C₂₈H₂₉ClN₄O₂ | 489/491 [M+H)⁺ | 223-225 | 0.50 (A) |
| 11.2 | -F | 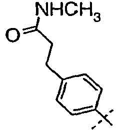 | -CH₂-NMe₂ | 10.1 ** | C₂₈H₂₉FN₄O₂ | 473 [M+H]⁺ | 148-150 | 0.40 (B) |
| 11.3 | -F | 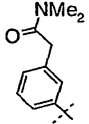 | -CH₂-NMe₂ | 10.2 *** | C₂₈H₂₉FN₄O₂ | 473 [M+H]⁺ | 98-103 | 0.30 (C) |
| 11.4 | -F | 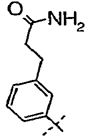 | -CH₂-NMe₂ | 10.3 | C₂₇H₂₇FN₄O₂ | 459 [M+H]⁺ | 223-225 | 0.50 (A) |
| 11.5 | -F | 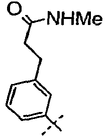 | -CH₂-NMe₂ | 10.3 ** | C₂₈H₂₉FN₄O₂ | 473 [M+H]⁺ | 210-213 | 0.70 (A) |
| 11.6 | -F | 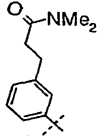 | -CH₂-NMe₂ | 10.3 *** | C₂₉H₃₁FN₄O₂ | 487 [M+H]⁺ | 213-215 | 0.80 (A) |
| 11.7 | -F | 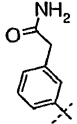 | -CH₂-NMe₂ | 10.2 | C₂₆H₂₅FN₄O₂ | 443 [M-H]⁻ | 115-120 | 0.25 (C) |
| 11.8 | -F | 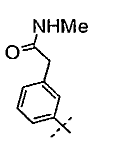 | -CH₂-NMe₂ | 10.2 ** | C₂₇H₂₇FN₄O₂ | 457 [M-H]⁻ | 222-225 | 0.25 (C) |
| 11.9 | -F | 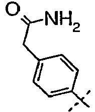 | -CH₂-NMe₂ | 10.4 | C₂₆H₂₅FN₄O₂ | 443 [M-H]⁻ | 143-146 | 0.40 (D) |
| 11.10 | -F | 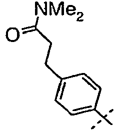 | -CH₂-NMe₂ | 10.1 *** | C₂₉H₃₁FN₄O₂ | 487 [M+H]⁺ | 198-200 | 0.60 (B) |
| 11.11 | -F | 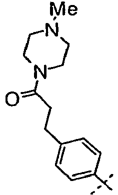 | -CH₂-NMe₂ | 10.1 **** | C₃₂H₃₆FN₅O₂ | 542 [M+H]⁺ | 175 | 0.60 (B) |
| 11.12 | -F | 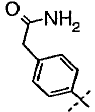 | 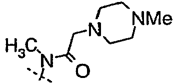 | 10.5 | C₃₁H₃₃FN₆O₃ | 557 [M+H]⁺ | 150-156 | 0.40 (E) |
| 11.13 | -F | 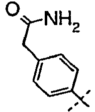 | -N(SO₂Me)-(CH₂)₂-NMe₂ | 10.6 | C₂₈H₃₀FN₅O₄S | 552 [M+H]⁺ | 197-199 | 0.50 (D) |
| 11.14 | -F | 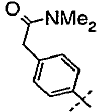 | -CH₂-NMe₂ | 10.4 *** | C₂₈H₂₉FN₄O₂ | 473 [M+H]⁺ | 147-152 | 0.35 (D) |
| 11.15 | -F | 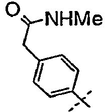 | -CH₂-NMe₂ | 10.4 ** | C₂₇H₂₇FN₄O₂ | 459 [M+H]⁺ | 208-214 | 0.35 (D) |
| 11.16 | -F | 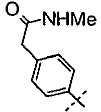 | -N(SO₂Me)-(CH₂)₂-NMe₂ | 10.6 ** | C₂₉H₃₂FN₅O₄S | 566 [M+H]⁺ | 218-222 | 0.70 (F) |
| 11.17 | -F | 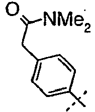 | -N(SO₂Me)-(CH₂)₂-NMe₂ | 10.6 *** | C₃₀H₃₄FN₅O₄S | 580 [M+H]⁺ | 199-205 | 0.40 (C) |
| 11.18 | -F | 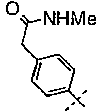 | 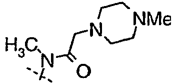 | 10.5 ** | C₃₂H₃₅FN₆O₃ | 571 [M+H]⁺ | 155-160 | 0.20 (C) |
| 11.19 | -F | 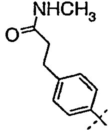 | -N(Me)-(CO)-CH₃ | 10.7 ** | C₂₈H₂₇FN₄O₃ | 487 [M+H]⁺ | 137-145 | 0.50 (C) |
| 11.20 | -F | 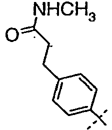 | 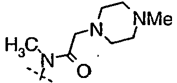 | 10.8 ** | C₃₃H₃₇FN₆O₃ | 585 [M+H]⁺ | 211-219 | 0.40 (C) |
| 11.21 | -F | 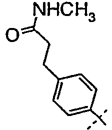 | -N(SO₂Me)-(CH₂)₂-NMe₂ | 10.9 ** | C₃₀H₃₄FN₅O₄S | 578 [M-H]⁻ | 192-200 | 0.50 (C) |
| 11.22 | -F | 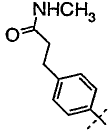 | 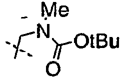 | 10.11 ** | C₃₂H₃₅FN₄O₄ | 559 [M+H]⁺ | 180-187 | 0.50 (C) |
| 11.23 | -F | 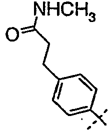 | 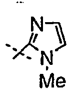 | 10.13 ** | C₂₉H₂₆FN₅O₂ | 496 [M+H]⁺ | 262-266 | 0.40 (C) |
| 11.24 | -F | 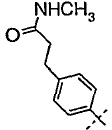 | -SO₂Me | 10.14 ** | C₂₆H₂₄FN₃O₄S | 494 [M+H]⁺ | 180-188 | 0.60 (C) |
| 11.25 | -F | 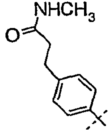 | 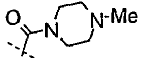 | 10.12 ** | C₃₁H₃₂FN₅O₃ | 542 [M+H]⁺ | 226-230 | 0.50 (C) |
| 11.26 | -F | 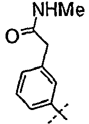 | 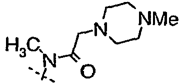 | 10.16 ** | C₃₂H₃₅FN₆O₃ | 571 [M+H]⁺ | 213 | 0.10 (G) |
| 11.27 | -F | 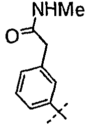 | 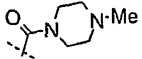 | 10.15 ** | C₃₀H₃₀FN₅O₃ | 528 [M+H]⁺ | 245 | 0.40 (G) |

| | | | | | | | | |
|---|---|---|---|---|---|---|---|---|
| *Fließmittelgemische: (A): Kieselgel, Methylenchlorid/Methanol/Ammoniak = 5:1:0,01 (B): Aluminiumoxid, Methylenchlorid/Ethanol = 20:1 (C): Kieselgel, Methylenchlorid/Methanol/Ammoniak = 9:1:0,1 (D): Kieselgel, Methylenchlorid/Methanol/Ammoniak = 6:1:0,1 (E): Kieselgel, Methylenchlorid/Methanol/Ammoniak = 5:1:0,1 (F): Kieselgel, Methylenchlorid/Methanol/Ammoniak = 7:1:0,1 (G): Kieselgel, Methylenchlorid/Methanol = 9:1 ** mit Methylammoniumchlorid als Basenäquivalent *** mit Dimethylammoniumchlorid als Basenäquivalent **** mit Piperidin-Hydrochlorid als Basenäquivalent | | | | | | | | |

### Beispiel 12.0

### 3-Z-[1-4-Dimethylaminomethyl-anilino)-1-(4-acetylaminomethyl-phenyl)-methylen]-6-chlor-2-indolinon

100 mg 3-Z-[1-(4-Dimethylaminomethyl-anilino)-1-(4-aminomethyl-phenyl)-methylen]-6-chlor-2-indolinon (Edukt 7.0) werden in 5 ml Methylenchlorid und 5 ml Pyridin gelöst und bei 0°C 20 *µ*l Acetylchlorid zugegeben. Der Ansatz wird für 10 Minuten bei 0°C und für weitere 4 Stunden bei Raumtemperatur gerührt. Danach werden weitere 20 *µ*l Acetylchlorid zugegeben und der Ansatz für 12 Stunden bei Raumtemperatur gerührt. Nach dieser Zeit wird das Lösungsmittel abgezogen, der Rückstand in Methylenchlorid aufgenommen mit Wasser gewaschen. Die wäßrige Phase wir zweimal mit Methylenchlorid extrahiert und die vereinigten organischen Phasen über Natriumsulfat getrocknet. Das Lösungsmittel wird einrotiert und der Rückstand mit Ether gewaschen.
Ausbeute: 51 mg (47% der Theorie),
R_{f}-Wert: 0.30 (Kieselgel, Kieselgel, Methylenchlorid/Methanol/Ammoniak = 9:1:0,01)
Fp. 219-220 °C
C₂₇H₂₇ClN₄O₂
Massenspektrum: m/z = 473/475 [M-H]⁻

Analog Beispiel 12.0 werden folgende Verbindungen der allgemeinen Formel I-12 hergestellt:

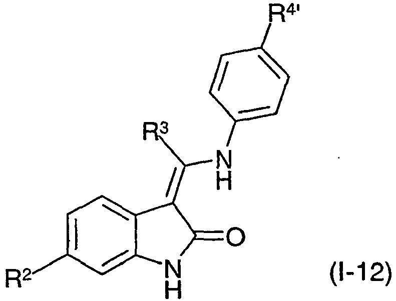

| Beispiel | R² | R³ | R⁴' | Edukt | Summenformel | Massenspektrum | Fp. [°C] | R_{f}-Wert* |
|---|---|---|---|---|---|---|---|---|
| 12.1 | -Cl | 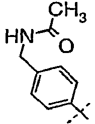 | 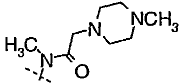 | 8.0 | C₃₂H₃₅ClN₆O₃ | 585/587 [M-H]⁻ | 252-255 | 0.25 (B) |
| 12.2 | -Cl | 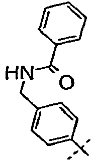 | -CH₂-NMe₂ | 7.0 | C₃₂H₂₉ClN₄O₂ | 535/537 [M-H]⁻ | 238 (Zer. ) | 0.45 (B) |
| 12.3 | -Cl | 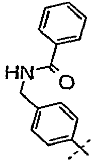 | 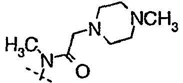 | 8.0 | C₃₇H₃₇ClN₆O₃ | 647/649 [M-H]⁻ | 282-284 | 0.40 (B) |
| 12.4 | -F | 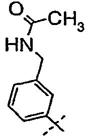 | -CH₂-NMe₂ | 9.0 | C₂₇H₂₇FN₄O₂ | 457 [M-H]⁻ | 245-250 | 0.40 (C) |
| 12.5 | -F | 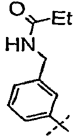 | -CH₂-NMe₂ | 9.0 | C₂₈H₂₉FN₄O₂ | 471 [M-H]⁻ | 212-214 | 0.35 (D) |
| 12.6 | -F | 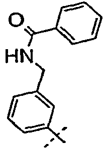 | -CH₂-NMe₂ | 9.0 | C₃₂H₂₉FN₄O₂ | 519 [M-H]⁻ | 237-240 | 0.40 (D) |
| 12.7 | -F | 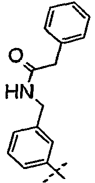 | -CH₂-NMe₂ | 9.0 | C₃₃H₃₁FN₄O₂ | 533 [M-H]⁻ | 187-190 | 0.30 (D) |
| 12.8 | -F | 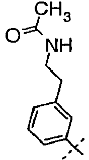 | -CH₂-NMe₂ | 9.1 | C₂₈H₂₉FN₄O₂ | 471 [M-H]⁻ | 234-237 | 0.30 (D) |
| 12.9 | -F | 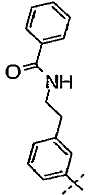 | -CH₂-NMe₂ | 9.1 | C₃₃H₃₁FN₄O₂ | 533 [M-H]⁻ | 144-150 | 0.45 (C) |
| 12.10 | -F | 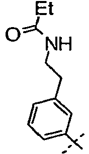 | -CH₂-NMe₂ | 9.1 | C₂₉H₃₁FN₄O₂ | 485 [M-H]⁻ | 235-237 | 0.25 (D) |
| 12.11 | -F | 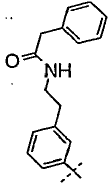 | -CH₂-NMe₂ | 9.1 | C₃₄H₃₃FN₄O₂ | 547 [M-H]⁻ | 217-220 | 0.30 (D) |
| 12.12 | -F | 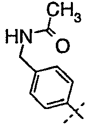 | -CH₂-NMe₂ | 9.2 | C₂₇H₂₇FN₄O₂ | 457 [M-H]⁻ | 112-120 | 0.25 (D) |
| 12.13 | -F | 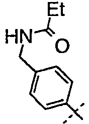 | -CH₂-NMe₂ | 9.2 | C₂₈H₂₉FN₄O₂ | 586 [M+H]⁺ | 176-180 | 0.30 (D) |
| 12.14 | -F | 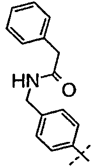 | -CH₂-NMe₂ | 9.2 | C₃₃H₃₁FN₄O₂ | 535 [M+H]⁺ | 80-85 | 0.35 (D) |
| 12.15 | -F | 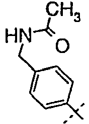 | 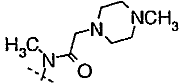 | 9.3 | C₃₂H₃₅FN₆O₃ | 569 [M-H]⁻ | 230-235 | 0.35 (D) |
| 12.16 | -F | 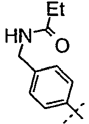 | 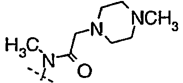 | 9.3 | C₃₃H₃₇FN₆O₃ | 583 [M-H]⁻ | 205-210 | 0.30 (D) |
| 12.17 | -F | 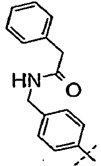 | 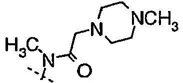 | 9.3 | C₃₈H₃₉FN₆O₃ | 645 [M-H]⁻ | 217-220 | 0.35 (D) |
| 12.18 | -F | 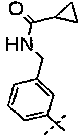 | 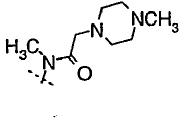 | 9.6 | C₃₄H₃₇FN₆O₃ | 597 [M+H]⁺ | 209-212 | 0.30 (D) |
| 12.19 | -F | 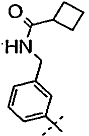 | 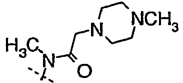 | 9.6 | C₃₅H₃₉FN₆O₃ | 611 [M+H]⁺ | 190-193 | 0.30 (D) |
| 12.20 | -F | 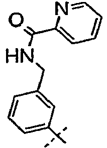 | 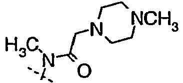 | 9.6 | C₃₆H₃₆FN₇O₃ | 634 [M+H]⁺ | 160-163 | 0.30 (D) |
| 12.21 | -F | 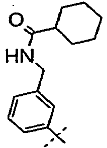 | 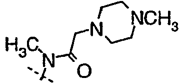 | 9.6 | C₃₇H₄₃FN₆O₃ | 639 [M+H]⁺ | 223-227 | 0.30 (D) |
| 12.22 | -F | 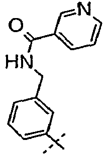 | 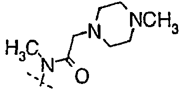 | 9.6 | C₃₆H₃₆FN₇O₃ | 634 [M+H]⁺ | 170-175 | 0.25 (D) |
| 12.23 | -F | 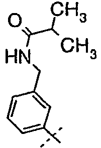 | 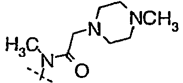 | 9.6 | C₃₄H₃₉FN₆O₃ | 599 [M+H]⁺ | 194-196 | 0.20 (D) |
| 12.24 | -F | 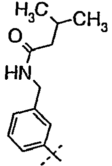 | 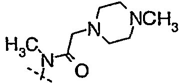 | 9.6 | C₃₅H₄₁FN₆O₃ | 613 [M+H]⁺ | 197-200 | 0.70 (E) |
| 12.25 | -F | 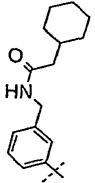 | 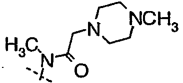 | 9.6 | C₃₈H₄₅FN₆O₃ | 653 [M+H]⁺ | 130-135 | 0.75 (E) |
| 12.26 | -F | 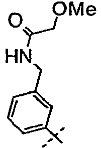 | 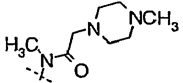 | 9.6 | C₃₃H₃₇FN₆O₄ | 601 [M+H]⁺ | 155-159 | 0.60 (E) |
| 12.27 | -F | 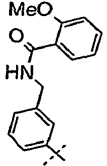 | 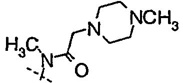 | 9.6 | C₃₈H₃₉FN₆O₄ | 663 [M+H]⁺ | 168-172 | 0.35 (C) |
| 12.28 | -F | 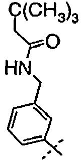 | 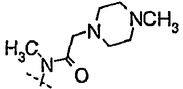 | 9.6 | C₃₆H₄₃FN₆O₃ | 627 [M+H]⁺ | 85-90 | 0.35 (C) |
| 12.29 | -F | 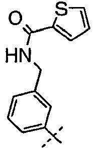 | 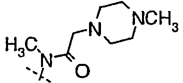 | 9.6 | C₃₅H₃₅FN₆O₃S | 639 [M+H]⁺ | 170-175 | 0.25 (C) |
| 12.30 | -F | 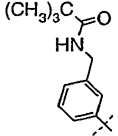 | 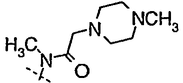 | 9.6 | C₃₅H₄₁FN₆O₃ | 613 [M+H]⁺ | 242-245 | 0.30 (C) |
| 12.31 | -F | 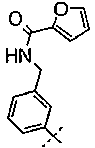 | 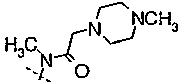 | 9.6 | C₃₅H₃₅FN₆O₄ | 623 [M+H]⁺ | 155-160 | 0.65 (F) |
| 12.32 | -F | 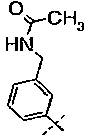 | 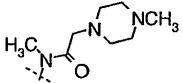 | 9.6 | C₃₂H₃₅FN₆O₃ | 571 [M+H]⁺ | 190-195 | 0.60 (F) |
| 12.33 | -F | 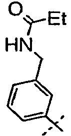 | 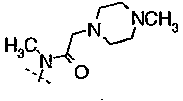 | 9.6 | C₃₃H₃₇FN₆O₃ | 585 [M+H]⁺ | 203-209 | 0.65 (E) |
| 12.34 | -F | 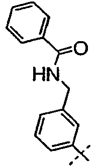 | 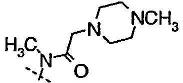 | 9.6 | C₃₇H₃₇FN₆O₃ | 633 [M+H]⁺ | 145-150 | 0.60 (F) |
| 12.35 | -F | 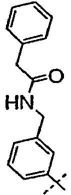 | 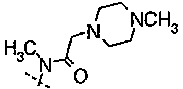 | 9.6 | C₃₈H₃₉FN₆O₃ | 647 [M+H]⁺ | 148-151 | 0.65 (F) |
| 12.36 | -F | 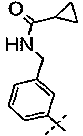 | -CH₂-NMe₂ | 9.0 | C₂₉H₂₉FN₄O₂ | 485 [M+H]⁺ | 216-220 | 0.35 (D) |
| 12.37 | -F | 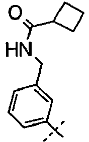 | -CH₂-NMe₂ | 9.0 | C₃₀H₃₁FN₄O₂ | 499 [M+H]⁺ | 214-217 | 0.35 (D) |
| 12.38 | -F | 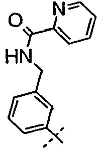 | -CH₂-NMe₂ | 9.0 | C₃₁H₂₈FN₅O₂ | 522 [M+H]⁺ | 205-210 | 0.35 (D) |
| 12.39 | -F | 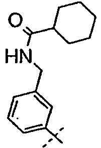 | -CH₂-NMe₂ | 9.0 | C₃₂H₃₅FN₄O₂ | 527 [M+H]⁺ | 235-237 | 0.35 (D) |
| 12.40 | -F | 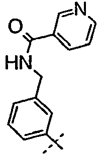 | -CH₂-NMe₂ | 9.0 | C₃₁H₂₈FN₅O₂ | 520 [M-H]⁻ | 135-140 | 0.20 (D) |
| 12.41 | -F | 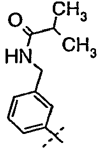 | -CH₂-NMe₂ | 9.0 | C₂₉H₃₁FN₄O₂ | 487 [M+H]⁺ | 210-215 | 0.20 (D) |
| 12.42 | -F | 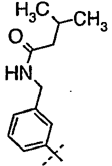 | -CH₂-NMe₂ | 9.0 | C₃₀H₃₃FN₄O₂ | 501 [M+H]⁺ | 202-206 | 0.25 (D) |
| 12.43 | -F | 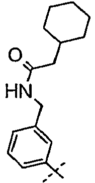 | -CH₂-NMe₂ | 9.0 | C₃₃H₃₇FN₄O₂ | 541 [M+H]⁺ | 198-203 | 0.35 (D) |
| 12.44 | -F | 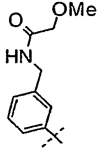 | -CH₂-NMe₂ | 9.0 | C₂₈H₂₉FN₄O₃ | 489 [M+H]⁺ | 173-177 | 0.35 (D) |
| 12.45 | -F | 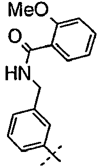 | -CH₂-NMe₂ | 9.0 | C₃₃H₃₁FN₄O₃ | 549 [M-H]⁻ | 202-207 | 0.50 (C) |
| 12.46 | -F | 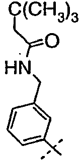 | -CH₂-NMe₂ | 9.0 | C₃₁H₃₅FN₄O₂ | 513 [M-H]⁻ | 203-209 | 0.45 (C) |
| 12.47 | -F | 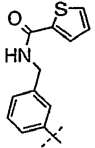 | -CH₂-NMe₂ | 9.0 | C₃₀H₂₇FN₄O₂S | 527 [M+H]⁺ | 245-250 | 0.35 (C) |
| 12.48 | -F | 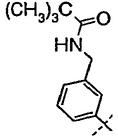 | -CH₂-NMe₂ | 9.0 | C₃₀H₃₃FN₄O₂ | 501 [M+H]⁺ | 248-252 | 0.45 (C) |
| 12.49 | -F | 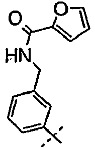 | -CH₂-NMe₂ | 9.0 | C₃₀H₂₇FN₄O₃ | 511 [M+H]⁺ | 216-219 | 0.30 (C) |
| 12.50 | -F | 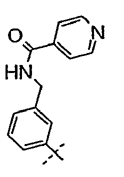 | -CH₂-NMe₂ | 9.0 | C₃₁H₂₈FN₅O₂ | 522 [M+H]⁺ | 167-170 | 0.20 (D) |

| | | | | | | | | |
|---|---|---|---|---|---|---|---|---|
| *Fließmittelgemische: (A): Kieselgel, Methylenchlorid/Ethanol/Ammoniak = 20:1:0,01 (B): Kieselgel, Methylenchlorid/Methanol/Ammoniak = 9:1:0,01 (C): Aluminiumoxid, Methylenchlorid/Methanol = 19:1 (D): Kieselgel, Methylenchlorid/Methanol/Ammoniak = 9:1:0,1 (E): Kieselgel, Methylenchlorid/Methanol/Ammoniak = 8:2:0,2 (F): Aluminiumoxid, Methylenchlorid/Methanol = 9:1 | | | | | | | | |

alternativ wurden als Acylierungsmittel verwendet:
Benzoylchlorid, Propionylchlorid, Phenylacetylchlorid, Cyclopropancarbonylchlorid, Cyclobutancarbonylchlorid, Pyridin-2-yl-carbonylchlorid, Pyridin-3-yl-carbonylchlorid, Pyridin-4-yl-carbonylchlorid, Cyclohexylcarbonylchlorid, Isobutyrylchlorid, 3-Methylbutyrylchlorid, Cyclohexylmethylcarbonylchlorid, Methoxyacetylchlorid, 2-Methoxybenzoylchlorid, tert.-Butylacetylchlorid, Thiophen-2-carbonylchlorid, Pivaloylchlorid, 2-Furoyl-chlorid

### Beispiel 13.0

### 3-Z-[1-(4-Trimethylammoniummethyl-anilino)-1-(4-(2-carboxy-ethyl)-phenyl)-methylen]-6-fluor-2-indolinon-iodid

200 mg 3-Z-[1-(4-Dimethylaminomethyl-anilino)-1-(4-(2-carboxy-ethyl)-phenyl)-methylen]-6-fluor-2-indolinon (Edukt 10.1) werden in 40 ml Aceton gelöst und 250 ml Methyliodid zugegeben. Der Ansatz wird für 20 Stunden bei Raumtemperatur gerührt. Nach dieser Zeit wird der ausgefallene Rückstand abgesaugt. Das Produkt wird bei 80°C im Vakuum getrocknet.
Ausbeute: 200 mg (83% der Theorie),
R_{f}-Wert: 0.50 (Reversed Phase RP8, Methanol/Kochsalzlösung(5%) = 4:1)
Fp. 210 °C
C₂₈H₂₉FN₃O₃I
Massenspektrum: m/z = 474 [M+H]⁺

Analog Beispiel 13.0 wird folgende Verbindung der allgemeinen Formel I-13 hergestellt:

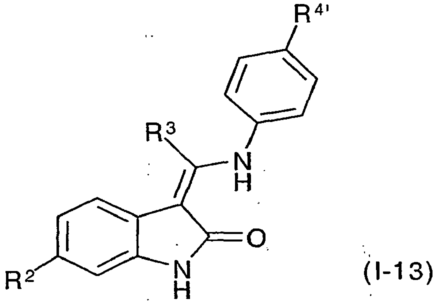

| Beispiel | R² | R³ | R^{4'} | Edukt | Summenformel | Massenspektrum | Fp. [°C] | R_{f}-Wert* |
|---|---|---|---|---|---|---|---|---|
| 13.1 | -F | 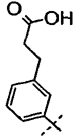 | 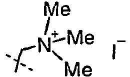 | 10.3 | C₂₈H₂₉FN₃O₃I | 474 [M+H]⁺ | 150 | 0.50 (A) |

| | | | | | | | | |
|---|---|---|---|---|---|---|---|---|
| *Fließmittelgemische: (A): Reversed Phase RP8, Methanol/Kochsalzlösung(5%) = 4:1 | | | | | | | | |

### Beispiel 14.0

### 3-Z-[1-(4-Guanidinomethyl-anilino)-1-(4-(2-carboxy-ethyl)-phenyl)-methylen]-6-fluor-2-indolinon-iodid

170 mg 3-Z-[1-(4-Aminomethyl-anilino)-1-(4-(2-carboxy-ethyl)-phenyl)-methylen]-6-fluor-2-indolinon (Edukt 10.50) werden in 20 ml Tetrahydrofuran gelöst und 390 mg 3,5-Dimethylpyrazol-1-carbonsäureamidin-nitrat und 330 ml Diethylisopropylamin zugegeben. Der Ansatz wird für 10 Stunden unter Rückfluß gerührt. Nach dieser Zeit wird das Lösungsmittel eingeengt, Wasser zugegeben und der ausgefallene Rückstand abgesaugt. Das Produkt wird bei 80°C getrocknet.
Ausbeute: 150 mg (81 % der Theorie),
R_{f}-Wert: 0.40 (Kieselgel, Methylenchlorid/Methanol/Essigsäure = 5:1:0,1)
Fp. 290 °C
C₂₆H₂₄FN₅O₃
Massenspektrum: m/z = 474 [M+H]⁺

Analog Beispiel 14.0 wird folgende Verbindung der allgemeinen Formel I-14 hergestellt:

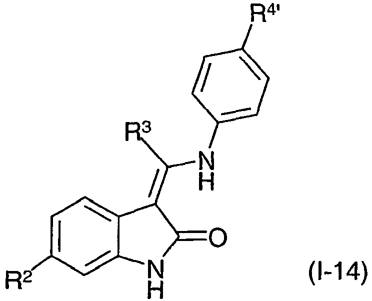

| Beispiel | R² | R³ | R⁴' | Edukt | Summenformel | Massenspektrum | Fp. [°C] | R_{f}-Wert* |
|---|---|---|---|---|---|---|---|---|
| 14.1 | -F | 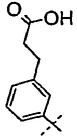 | 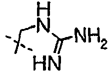 | 10.64 | C₂₆H₂₄FN₅O₃ | 474 [M+H]⁺ | 305 | 0.70 (A) |

| | | | | | | | | |
|---|---|---|---|---|---|---|---|---|
| *Fließmittelgemische: (A): Reversed Phase RP8, Methanoi/Kochsalzlösung(5%) = 4:1 | | | | | | | | |

### Beispiel 15

Trockenampulle mit 75 mg Wirkstoff pro 10 ml

### Zusammensetzung:

| | |
|---|---|
| Wirkstoff | 75,0 mg |
| Mannitol | 50,0 mg |
| Wasser für Injektionszwecke | ad 10,0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel 16

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

### Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

### Beispiel 17

Tablette mit 50 mg Wirkstoff

### Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2.0 mg |
| | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreBt, biplan mit beidseitiger Facette und einseitiger Teilkerbe. Durchmesser der Tabletten: 9 mm.

### Beispiel 18

Tablette mit 350 mg Wirkstoff

### Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4.0 mg |
| | 600,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

### Beispiel 19

Kapseln mit 50 mg Wirkstoff

### Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2.0 mg |
| | 160,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 3 abgefüllt.

### Beispiel 20

Kapseln mit 350 mg Wirkstoff

### Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4.0 mg |
| | 430,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 0 abgefüllt.

### Beispiel 21

Suppositorien mit 100 mg Wirkstoff

### 1 Zäpfchen enthält:

| | |
|---|---|
| Wirkstoff | 100,0 mg |
| Polyethylenglykol (M.G. 1500) | 600,0 mg |
| Polyethylenglykol (M.G. 6000) | 460,0 mg |
| Polyethylensorbitanmonostearat | 840,0 mg |
| | 2 000,0 mg |

### Herstellung::

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

Analog den vorstehenden Beispielen können folgende Verbindungen hergestellt werden:
(1) 3-Z-[1-(4-(N-(2-Dimethylamino-ethyl)-N-methylsulfonyl-amino)-anilino)-1-(4-(2-carboxy-ethyl)-phenyl)-methylen]-6-chlor-2-indolinon
(2) 3-Z-[1-(4-(N-(Dimethylamino-methylcarbonyl)-N-methyl-amino)-anilino)-1-(4-(2-carboxy-ethyl)-phenyl)-methylen]-6-chlor-2-indolinon
(3) 3-Z-[1-(4-(N-(2-Dimethylamino-ethyl)-N-acetyl-amino)-anilino)-1-(4-(2-carboxyethyl)-phenyl)-methylen]-6-chlor-2-indolinon
(4) 3-Z-[1-(4-(N-(2-Methylamino-ethyl)-N-acetyl-amino)-anilino)-1-(4-(2-carboxyethyl)-phenyl)-methylen]-6-chlor-2-indolinon
(5) 3-Z-[1-(4-(N-(3-Dimethylamino-propyl)-N-acetyl-amino)-anilino)-1-(4-(2-carboxyethyl)-phenyl)-methylen]-6-chlor-2-indolinon
(6) 3-Z-[1-(4-(N-(3-Methylamino-propyl)-N-acetyl-amino)-anilino)-1-(4-(2-carboxyethyl)-phenyl)-methylen]-6-chlor-2-indolinon
(7) 3-Z-[1-(4-(3-Dimethylamino-propyl)-anilino)-1-(4-(2-carboxy-ethyl)-phenyl)-methylen]-6-chlor-2-indolinon
(8) 3-Z-[1-(4-Ethylaminomethyl-anilino)-1-(4-(2-carboxy-ethyl)-phenyl)-methylen]-6-chlor-2-indolinon
(9) 3-Z-[1-(4-Methylaminomethyl-anilino)-1-(4-(2-carboxy-ethyl)-phenyl)-methylen]-6-chlor-2-indolinon
(10) 3-Z-[1-(4-(N-(4-Methyl-piperazin-1-yl-methylcarbonyl)-N-methyl-amino)-anilino)-1-(4-(2-carboxy-ethyl)-phenyl)-methylen]-6-chlor-2-indolinon
(11) 3-Z-[1-(4-(4-Methyl-piperazin-1-yl-carbonyl)-anilino)-1-(4-(2-carboxy-ethyl)-phenyl)-methylen]-6-chlor-2-indolinon
(12) 3-Z-[1-(4-(N-(3-Dimethylamino-propyl)-N-methylsulfonyl-amino)-anilino)-1-(4-(2-carboxy-ethyl)-phenyl)-methylen]-6-chlor-2-indolinon
(13) 3-Z-[1-(4-(N-(2-Dimethylamino-ethyl)-N-propylsulfonyl-amino)-anilino)-1-(4-(2-carboxy-ethyl)-phenyl)-methylen]-6-chlor-2-indolinon
(14) 3-Z-[1-(4-Aminomethyl-anilino)-1-(4-(2-carboxy-ethyl)-phenyl)-methylen]-6-chlor-2-indolinon
(15) 3-Z-[1-(3-(Methylamino-methyl)-anilino)-1-(4-(2-carboxy-ethyl)-phenyl)-methylen]-6-chlor-2-indolinon
(16) 3-Z-[1-(3-(2-Dimethylamino-ethyl)-anilino)-1-(4-(2-carboxy-ethyl)-phenyl)-methylen]-6-chlor-2-indolinon
(17) 3-Z-[1-(3-(3-Dimethylamino-propyl)-anilino)-1-(4-(2-carboxy-ethyl)-phenyl)-methylen]-6-chlor-2-indolinon
(18) 3-Z-[1-(4-(N-(Dimethylamino-carbonylmethyl)-N-methylsulfonyl-amino)-anilino)-1-(4-(2-carboxy-ethyl)-phenyl)-methylen]-6-chlor-2-indolinon
(19) 3-Z-[1-(4-(N-Methyl-N-methylsulfonyl-amino)-anilino)-1-(4-(2-carboxy-ethyl)-phenyl)-methylen]-6-chlor-2-indolinon
(20) 3-Z-[1-(4-(N-Methyl-N-acetyl-amino)-anilino)-1-(4-(2-carboxy-ethyl)-phenyl)-methylen]-6-chlor-2-indolinon
(21) 3-Z-[1-(4-(N-(N-(2-Dimethylamino-ethyl)-N-methyl-amino-methylcarbonyl)-N-methy(-amino)-anilino)-1-(4-(2-carboxy-ethyl)-phenyl)-methylen]-6-chlor-2-indolinon
(22) 3-Z-[1-(4-(2-Diethylamino-ethyl-sulfonyl)-anilino)-1-(4-(2-carboxy-ethyl)-phenyl)-methylen]-6-chlor-2-indolinon
(23) 3-Z-[1-(4-(N-(2-Dimethylamino-ethyl-carbonyl)-N-methyl-amino)-anilino)-1-(4-(2-carboxy-ethyl)-phenyl)-methylen]-6-chlor-2-indolinon
(24) 3-Z-[1-(4-(N-(2-Dimethylamino-ethyl)-N-methyl-amino-methyl)-anilino)-1-(4-(2-carboxy-ethyl)-phenyl)-methylen]-6-chlor-2-indolinon
(25) 3-Z-[1-(4-(2-Dimethylamino-ethoxy)-anilino)-1-(4-(2-carboxy-ethyl)-phenyl)-methylen]-6-chlor-2-indolinon
(26) 3-Z-[1-(4-(N-(4-Dimethylamino-butyl-carbonyl)-N-methyl-amino)-anilino)-1-(4-(2-carboxy-ethyl)-phenyl)-methylen]-6-chlor-2-indolinon
(27) 3-Z-[1-(4-(N-(3-Dimethylamino-propyl-carbonyl)-N-methyl-amino)-anilino)-1-(4-(2-carboxy-ethyl)-phenyl)-methylen]-6-chlor-2-indolinon
(28) 3-Z-[1-(4-(Methylethylamino-methyl)-anilino)-1-(4-(2-carboxy-ethyl)-phenyl)-methylen]-6-chlor-2-indolinon
(29) 3-Z-[1-(4-(Methylpropylamino-methyl)-anilino)-1-(4-(2-carboxy-ethyl)-phenyl)-methylen]-6-chlor-2-indolinon
(30) 3-Z-[1-(4-(Methylbenzylamino-methyl)-anilino)-1-(4-(2-carboxy-ethyl)-phenyl)-methylen]-6-chlor-2-indolinon
(31) 3-Z-[1-(4-(N-(2-Dimethylamino-ethyl)-N-methyl-amino-methyl)-anilino)-1-(4-(2-carboxy-ethyl)-phenyl-methylen]-6-chlor-2-indolinon
(32) 3-Z-[1-(4-(Azetidin-1-yl-methyl)-anilino)-1-(4-(2-carboxy-ethyl)-phenyl)-methylen]-6-chlor-2-indolinon
(33) 3-Z-[1-(4-((4-Methyl-piperazin-1-yl)-methyl)-anilino)-1-(4-(2-carboxy-ethyl)-phenyl)-methylen]-6-chlor-2-indolinon
(34) 3-Z-[1-(4-(Piperazin-1-yl-methyl)-anilino)-1-(4-(2-carboxy-ethyl)-phenyl)-methylen]-6-chlor-2-indolinon
(35) 3-Z-[1-(4-(Morpholin-4-yl-methyl)-anilino)-1-(4-(2-carboxy-ethyl)-phenyl)-methylen]-6-chlor-2-indolinon
(36) 3-Z-[1-(4-(Thiomorpholin-4-yl-methyl)-anilino)-1-(4-(2-carboxy-ethyl)-phenyl)-methylen]-6-chlor-2-indolinon
(37) 3-Z-[1-(4-(Imidazol-1-yl-methyl)-anilino)-1-(4-(2-carboxy-ethyl)-phenyl)-methylen]-6-chlor-2-indolinon
(38) 3-Z-[1-(4-(N-(2-Dimethylamino-ethyl)-N-methylsulfonyl-amino)-anilino)-1-(3-(2-carboxy-ethyl)-phenyl)-methylen]-6-chlor-2-indolinon
(39) 3-Z-[1-(4-(N-(Dimethylamino-methylcarbonyl)-N-methyl-amino)-anilino)-1-(3-(2-carboxy-ethyl)-phenyl)-methylen]-6-chlor-2-indolinon
(40) 3-Z-[1-(4-(N-(2-Dimethylamino-ethyl)-N-acetyl-amino)-anilino)-1-(3-(2-carboxy-ethyl)-phenyl)-methylen]-6-chlor-2-indolinon
(41) 3-Z-[1-(4-(N-(2-Methylamino-ethyl)-N-acetyl-amino)-anilino)-1-(3-(2-carboxyethyl)-phenyl)-methylen]-6-chlor-2-indolinon
(42) 3-Z-[1-(4-(N-(3-Dimethylamino-propyl)-N-acetyl-amino)-anilino)-1-(3-(2-carboxy-ethyl)-phenyl)-methylen]-6-chlor-2-indolinon
(43) 3-Z-[1-(4-(N-(3-Methylamino-propyl)-N-acetyl-amino)-anilino)-1-(3-(2-carboxyethyl)-phenyl)-methylen]-6-chlor-2-indolinon
(44) 3-Z-[1-(4-(3-Dimethylamino-propyl)-anilino)-1-(3-(2-carboxy-ethyl)-phenyl)-methylen]-6-chlor-2-indolinon
(45) 3-Z-[1-(4-Ethylaminomethyl-anilino)-1-(3-(2-carboxy-ethyl)-phenyl)-methylen]-6-chlor-2-indolinon
(46) 3-Z-[1-(4-Methylaminomethyl-anilino)-1-(3-(2-carboxy-ethyl)-phenyl)-methylen]-6-chlor-2-indolinon
(47) 3-Z-[1-(4-(N-(4-Methyl-piperazin-1-yl-methylcarbonyl)-N-methyl-amino)-anilino)-1-(3-(2-carboxy-ethyl)-phenyl)-methylen]-6-chlor-2-indolinon
(48) 3-Z-[1-(4-(4-Methyl-piperazin-1-yl-carbonyl)-anilino)-1-(3-(2-carboxy-ethyl)-phenyl)-methylen]-6-chlor-2-indolinon
(49) 3-Z-[1-(4-(N-(3-Dimethylamino-propyl)-N-methylsulfonyl-amino)-anilino)-1-(3-(2-carboxy-ethyl)-phenyl)-methylen]-6-chlor-2-indolinon
(50) 3-Z-[1-(4-(N-(2-Dimethylamino-ethyl)-N-propylsulfonyl-amino)-anilino)-1-(3-(2-carboxy-ethyl)-phenyl)-methylen]-6-chlor-2-indolinon
(51) 3-Z-[1-(4-Aminomethyl-anilino)-1-(3-(2-carboxy-ethyl)-phenyl)-methylen]-6-chlor-2-indolinon
(52) 3-Z-[1-(3-(Dimethylamino-methyl)-anilino)-1-(3-(2-carboxy-ethyl)-phenyl)-methylen]-6-chlor-2-indolinon
(53) 3-Z-[1-(3-(Methylamino-methyl)-anilino)-1-(3-(2-carboxy-ethyl)-phenyl)-methylen]-6-chlor-2-indolinon
(54) 3-Z-[1-(3-(2-Dimethylamino-ethyl)-anilino)-1-(3-(2-carboxy-ethyl)-phenyl)-methylen]-6-chlor-2-indolinon
(55) 3-Z-[1-(3-(3-Dimethylamino-propyl)-anilino)-1-(3-(2-carboxy-ethyl)-phenyl)-methylen]-6-chlor-2-indolinon
(56) 3-Z-[1-(4-(2-Dimethylamino-ethyl)-anilino)-1-(3-(2-carboxy-ethyl)-phenyl)-methylen]-6-chlor-2-indolinon
(57) 3-Z-[1-(4-(N-(Dimethylamino-carbonylmethyl)-N-methylsulfonyl-amino)-anilino)-1-(3-(2-carboxy-ethyl)-phenyl)-methylen]-6-chlor-2-indolinon
(58) 3-Z-[1-(4-(N-Methyl-N-methylsulfonyl-amino)-anilino)-1-(3-(2-carboxy-ethyl)-phenyl)-methylen]-6-chlor-2-indolinon
(59) 3-Z-[1-(4-(N-Methyl-N-acetyl-amino)-anilino)-1-(3-(2-carboxy-ethyl)-phenyl)-methylen]-6-chlor-2-indolinon
(60) 3-Z-[1-(4-(1-Methyl-imidazol-2-yl)-anilino)-1-(3-(2-carboxy-ethyl)-phenyl)-methylen]-6-chlor-2-indolinon
(61) 3-Z-[1-(4-(N-(N-(2-Dimethylamino-ethyl)-N-methyl-amino-methylcarbonyl)-N-methyl-amino)-anili no)-1-(3-(2-carboxy-ethyl)-phenyl)-methylen]-6-chlor-2-indolinon
(62) 3-Z-[1-(4-(2-Diethylamino-ethyl-sulfonyl)-anilino)-1-(3-(2-carboxy-ethyl)-phenyl)-methylen]-6-chlor-2-indolinon
(63) 3-Z-[1-(4-(N-(2-Dimethylamino-ethyl-carbonyl)-N-methyl-amino)-anilino)-1-(3-(2-carboxy-ethyl)-phenyl)-methylen]-6-chlor-2-indolinon
(64) 3-Z-[1-(4-(N-(2-Dimethylamino-ethyl)-N-methyl-amino-methyl)-anilino)-1-(3-(2-carboxy-ethyl)-phenyl)-methylen]-6-chlor-2-indolinon
(65) 3-Z-[1-(4-(2-Dimethylamino-ethoxy)-anilino)-1-(3-(2-carboxy-ethyl)-phenyl)-methylen]-6-chlor-2-indolinon
(66) 3-Z-[1-(4-(N-(4-Dimethylamino-butyl-carbonyl)-N-methyl-amino)-anilino)-1-(3-(2-carboxy-ethyl)-phenyl)-methylen]-6-chlor-2-indolinon
(67) 3-Z-[1-(4-(N-(3-Dimethylamino-propyl-carbonyl)-N-methyl-amino)-anilino)-1-(3-(2-carboxy-ethyl)-phenyl)-methylen]-6-chlor-2-indolinon
(68) 3-Z-[1-(4-(Methylethylamino-methyl)-anilino)-1-(3-(2-carboxy-ethyl)-phenyl)-methylen]-6-chlor-2-indolinon
(69) 3-Z-[1-(4-(Methylpropylamino-methyl)-anilino)-1-(3-(2-carboxy-ethyl)-phenyl)-methylen]-6-chlor-2-indolinon
(70) 3-Z-[1-(4-(Methylbenzylamino-methyl)-anilino)-1-(3-(2-carboxy-ethyl)-phenyl)-methylen]-6-chlor-2-indolinon
(71) 3-Z-[1-(4-(Diethylamino-methyl)-anilino)-1-(3-(2-carboxy-ethyl)-phenyl)-methylen]-6-chlor-2-indolinon
(72) 3-Z-[1-(4-(N-(2-Dimethylamino-ethyl)-N-methyl-amino-methyl)-anilino)-1-(3-(2-carboxy-ethyl)-phenyl-methylen]-6-chlor-2-indolinon
(73) 3-Z-[1-(4-(Pyrrolidin-1-yl-methyl)-anilino)-1-(3-(2-carboxy-ethyl)-phenyl)-methylen]-6-chlor-2-indolinon
(74) 3-Z-[1-(4-(Azetidin-1-yl-methyl)-anilino)-1-(3-(2-carboxy-ethyl)-phenyl)-methylen]-6-chlor-2-indolinon
(75) 3-Z-[1-(4-((4-Methyl-piperazin-1-yl)-methyl)-anilino)-1-(3-(2-carboxy-ethyl)-phenyl)-methylen]-6-chlor-2-indolinon
(76) 3-Z-[1-(4-(Piperazin-1-yl-methyl)-anilino)-1-(3-(2-carboxy-ethyl)-phenyl)-methylen]-6-chlor-2-indolinon
(77) 3-Z-[1-(4-(Morpholin-4-yl-methyl)-anilino)-1-(3-(2-carboxy-ethyl)-phenyl)-methylen]-6-chlor-2-indolinon
(78) 3-Z-[1-(4-(Thiomorpholin-4-yl-methyl)-anilino)-1-(3-(2-carboxy-ethyl)-phenyl)-methylen]-6-chlor-2-indolinon
(79) 3-Z-[1-(4-(Imidazol-1-yl-methyl)-anilino)-1-(3-(2-carboxy-ethyl)-phenyl)-methylen]-6-chlor-2-indolinon
(80) 3-Z-[1-(4-(N-(2-Methylamino-ethyl)-N-acetyl-amino)-anilino)-1-(4-(2-carboxyethyl)-phenyl)-methylen]-6-fluor-2-indolinon
(81) 3-Z-[1-(4-(N-(3-Methylamino-propyl)-N-acetyl-amino)-anilino)-1-(4-(2-carboxyethyl)-phenyl)-methylen]-6-fluor-2-indolinon
(82) 3-Z-[1-(4-(3-Dimethylamino-propyl)-anilino)-1-(4-(2-carboxy-ethyl)-phenyl)-methylen]-6-fluor-2-indolinon
(83) 3-Z-[1-(4-Ethylaminomethyl-anilino)-1-(4-(2-carboxy-ethyl)-phenyl)-methylen]-6-fluor-2-indolinon
(84) 3-Z-[1-(4-(N-(3-Dimethylamino-propyl)-N-methylsulfonyl-amino)-anilino)-1-(4-(2-carboxy-ethyl)-phenyl)-methylen]-6-fluor-2-indolinon
(85) 3-Z-[1-(4-(N-(2-Dimethylamino-ethyl)-N-propylsulfonyl-amino)-anilino)-1-(4-(2-carboxy-ethyl)-phenyl)-methylen]-6-fluor-2-indolinon
(86) 3-Z-[1-(3-(Methylamino-methyl)-anilino)-1-(4-(2-carboxy-ethyl)-phenyl)-methylen]-6-fluor-2-indolinon
(87) 3-Z-[1-(3-(2-Dimethylamino-ethyl)-anilino)-1-(4-(2-carboxy-ethyl)-phenyl)-methylen]-6-fluor-2-indolinon
(88) 3-Z-[1-(3-(3-Dimethylamino-propyl)-anilino)-1-(4-(2-carboxy-ethyl)-phenyl)-methylen]-6-fluor-2-indolinon
(89) 3-Z-[1-(4-(N-(Dimethylamino-carbonylmethyl)-N-methylsulfonyl-amino)-anilino)-1-(4-(2-carboxy-ethyl)-phenyl)-methylen]-6-fluor-2-indolinon
(90) 3-Z-[1-(4-(N-Methyl-N-methylsulfonyl-amino)-anilino)-1-(4-(2-carboxy-ethyl)-phenyl)-methylen]-6-fluor-2-indolinon
(91) 3-Z-[1-(4-(N-(N-(2-Dimethylamino-ethyl)-N-methyl-amino-methylcarbonyl)-N-methyl-amino)-anilino)-1-(4-(2-carboxy-ethyl)-phenyl)-methylen]-6-fluor-2-indolinon
(92) 3-Z-[1-(4-(2-Diethylamino-ethyl-sulfonyl)-anilino)-1-(4-(2-carboxy-ethyl)-phenyl)-methylen]-6-fluor-2-indolinon
(93) 3-Z-[1-(4-(2-Dimethylamino-ethoxy)-anilino)-1-(4-(2-carboxy-ethyl)-phenyl)-methylen]-6-fluor-2-indolinon
(94) 3-Z-[1-(4-(N-(3-Dimethylamino-propyl-carbonyl)-N-methyl-amino)-anilino)-1-(4-(2-carboxy-ethyl)-phenyl)-methylen]-6-fluor-2-indolinon
(95) 3-Z-[1-(4-(Methylethylamino-methyl)-anilino)-1-(4-(2-carboxy-ethyl)-phenyl)-methylen]-6-fluor-2-indolinon
(96) 3-Z-[1-(4-(Methylpropylamino-methyl)-anilino)-1-(4-(2-carboxy-ethyl)-phenyl)-methylen]-6-fluor-2-indolinon
(97) 3-Z-[1-(4-(Methylbenzylamino-methyl)-anilino)-1-(4-(2-carboxy-ethyl)-phenyl)-methylen]-6-fluor-2-indolinon
(98) 3-Z-[1-(4-(Azetidin-1-yl-methyl)-anilino)-1-(4-(2-carboxy-ethyl)-phenyl)-methylen]-6-fluor-2-indolinon
(99) 3-Z-[1-(4-(Piperazin-1-yl-methyl)-anilino)-1-(4-(2-carboxy-ethyl)-phenyl)-methylen]-6-fluor-2-indolinon
(100) 3-Z-[1-(4-(Morpholin-4-yl-methyl)-anilino)-1-(4-(2-carboxy-ethyl)-phenyl)-methylen]-6-fluor-2-indolinon
(101) 3-Z-[1-(4-(Thiomorpholin-4-yl-methyl)-anilino)-1-(4-(2-carboxy-ethyl)-phenyl)-methylen]-6-fluor-2-indolinon
(102) 3-Z-[1-(4-(N-(2-Dimethylamino-ethyl)-N-acetyl-amino)-anilino)-1-(3-(2-carboxyethyl)-phenyl)-methylen]-6-fluor-2-indolinon
(103) 3-Z-[1-(4-(N-(2-Methylamino-ethyl)-N-acetyl-amino)-anilino)-1-(3-(2-carboxyethyl)-phenyl)-methylen]-6-fluor-2-indolinon
(104) 3-Z-[1-(4-(N-(3-Methylamino-propyl)-N-acetyl-amino)-anilino)-1-(3-(2-carboxyethyl)-phenyl)-methylen]-6-fluor-2-indolinon
(105) 3-Z-[1-(4-(3-Dimethylamino-propyl)-anilino)-1-(3-(2-carboxy-ethyl)-phenyl)-methylen]-6-fluor-2-indolinon
(106) 3-Z-[1-(4-Ethylaminomethyl-anilino)-1-(3-(2-carboxy-ethyl)-phenyl)-methylen]-6-fluor-2-indolinon
(107) 3-Z-[1-(4-(4-Methyl-piperazin-1-yl-carbonyl)-anilino)-1-(3-(2-carboxy-ethyl)-phenyl)-methylen]-6-fluor-2-indolinon
(108) 3-Z-[1-(4-(N-(3-Dimethylamino-propyl)-N-methylsulfonyl-amino)-anilino)-1-(3-(2-carboxy-ethyl)-phenyl)-methylen]-6-fluor-2-indolinon
(109) 3-Z-[1-(4-(N-(2-Dimethylamino-ethyl)-N-propylsulfonyl-amino)-anilino)-1-(3-(2-carboxy-ethyl)-phenyl)-methylen]-6-fluor-2-indolinon
(110) 3-Z-[1-(3-(Methylamino-methyl)-anilino)-1-(3-(2-carboxy-ethyl)-phenyl)-methylen]-6-fluor-2-indolinon
(111) 3-Z-[1-(3-(2-Dimethylamino-ethyl)-anilino)-1-(3-(2-carboxy-ethyl)-phenyl)-methylen]-6-fluor-2-indolinon
(112) 3-Z-[1-(3-(3-Dimethylamino-propyl)-anilino)-1-(3-(2-carboxy-ethyl)-phenyl)-methylen]-6-fluor-2-indolinon
(113) 3-Z-[1-(4-(N-(Dimethylamino-carbonylmethyl)-N-methylsulfonyl-amino)-anilino)-1-(3-(2-carboxy-ethyl)-phenyl)-methylen]-6-fluor-2-indolinon
(114) 3-Z-[1-(4-(N-Methyl-N-methylsulfonyl-amino)-anilino)-1-(3-(2-carboxy-ethyl)-phenyl)-methylen]-6-fluor-2-indolinon
(115) 3-Z-[1-(4-(N-Methyl-N-acetyl-amino)-anilino)-1-(3-(2-carboxy-ethyl)-phenyl)-methylen]-6-fluor-2-indolinon
(116) 3-Z-[1-(4-(N-(N-(2-Dimethylamino-ethyl)-N-methyl-amino-methylcarbonyl)-N-methyl-amino)-anilino)-1-(3-(2-carboxy-ethyl)-phenyl)-methylen]-6-fluor-2-indolinon
(117) 3-Z-[1-(4-(2-Diethylamino-ethyl-sulfonyl)-anilino)-1-(3-(2-carboxy-ethyl)-phenyl)-methylen]-6-fluor-2-indolinon
(118) 3-Z-[1-(4-(N-(2-Dimethylamino-ethyl-carbonyl)-N-methyl-amino)-anilino)-1-(3-(2-carboxy-ethyl)-phenyl)-methylen]-6-fluor-2-indolinon
(119) 3-Z-[1-(4-(N-(2-Dimethylamino-ethyl)-N-methyl-amino-methyl)-anilino)-1-(3-(2-carboxy-ethyl)-phenyl)-methylen]-6-fluor-2-indolinon
(120) 3-Z-[1-(4-(2-Dimethylamino-ethoxy)-anilino)-1-(3-(2-carboxy-ethyl)-phenyl)-methylen]-6-fluor-2-indolinon
(121) 3-Z-[1-(4-(Methylethylamino-methyl)-anilino)-1-(3-(2-carboxy-ethyl)-phenyl)-methylen]-6-fluor-2-indolinon
(122) 3-Z-[1-(4-(Methylpropylamino-methyl)-anilino)-1-(3-(2-carboxy-ethyl)-phenyl)-methylen]-6-fluor-2-indolinon
(123) 3-Z-[1-(4-(Methylbenzylamino-methyl)-anilino)-1-(3-(2-carboxy-ethyl)-phenyl)-methylen]-6-fluor-2-indolinon
(124) 3-Z-[1-(4-(Diethylamino-methyl)-anilino)-1-(3-(2-carboxy-ethyl)-phenyl)-methylen]-6-fluor-2-indolinon
(125) 3-Z-[1-(4-(Pyrrolidin-1-yl-methyl)-anilino)-1-(3-(2-carboxy-ethyl)-phenyl)-methylen]-6-fluor-2-indolinon
(126) 3-Z-[1-(4-(Azetidin-1-yl-methyl)-anilino)-1-(3-(2-carboxy-ethyl)-phenyl)-methylen]-6-fluor-2-indolinon
(127) 3-Z-[1-(4-(Piperazin-1-yl-methyl)-anilino)-1-(3-(2-carboxy-ethyl)-phenyl)-methylen]-6-fluor-2-indolinon
(128) 3-Z-[1-(4-(Morpholin-4-yl-methyl)-anilino)-1-(3-(2-carboxy-ethyl)-phenyl)-methylen]-6-fluor-2-indolinon
(129) 3-Z-[1-(4-(Thiomorpholin-4-yl-methyl)-anilino)-1-(3-(2-carboxy-ethyl)-phenyl)-methylen]-6-fluor-2-indolinon
(130) 3-Z-[1-(4-(N-(2-Dimethylamino-ethyl)-N-methylsulfonyl-amino)-anilino)-1-(4-(2-carboxy-ethyl)-phenyl)-methylen]-6-brom-2-indolinon
(131) 3-Z-[1-(4-(N-(Dimethylamino-methylcarbonyl)-N-methyl-amino)-anilino)-1-(4-(2-carboxy-ethyl)-phenyl)-methylen]-6-brom-2-indolinon
(132) 3-Z-[1-(4-(N-(2-Dimethylamino-ethyl)-N-acetyl-amino)-anilino)-1-(4-(2-carboxyethyl)-phenyl)-methylen]-6-brom-2-indolinon
(133) 3-Z-[1-(4-(N-(2-Methylamino-ethyl)-N-acetyl-amino)-anilino)-1-(4-(2-carboxyethyl)-phenyl)-methylen]-6-brom-2-indolinon
(134) 3-Z-[1-(4-(N-(3-Dimethylamino-propyl)-N-acetyl-amino)-anilino)-1-(4-(2-carboxy-ethyl)-phenyl)-methylen]-6-brom-2-indolinon
(135) 3-Z-[1-(4-(N-(3-Methylamino-propyl)-N-acetyl-amino)-anilino)-1-(4-(2-carboxyethyl)-phenyl)-methylen]-6-brom-2-indolinon
(136) 3-Z-[1-(4-(3-Dimethylamino-propyl)-anilino)-1-(4-(2-carboxy-ethyl)-phenyl)-methylen]-6-brom-2-indolinon
(137) 3-Z-[1-(4-Ethylaminomethyl-anilino)-1-(4-(2-carboxy-ethyl)-phenyl)-methylen]-6-brom-2-indolinon
(138) 3-Z-[1-(4-Methylaminomethyl-anilino)-1-(4-(2-carboxy-ethyl)-phenyl)-methylen]-6-brom-2-indolinon
(139) 3-Z-[1-(4-(N-(4-Methyl-piperazin-1-yl-methylcarbonyl)-N-methyl-amino)-anilino)-1-(4-(2-carboxy-ethyl)-phenyl)-methylen]-6-brom-2-indolinon
(140) 3-Z-[1-(4-(4-Methyl-piperazin-1-yl-carbonyl)-anilino)-1-(4-(2-carboxy-ethyl)-phenyl)-methylen]-6-brom-2-indolinon
(141) 3-Z-(1-(4-(N-(3-Dimethylamino-propyl)-N-methylsulfonyl-amino)-anilino)-1-(4-(2-carboxy-ethyl)-phenyl)-methylen]-6-brom-2-indolinon
(142) 3-Z-[1-(4-(N-(2-Dimethylamino-ethyl)-N-propylsulfonyl-amino)-anilino)-1-(4-(2-carboxy-ethyl)-phenyl)-methylen]-6-brom-2-indolinon
(143) 3-Z-[1-(4-Aminomethyl-anilino)-1-(4-(2-carboxy-ethyl)-phenyl)-methylen]-6-brom-2-indolinon
(144) 3-Z-[1-(3-(Dimethylamino-methyl)-anilino)-1-(4-(2-carboxy-ethyl)-phenyl)-methylen]-6-brom-2-indolinon
(145) 3-Z-[1-(3-(Methylamino-methyl)-anilino)-1-(4-(2-carboxy-ethyl)-phenyl)-methylen]-6-brom-2-indolinon
(146) 3-Z-[1-(3-(2-Dimethylamino-ethyl)-anilino)-1-(4-(2-carboxy-ethyl)-phenyl)-methylen]-6-brom-2-indolinon
(147) 3-Z-[1-(3-(3-Dimethylamino-propyl)-anilino)-1-(4-(2-carboxy-ethyl)-phenyl)-methylen]-6-brom-2-indolinon
(148) 3-Z-[1-(4-(2-Dimethylamino-ethyl)-anilino)-1-(4-(2-carboxy-ethyl)-phenyl)-methylen]-6-brom-2-indolinon
(149) 3-Z-[1-(4-(N-(Dimethylamino-carbonylmethyl)-N-methylsulfonyl-amino)-anilino)-1-(4-(2-carboxy-ethyl)-phenyl)-methylen]-6-brom-2-indolinon
(150) 3-Z-[1-(4-(N-Methyl-N-methylsulfonyl-amino)-anilino)-1-(4-(2-carboxy-ethyl)-phenyl)-methylen]-6-brom-2-indolinon
(151) 3-Z-[1-(4-(N-Methyl-N-acetyl-amino)-anilino)-1-(4-(2-carboxy-ethyl)-phenyl)-methylen]-6-brom-2-indolinon
(152) 3-Z-[1-(4-(1-Methyl-imidazol-2-yl)-anilino)-1-(4-(2-carboxy-ethyl)-phenyl)-methylen]-6-brom-2-indolinon
(153) 3-Z-[1-(4-(N-(N-(2-Dimethylamino-ethyl)-N-methyl-amino-methylcarbonyl)-N-methyl-amino)-anilino)-1-(4-(2-carboxy-ethyl)-phenyl)-methylen]-6-brom-2-indolinon
(154) 3-Z-[1-(4-(2-Diethylamino-ethyl-sulfonyl)-anilino)-1-(4-(2-carboxy-ethyl)-phenyl)-methylen]-6-brom-2-indolinon
(155) 3-Z-[1-(4-(N-(2-Dimethylamino-ethyl-carbonyl)-N-methyl-amino)-anilino)-1-(4-(2-carboxy-ethyl)-phenyl)-methylen]-6-brom-2-indolinon
(156) 3-Z-[1-(4-(N-(2-Dimethylamino-ethyl)-N-methyl-amino-methyl)-anilino)-1-(4-(2-carboxy-ethyl)-phenyl)-methylen]-6-brom-2-indolinon
(157) 3-Z-[1-(4-(2-Dimethylamino-ethoxy)-anilino)-1-(4-(2-carboxy-ethyl)-phenyl)-methylen]-6-brom-2-indolinon
(158) 3-Z-[1-(4-(N-(4-Dimethylamino-butyl-carbonyl)-N-methyl-amino)-anilino)-1-(4-(2-carboxy-ethyl)-phenyl)-methylen]-6-brom-2-indolinon
(159) 3-Z-[1-(4-(N-(3-Dimethylamino-propyl-carbonyl)-N-methyl-amino)-anilino)-1-(4-(2-carboxy-ethyl)-phenyl)-methylen]-6-brom-2-indolinon
(160) 3-Z-[1-(4-(Methylethylamino-methyl)-anilino)-1-(4-(2-carboxy-ethyl)-phenyl)-methylen]-6-brom-2-indolinon
(161) 3-Z-[1-(4-(Methylpropylamino-methyl)-anilino)-1-(4-(2-carboxy-ethyl)-phenyl)-methylen]-6-brom-2-indolinon
(162) 3-Z-[1-(4-(Methylbenzylamino-methyl)-anilino)-1-(4-(2-carboxy-ethyl)-phenyl)-methylen]-6-brom-2-indolinon
(163) 3-Z-[1-(4-(Diethylamino-methyl)-anilino)-1-(4-(2-carboxy-ethyl)-phenyl)-methylen]-6-brom-2-indolinon
(164) 3-Z-[1-(4-(N-(2-Dimethylamino-ethyl)-N-methyl-amino-methyl)-anilino)-1-(4-(2-carboxy-ethyl)-phenyl-methylen]-6-brom-2-indolinon
(165) 3-Z-[1-(4-(Pyrrolidin-1-yl-methyl)-anilino)-1-(4-(2-carboxy-ethyl)-phenyl)-methylen]-6-brom-2-indolinon
(166) 3-Z-[1-(4-(Azetidin-1-yl-methyl)-anilino)-1-(4-(2-carboxy-ethyl)-phenyl)-methylen]-6-brom-2-indolinon
(167) 3-Z-[1-(4-((4-Methyl-piperazin-1-yl)-methyl)-anilino)-1-(4-(2-carboxy-ethyl)-phenyl)-methylen]-6-brom-2-indolinon
(168) 3-Z-[1-(4-(Piperazin-1-yl-methyl)-anilino)-1-(4-(2-carboxy-ethyl)-phenyl)-methylen]-6-brom-2-indolinon
(169) 3-Z-[1-(4-(Morpholin-4-yl-methyl)-anilino)-1-(4-(2-carboxy-ethyl)-phenyl)-methylen]-6-brom-2-indolinon
(170) 3-Z-[1-(4-(Thiomorpholin-4-yl-methyl)-anilino)-1-(4-(2-carboxy-ethyl)-phenyl)-methylen]-6-brom-2-indolinon
(171) 3-Z-[1-(4-(Imidazol-1-yl-methyl)-anilino)-1-(4-(2-carboxy-ethyl)-phenyl)-methylen]-6-brom-2-indolinon
(172) 3-Z-[1-(4-Dimethylaminomethyl-anilino)-1-(3-(2-carboxy-ethyl)-phenyl)-methylen]-6-brom-2-indolinon
(173) 3-Z-[1-(4-(N-(2-Dimethylamino-ethyl)-N-methylsulfonyl-amino)-änilino)-1-(3-(2-carboxy-ethyl)-phenyl)-methylen]-6-brom-2-indolinon
(174) 3-Z-[1-(4-(N-(Dimethylamino-methylcarbonyl)-N-methyl-amino)-anilino)-1-(3-(2-carboxy-ethyl)-phenyl)-methylen]-6-brom-2-indolinon
(175) 3-Z-[1-(4-(N-(2-Dimethylamino-ethyl)-N-acetyl-amino)-anilino)-1-(3-(2-carboxyethyl)-phenyl)-methylen]-6-brom-2-indolinon
(176) 3-Z-[1-(4-(N-(2-Methylamino-ethyl)-N-acetyl-amino)-anilino)-1-(3-(2-carboxyethyl)-phenyl)-methylen]-6-brom-2-indolinon
(177) 3-Z-[1-(4-(N-(3-Dimethylamino-propyl)-N-acetyl-amino)-anilino)-1-(3-(2-carboxy-ethyl)-phenyl)-methylen]-6-brom-2-indolinon
(178) 3-Z-[1-(4-(N-(3-Methylamino-propyl)-N-acetyl-amino)-anilino)-1-(3-(2-carboxyethyl)-phenyl)-methylen]-6-brom-2-indolinon
(179) 3-Z-[1-(4-(3-Dimethylamino-propyl)-anilino)-1-(3-(2-carboxy-ethyl)-phenyl)-methylen]-6-brom-2-indolinon
(180) 3-Z-[1-(4-Ethylaminomethyl-anilino)-1-(3-(2-carboxy-ethyl)-phenyl)-methylen]-6-brom-2-indolinon
(181) 3-Z-[1-(4-Methylaminomethyl-anilino)-1-(3-(2-carboxy-ethyl)-phenyl)-methylen]-6-brom-2-indolinon
(182) 3-Z-[1-(4-(N-(4-Methyl-piperazin-1-yl-methylcarbonyl)-N-methyl-amino)-anilino)-1-(3-(2-carboxy-ethyl)-phenyl)-methylen]-6-brom-2-indolinon
(183) 3-Z-[1-(4-(4-Methyl-piperazin-1-yl-carbonyl)-anilino)-1-(3-(2-carboxy-ethyl)-phenyl)-methylen]-6-brom-2-indolinon
(184) 3-Z-[1-(4-(N-(3-Dimethylamino-propyl)-N-methylsulfonyl-amino)-anilino)-1-(3-(2-carboxy-ethyl)-phenyl)-methylen]-6-brom-2-indolinon
(185) 3-Z-[1-(4-(N-(2-Dimethylamino-ethyl)-N-propylsulfonyl-amino)-anilino)-1-(3-(2-carboxy-ethyl)-phenyl)-methylen]-6-brom-2-indolinon
(186) 3-Z-[1-(4-Aminomethyl-anilino)-1-(3-(2-carboxy-ethyl)-phenyl)-methylen]-6-brom-2-indolinon
(187) 3-Z-[1-(3-(Dimethylamino-methyl)-anilino)-1-(3-(2-carboxy-ethyl)-phenyl)-methylen]-6-brom-2-indolinon
(188) 3-Z-[1-(3-(Methylamino-methyl)-anilino)-1-(3-(2-carboxy-ethyl)-phenyl)-methylen]-6-brom-2-indolinon
(189) 3-Z-[1-(3-(2-Dimethylamino-ethyl)-anilino)-1-(3-(2-carboxy-ethyl)-phenyl)-methylen]-6-brom-2-indolinon
(190) 3-Z-[1-(3-(3-Dimethylamino-propyl)-anilino)-1-(3-(2-carboxy-ethyl)-phenyl)-methylen]-6-brom-2-indolinon
(191) 3-Z-[1-(4-(2-Dimethylamino-ethyl)-anilino)-1-(3-(2-carboxy-ethyl)-phenyl)-methylen]-6-brom-2-indolinon
(192) 3-Z-[1-(4-(N-(Dimethylamino-carbonylmethyl)-N-methylsulfonyl-amino)-anilino)-1-(3-(2-carboxy-ethyl)-phenyl)-methylen]-6-brom-2-indolinon
(193) 3-Z-[1-(4-(N-Methyl-N-methylsulfonyl-amino)-anilino)-1-(3-(2-carboxy-ethyl)-phenyl)-methylen]-6-brom-2-indolinon
(194) 3-Z-[1-(4-(N-Methyl-N-acetyl-amino)-anilino)-1-(3-(2-carboxy-ethyl)-phenyl)-methylen]-6-brom-2-indolinon
(195) 3-Z-[1-(4-(1-Methyl-imidazol-2-yl)-anilino)-1-(3-(2-carboxy-ethyl)-phenyl)-methylen]-6-brom-2-indolinon
(196) 3-Z-[1-(4-(N-(N-(2-Dimethylamino-ethyl)-N-methyl-amino-methylcarbonyl)-N-methyl-amino)-anilino)-1-(3-(2-carboxy-ethyl)-phenyl)-methylen]-6-brom-2-indolinon
(197) 3-Z-[1-(4-(2-Diethylamino-ethyl-sulfonyl)-anilino)-1-(3-(2-carboxy-ethyl)-phenyl)-methylen]-6-brom-2-indolinon.
(198) 3-Z-[1-(4-(N-(2-Dimethylamino-ethyl-carbonyl)-N-methyl-amino)-anilino)-1-(3-(2-carboxy-ethyl)-phenyl)-methylen]-6-brom-2-indolinon
(199) 3-Z-[1-(4-(N-(2-Dimethylamino-ethyl)-N-methyl-amino-methyl)-anilino)-1-(3-(2-carboxy-ethyl)-phenyl)-methylen]-6-brom-2-indolinon
(200) 3-Z-[1-(4-(2-Dimethylamino-ethoxy)-anilino)-1-(3-(2-carboxy-ethyl)-phenyl)-methylen]-6-brom-2-indolinon
(201) 3-Z-[1-(4-(N-(4-Dimethylamino-butyl-carbonyl)-N-methyl-amino)-anilino)-1-(3-(2-carboxy-ethyl)-phenyl)-methylen]-6-brom-2-indolinon
(202) 3-Z-[1-(4-(N-(3-Dimethylamino-propyl-carbonyl)-N-methyl-amino)-anilino)-1-(3-(2-carboxy-ethyl)-phenyl)-methylen]-6-brom-2-indolinon
(203) 3-Z-[1-(4-(Methylethylamino-methyl)-anilino)-1-(3-(2-carboxy-ethyl)-phenyl)-methylen]-6-brom-2-indolinon
(204) 3-Z-[1-(4-(Methylpropylamino-methyl)-anilino)-1-(3-(2-carboxy-ethyl)-phenyl)-methylen]-6-brom-2-indolinon
(205) 3-Z-[1-(4-(Methylbenzylamino-methyl)-anilino)-1-(3-(2-carboxy-ethyl)-phenyl)-methylen]-6-brom-2-indolinon
(206) 3-Z-[1-(4-(Diethylamino-methyl)-anilino)-1-(3-(2-carboxy-ethyl)-phenyl)-methylen]-6-brom-2-indolinon
(207) 3-Z-[1-(4-(N-(2-Dimethylamino-ethyl)-N-methyl-amino-methyl)-anilino)-1-(3-(2-carboxy-ethyl)-phenyl-methylen]-6-brom-2-indolinon
(208) 3-Z-[1-(4-(Pyrrolidin-1-yl-methyl)-anilino)-1-(3-(2-carboxy-ethyl)-phenyl)-methylen]-6-brom-2-indolinon
(209) 3-Z-[1-(4-(Azetidin-1-yl-methyl)-anilino)-1-(3-(2-carboxy-ethyl)-phenyl)-methylen]-6-brom-2-indolinon
(210) 3-Z-[1-(4-((4-Methyl-piperazin-1-yl)-methyl)-anilino)-1-(3-(2-carboxy-ethyl)-phenyl)-methylen]-6-brom-2-indolinon
(211) 3-Z-[1-(4-(Piperazin-1-yl-methyl)-anilino)-1-(3-(2-carboxy-ethyl)-phenyl)-methylen]-6-brom-2-indolinon
(212) 3-Z-[1-(4-(Morpholin-4-yl-methyl)-anilino)-1-(3-(2-carboxy-ethyl)-phenyl)-methylen]-6-brom-2-indolinon
(213) 3-Z-[1-(4-(Thiomorpholin-4-yl-methyl)-anilino)-1-(3-(2-carboxy-ethyl)-phenyl)-methylen]-6-brom-2-indolinon
(214) 3-Z-[1-(4-(Imidazol-1-yl-methyl)-anilino)-1-(3-(2-carboxy-ethyl)-phenyl)-methylen]-6-brom-2-indolinon
(215) 3-Z-[1-(4-Dimethylaminomethyl-anilino)-1-(4-carboxymethylamino-phenyl)-methylen]-6-fluor-2-indolinon
(216) 3-Z-[1-(4-Dimethylaminomethyl-anilino)-1-(3-carboxymethylamino-phenyl)-methylen]-6-fluor-2-indolinon
(217) 3-Z-[1-(4-Dimethylaminomethyl-anilino)-1-(4-(N-methyl-carboxymethylamino)-phenyl)-methylen]-6-fluor-2-indolinon
(218) 3-Z-[1-(4-Dimethylaminomethyl-anilino)-1-(3-(N-methyl-carboxymethylamino)-phenyl)-methylen]-6-fluor-2-indolinon
(219) 3-Z-[1-(4-Dimethylaminomethyl-anilino)-1-(4-carboxymethoxy-phenyl)-methylen]-6-chlor-2-indolinon
(220) 3-Z-[1-(4-Dimethylaminomethyl-anilino)-1-(3-carboxymethoxy-phenyl)-methylen]-6-chlor-2-indolinon
(221) 3-Z-[1-(4-Dimethylaminomethyl-anilino)-1-(4-carboxymethylamino-phenyl)-methylen]-6-chlor-2-indolinon
(222) 3-Z-[1-(4-Dimethylaminomethyl-anilino)-1-(3-carboxymethylamino-phenyl)-methylen]-6-chlor-2-indolinon
(223) 3-Z-[1-(4-Dimethylaminomethyl-anilino)-1-(4-(N-methyl-carboxymethylamino)-phenyl)-methylen]-6-chlor-2-indolinon
(224) 3-Z-[1-(4-Dimethylaminomethyl-anilino)-1-(3-(N-methyl-carboxymethylamino)-phenyl)-methylen]-6-chlor-2-indolinon
(225) 3-Z-[1-(4-Dimethylaminomethyl-anilino)-1-(4-carboxymethoxy-phenyl)-methylen]-6-brom-2-indolinon
(226) 3-Z-[1-(4-Dimethylaminomethyl-anilino)-1-(3-carboxymethoxy-phenyl)-methylen]-6-brom-2-indolinon
(227) 3-Z-[1-(4-Dimethylaminomethyl-anilino)-1-(4-carboxymethylamino-phenyl)-methylen]-6-brom-2-indolinon
(228) 3-Z-[1-(4-Dimethylaminomethyl-anilino)-1-(3-carboxymethylamino-phenyl)-methylen]-6-brom-2-indolinon
(229) 3-Z-[1-(4-Dimethylaminomethyl-anilino)-1-(4-(N-methyl-carboxymethylamino)-phenyl)-methylen]-6-brom-2-indolinon
(230) 3-Z-[1-(4-Dimethylaminomethyl-anilino)-1-(3-(N-methyl-carboxymethylamino)-phenyl)-methylen]-6-brom-2-indolinon

In den obigen Tabellen bedeuten

| | |
|---|---|
| Me | Methyl, |
| Et | Ethyl, |
| Pr | Propyl, |
| nPr | n-Propyl, |
| iPr | Isopropyl, |
| nBu | n-Butyl, |
| tBu | tert.-Butyl und |
| Bn | Benzyl. |

## Patentansprüche

1. Verbindungen der allgemeinen Formel 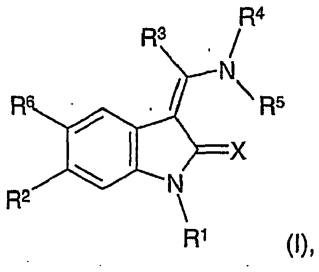 in der
X ein Sauerstoffatom,
R¹ ein Wasserstoffatom,
R² ein Fluor-, Chlor- oder Brom-atom oder eine Cyanogruppe,
R³ eine Phenylgruppe in 3- oder 4-Position
durch eine Carboxy-C₁₋₃-alkyl-, C₁₋₄-Alkoxy-carbonyl-C₁₋₃-alkyl-, Aminocarbonyl-C₁₋₃-alkyl-, (C₁₋₂-Alkylamino)-carbonyl-C₁₋₃-alkyl-C₁₋₄-Alkoxy-carbonyl-C₂₋₃-alkenyl-gruppe,
substituiert
R⁴ eine Phenylgruppe oder eine
durch eine endständig durch eine Amino-, Guanidino-, Mono- oder Di-(C₁₋₂-alkyl)-amino-, *N*-[ω-Di-(C₁₋₃-alkyl)-amino-C₂₋₃-alkyl]-*N*-(C₁₋₃-alkyl)-amino-, N-Methyl-(C₃₋₄-alkyl)-amino-, N-(C₁₋₃-Alkyl)-N-benzylamino-, N-(C₁₋₄-Alkoxycarbonyl)-amino-, N-(C₁₋₄-Alkoxycarbonyl)-C₁₋₄-alkylamino-, 4-(C₁₋₃-Alkyl)-piperazin-1-yl-, Imidazol-1-yl-, Pyrrolidin-1-yl-, Azetidin-1-yl-, Morpholin-4-yl-, Piperazin-1-yl-, Thiomorpholin-4-yl-gruppe substituierte C₁₋₃-Alkylgruppe,
durch eine Di-(C₁₋₃-alkyl)-amino-(C₁₋₃-alkyl)-sulfonyl-, 2-[Di-(C₁₋₃-alkyl)-amino]-ethoxy-, 4-(C₁₋₃-Alkyl)-piperazin-1-yl-carbonyl-, {ω-[Di-(C₁₋₃-alkyl)-amino]-(C₂₋₃-alkyl)}-N-(C₁₋₃-alkyl)-amino-carbonyl-, 1-(C₁₋₃-Alkyl)-imidazol-2-yl-, (C₁₋₃-Alkyl)-sulfonyl-gruppe, oder
durch eine Gruppe der Formel 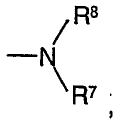 in der
R⁷ eine C₁₋₂-Alkyl-, C₁₋₂-Alkyl-carbonyl-, Di-(C₁₋₂-alkyl)-amino-carbonyl-C₁₋₃-alkyl- oder C₁₋₃-Alkylsulfonyl-gruppe und
R⁸ eine C₁₋₃-Alkyl-, ω-[Di-(C₁₋₂-alkyl)-amino]-C₂₋₃-alkyl-, ω[Mono-(C₁₋₂-alkyl)-amino]-C₂₋₃-alkyl-gruppe, oder
eine endständig durch eine Di-(C₁₋₂-alkyl)-amino-, Piperazin -1-yl- oder 4-(C₁₋₃-Alkyl)-piperazin-1-yl-gruppe substituierte (C₁₋₃-Alkyl)-carbonyl-, (C₄₋₆-Alkyl)-carbonyl-, oder Carbonyl-(C₁₋₃-alkyl)-gruppe bedeuten,
monosubstituierte Phenylgruppe ist,
wobei alle im Rest R⁴ enthaltenen Dialkylaminogruppen auch in quaternisierter Form vorliegen können, beispielsweise als N-Methyl-(N,N-dialkyl)-ammoniumgruppe, wobei das Gegenion vorzugsweise ausgewählt ist aus Iodid, Chlorid, Bromid, Methylsulfonat, para-Toluolsulfonat, oder Trifluoracetat,
R⁵ ein Wasserstoffatom und
R⁶ ein Wasserstoffatom bedeuten,
wobei die oben erwähnten Alkylgruppen lineare und verzweigten Alkylgruppen einschließen, in denen zusätzlich ein bis 3 Wasserstoffatome durch Fluoratome ersetzt sein können,
deren Tautomere, Enantiomere, Diastereomere, deren Gemische und deren Salze.

2. Verbindungen der allgemeinen Formel I gemäß Anspruch 1, in der
X, R¹, R², R⁴, R⁵ und R⁶ wie in Anspruch 1 definiert sind und
R³ eine
durch eine Carboxy-C₁₋₃-alkyl-, C₁₋₄-Alkoxy-carbonyl-C₁₋₃-alkyl-, Aminocarbonyl-C₁₋₃-alkyl-, (C₁₋₂-Alkylamino)-carbonyl-C₁₋₃-alkyl-, oder C₁₋₄-Alkoxycarbonyl-C₂₋₃-alkenyl-gruppe,
substituierte Phenylgruppe ist.

3. Verbindungen der allgemeinen Formel I gemäß Anspruch 1, in der
X, R¹, R², R⁴, R⁵ und R⁶ wie in Anspruch 1 definiert sind und
R³ eine durch eine Carboxy-C₁₋₃-alkyl- oder C₁₋₄-Alkoxy-carbonyl-C₁₋₃-alkyl-gruppe substituierte Phenylgruppe ist.

4. Verbindungen der allgemeinen Formel I gemäß einem der Ansprüche 1 bis 3, in der
X, R¹, R³, R⁴, R⁵ und R⁶ wie in einem der Ansprüch 1 bis 3 definiert sind und
R² ein Fluor- oder Chlor-atom ist.

5. Folgende Verbindungen der allgemeinen Formel I gemäß Anspruch 1:
(a) 3-Z-[1-(4-Dimethylaminomethyl-anilino)-1-(3-(2-carboxy-ethyl)-phenyl)-methylen]-6-chlor-2-indolinon
(b) 3-Z-[1-(4-Dimethylaminomethyl-anilino)-1-(4-(2-carboxy-ethyl)-phenyl)-methylen]-6-fluor-2-indolinon
(c) 3-Z-[1-(4-Dimethylaminomethyl-anilino)-1-(3-(2-carboxy-ethyl)-phenyl)-methylen]-6-fluor-2-indolinon
(d) 3-Z-[-(4-(N-(4-Methyl-piperazin-1-yl-methylcarbonyl)-N-methyl-amino)-anilino)-1-(4-(2-carboxy-ethyl)-phenyl)-methylen]-6-fluor-2-indolinon
(e) 3-Z-[1-(4-(N-(2-Dimethylamino-ethyl)-N-methylsulfonyl-amino)-anilino)-1-(4-(2-carboxy-ethyl)-phenyl)-methylen]-6-fluor-2-indolinon
(f) 3-Z-[1-(4-(N-(3-Dimethylamino-propyl)-N-acetyl-amino)-anilino)-1-(4-(2-carboxyethyl)-phenyl)-methylen]-6-fluor-2-indolinon
(g) 3-Z-[1-(4-(1-Methyl-imidazol-2-yl)-anilino)-1-(4-(2-carboxy-ethyl)-phenyl)-methylen]-6-fluor-2-indolinon
(h) 3-Z-[1-(4-(N-(Dimethylamino-methylcarbonyl)-N-methyl-amino)-anilino)-1-(4-(2-carboxy-ethyl)-phenyl)-methylen]-6-fluor-2-indolinon
(i) 3-Z-[1-(4-(N-(2-Dimethylamino-ethylcarbonyl)-N-mothyl-amino)-anilino)-1-(4-(2-carboxy-ethyl)-phenyl)-methylen]-6-fluor-2-Indolinon
(j) 3-Z-[1-(4-(Pyrrolidin-1-yl-methyl)-anilino)-1-(4-(2-carboxy-ethyl)-phenyl)-methylen]-6-fluor-2-indolinon
(k) 3-Z-[1-(4-(Diethylaminomethyl)-anilino)-1-(4-(2-carboxy-ethyl)-phenyl)-methylen]-6-fluor-2-indolinon
(l) 3-Z-[1-(4-(2-Dimethylamino-ethyl)-anilino)-1-(4-(2-carboxy-ethyl)-phenyl)-methylen]-6-chlor-2-indolinon
(m) 3-Z-[1-(4-Dimethylaminomethyl-anilino)-1-(4-(2-carboxy-ethyl)-phenyl)-methylen]-6-chlor-2-indolinon
(n) 3-Z-[1-(4-(Pyrrolidin-1-yl-methyl)-anilino)-1-(4-(2-carboxy-ethyl)-phenyl)-methylen]-6-chlor-2-indolinon
(o) 3-Z-[1-(4-(Pyrrolidin-1-yl-methyl)-anilino)-1-(4-(2-carboxy-ethyl)-phenyl)-methylen]-6-brom-2-indolinon
(p) 3-Z-[1-(4-(Dimethylaminomethyl)-anilino)-1-(4-(2-carboxy-ethyl)-phenyl)-methylen]-6-brom-2-indolinon
(q) 3-Z-[1-(4-(Diethylaminomethyl)-anilino)-1-(4-(2-carboxy-ethyl)-methylen]-6-brom-2-indolinon
sowie deren Salze.

6. Folgende Verbindung der allgemeinen Formel I gemäß Anspruch 1:
(b) 3-Z-[1-(4-Dimethylaminomethyl-anilino)-1-(4-(2-carboxy-ethyl)-phenyl)-methylen]-6-fluor-2-indolinon

7. Physiologisch verträgliche Salze der Verbindungen gemäß einem der Ansprüche 1 bis 6.

8. Arzneimittel enthaltend eine Verbindung der allgemeinen Formel I nach einem der Ansprüche 1 bis 6, oder ein physiologisch verträgliches Salz nach Anspruch 7, neben gegebenenfalls einem oder mehreren inerten Trägerstoffen und/oder Verdünnungsmitteln.

9. Verwendung einer Verbindung der allgemeinen Formel I nach mindestens einem der Ansprüche 1 bis 6, oder eines physiologisch verträglichen Salzes nach Anspruch 7 zur Herstellung eines Arzneimittels, welches zur Behandlung von exzessiven oder anomalen Zellproliferationen geeignet ist.

10. Verfahren zur Herstellung eines Arzneimittels gemäß Anspruchs 8, **dadurch gekennzeichnet, daß** auf nichtchemischem Wege eine Verbindung der allgemeinen Formel I nach mindestens einem der Ansprüche 1 bis 6, oder ein physiologisch verträgliches Salz nach Anspruch 7 in einen oder mehrere inerte Trägerstoffe und/oder Verdünnungsmittel eingearbeitet wird.

11. Verfahren zur Herstellung der Verbindungen gemäß den Ansprüchen 1 bis 5, **dadurch gekennzeichnet, daß**
a. eine Verbindung der allgemeinen Formel 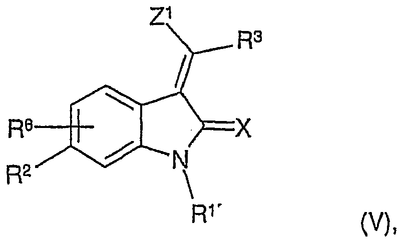 in der
die Reste Z¹ und R³ gegebenenfalls die Positionen tauschen können,
X, R², R³ und R⁶ wie in Anspruch 1 erwähnt definiert sind,
R¹' die für R¹ eingangs erwähnten Bedeutungen besitzt oder eine Schutzgruppe für das Stickstoffatom der Lactamgruppe darstellt, wobei R¹ auch eine gegebenenenfalls über einen Spacer gebildete Bindung an eine Festphase darstellen kann,
und Z¹ ein Halogenatom, eine Hydroxy-, Alkoxy- oder Aryl-alkoxygruppe, z.B. ein Chlor- oder Bromatom, eine Methoxy-, Ethoxy- oder Benzyloxygruppe, bedeutet,
mit einem Amin der allgemeinen Formel 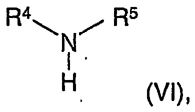 in der
R⁴ und R⁵ wie eingangs erwähnt definiert sind, umgesetzt wird und erforderlichenfalls anschließende Abspaltung einer verwendeten Schutzgruppe für das Stickstoffatom der Lactamgruppe oder von einer Festphase,
b. zur Herstellung einer Verbindung der allgemeinen Formel I, in der R³ eine durch eine C₁₋₄-Alkoxy-carbonyl-C₂₋₃-alkenylgruppe substituierte Phenyl-gruppe darstellt,
eine Verbindung der allgemeinen Formel 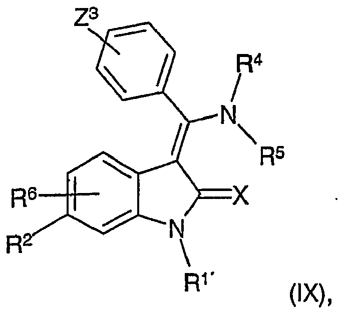 in der
R², R⁴, R⁵, R⁶ und X wie in Anspruch 1 erwähnt definiert sind,
R¹' die für R¹ eingangs erwähnten Bedeutungen besitzt oder eine Schutzgruppe für das Stickstoffatom der Lactamgruppe darstellt, wobei R¹' auch eine gegebenenenfalls über einen Spacer gebildete Bindung an eine Festphase darstellen kann, und Z³ eine Austrittsgruppe, beispielsweise ein Halogenatom oder eine Alkyl- oder Arylsulfonyloxygruppe wie das Chlor-, Brom- oder Iodatom oder die Methylsulfonyloxy-, Ethylsulfonyloxy-, p-Toluolsulfonyloxy-, oder Trifluormethansulfonyloxygruppe darstellt, mit einem Alken der allgemeinen Formel 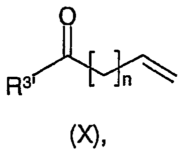 in der
R³ eine C₁₋₄-Alkoxygruppe und
n die Zahl 0 oder 1 bedeutet, umgesetzt wird;
c. zur Herstellung einer Verbindung der allgemeinen Formel I, in der R³ eine durch eine Carboxy-C₁₋₃-alkyl-, C₁₋₄-Alkoxy-carbonyl-C₁₋₃-alkyl-, Aminocarbonyl-C₁₋₃-alkyl-, oder (C₁₋₂-Alkylamino)-carbonyl-C₁₋₃-alkyl-gruppe substituierte Phenyl-gruppe darstellt,
eine Verbindung der allgemeinen Formel 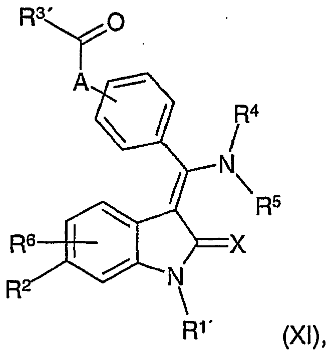 in der
R², R⁴, R⁵, R⁶ und X wie in Anspruch 1 erwähnt definiert sind,
R¹' die für R¹ eingangs erwähnten Bedeutungen besitzt oder eine Schutzgruppe für das Stickstoffatom der Lactamgruppe darstellt, wobei R¹' auch eine gegebenenenfalls über einen Spacer gebildete Bindung an eine Festphase darstellen kann,
A eine C₂₋₃-Alkenylgruppe und
R³ eine Hydroxy-, C₁₋₄-Alkoxy-, Amino-oder (C₁₋₂-Alkylamino)-gruppe darstellt, hydriert wird
und anschließend gegebenenfalls verwendete Schutzgruppen für das Stickstoffatom der Lactamgruppe oder von einer Festphase wie vorstehend unter Verfahren (a) beschrieben abgespalten wird,
und anschließend gegebenenfalls eine Alkoxycarbonylgruppe mittels Hydrolyse in eine entsprechende Carboxyverbindung übergeführt wird, oder
eine Amino- oder Alkylaminogruppe mittels reduktiver Alkylierung in eine entsprechende Alkylamino- oder Dialkylamino-verbindung übergeführt wird, oder
eine Dialkylaminogruppe mittels Alkylierung in eine entsprechenden Trialkylammoniumverbindung übergeführt wird
eine Amino- oder Alkylamino-gruppe mittels Acylierung oder Sulfonierung in eine entsprechende Acyl- oder Sulfonyl-verbindung übergeführt wird, oder
eine Carboxygruppe mittels Veresterung oder Amidierung in eine entsprechende Ester- oder Aminocarbonyl-verbindung übergeführt wird, oder
eine Nitrogruppe mittels Reduktion in eine entsprechende Aminoverbindung übergeführt wird, oder
eine Cyanogruppe mittels Reduktion in eine entsprechende Aminomethylverbindung übergeführt wird, oder
eine Arylalkyloxygruppe mittels Säure in eine entsprechende Hydroxyverbindung übergeführt wird, oder
eine Alkoxycarbonylgruppe mittels Verseifung in eine entsprechende Carboxyverbindung übergeführt wird, oder
eine durch eine Amino-, Alkylamino-, Aminoalkyl- oder N-Alkyl-amino-gruppe substituierte Phenylgruppe mittels Umsetzung mit einer entsprechenden die Amidinogruppe übertragenden Verbindung oder durch Umsetzung mit einem entsprechenden Nitril in eine entsprechende Guanidinoverbindung der allgemeinen Formel I übergeführt wird.

12. Folgende Verbindung der allgemeinen Formel I gemäß Anspruch 1: 3-Z-[1-(4-Dimethylaminomethyl-anilino)-1-(4-(2-ethoxycarbonylethyl)-phenyl)-methylen]-6-fluor-2-indolinon

13. 1-Acetyl-6-fluor-2-indolinon

## Claims

1. Compounds of general formula 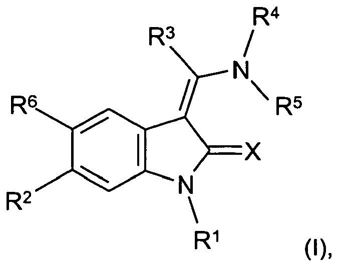 wherein
X is an oxygen atom,
R' is a hydrogen atom,
R² is a fluorine, chlorine or bromine atom or a cyano group,
R³ is a phenyl group substituted in the 3- or 4-position
by a carboxy-C₁₋₃-alkyl, C₁₋₄-alkoxy-carbonyl-C₁₋₃-alkyl, aminocarbonyl-C₁₋₃-alkyl, (C₁₋₂-alkylamino)-carbonyl-C₁₋₃-alkyl or C₁₋₄-alkoxy-carbonyl-C₂₋₃-alkenyl group,
R⁴ is a phenyl group or a phenyl group which is monosubstituted
by a C₁₋₃-alkyl group which is terminally substituted by an amino, guanidino, mono- or di-(C₁₋₂-alkyl)-amino-, N-[ω-di-(C₁₋₃-alkyl)-amino-C₂₋₃-alkyl]-N-(C₁₋₃-alkyl)-amino, N-methyl-(C₃₋₄-alkyl)-amino, N-(C₁₋₃-alkyl)-N-benzylamino, N-(C₁₋₄-alkoxycarbonyl)-amino, N-(C₁₋₄-alkoxycarbonyl)-C₁₋₄-alkylamino, 4-(C₁₋₃-alkyl)-piperazin-1-yl, imidazol-1-yl, pyrrolidin-1-yl, azetidin-1-yl, morpholin-4-yl, piperazin-1-yl, thiomorpholin-4-yl group,
by a di-(C₁₋₃-alkyl)-amino-(C₁₋₃-alkyl)-sulphonyl, 2-[di-(C₁₋₃-alkyl)-amino]-ethoxy, 4-(C₁₃-alkyl)-piperazin-1-yl-carbonyl, {ω[di-(C₁₋₃-alkyl)-amino]-(C₂₋₃-alkyl)}-N-(C₁₋₃-alkyl)-amino-carbonyl, 1-(C₁₋₃-alkyl)imidazol-2-yl, (C₁₋₃-alkyl)-sulphonyl group, or
by a group of the formula 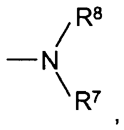 wherein
R⁷ is a C₁₋₂-alkyl, C₁₋₂-alkyl-carbonyl, di-(C₁₋₂-alkyl)-amino-carbonyl-C₁₋₃-alkyl or C₁₋₃-alkylsulphonyl group and
R⁸ is C₁₋₃-alkyl, ω-[di-(C₁₋₂-alkyl)-amino]-C₂₋₃-alkyl, ω-[mono-(C₁₋₂-alkyl)-amino]-C₂₋₃-alkyl group, or
a (C₁₋₃-alkyl)-carbonyl, (C₄₋₆-alkyl)-carbonyl or carbonyl-(C₁₋₃-alkyl) group which is terminally substituted by a di-(C₁₋₂-alkyl)-amino, piperazin-1-yl or 4-(C₁₋₃-alkyl)-piperazin-1-yl group,
where all dialkylamino groups present in the group R⁴ may also be present in quaternized form, for example as an N-methyl-(N,N-dialkyl)-ammonium group, where the counterion is preferably selected from the group consisting of iodide, chloride, bromide, methylsulphonate, para-toluenesulphonate and trifluoroacetate,
R⁵ is a hydrogen atom and
R⁶ is a hydrogen atom,
where the abovementioned alkyl groups include linear and branched alkyl groups in which additionally one to 3 hydrogen atoms may be replaced by fluorine atoms,
and the tautomers, enantiomers, diastereomers thereof, mixtures thereof and salts thereof.

2. Compounds of general formula I according to Claim 1, wherein
X, R¹, R², R⁴, R⁵ and R⁶ are as defined in Claim 1 and
R³ is a phenyl group which is substituted
by a carboxy-C₁₋₃-alkyl, C₁₋₄-alkoxy-carbonyl-C₁₋₃-alkyl, aminocarbonyl-C₁₋₃-alkyl, (C₁₋₂-alkylamino)-carbonyl-C₁₋₃-alkyl or C₁₋₄-alkoxy-carbonyl-C₂₋₃-alkenyl group.

3. Compounds of general formula I according to Claim 1, wherein
X, R¹, R², R⁴, R⁵ and R⁶ are as defined in Claim 1 and
R³ is a phenyl group substituted by a carboxy-C₁₋₃-alkyl or C₁₋₄-alkoxy-carbonyl-C₁₋₃-alkyl group.

4. Compounds of general formula I according to one of Claims 1 to 3, wherein
X, R¹, R³, R⁴, R⁵ and R⁶ are as defined in one of Claims 1 to 3 and
R² is a fluorine or chlorine atom.

5. The following compounds of general formula I according to Claim 1:
(a) 3-Z-[1-(4-dimethylaminomethylanilino)-1-(3-(2-carboxyethyl)phenyl)methylene]-6-chloro-2-indolinone
(b) 3-Z-[1-(4-dimethylaminomethylanilino)-1-(4-(2-carboxyethyl)phenyl)methylene]-6-fluoro-2-indolinone
(c) 3-Z-[1-(4-dimethylaminomethylanilino)-1-(3-(2-carboxyethyl)phenyl)methylene]-6-fluoro-2-indolinone
(d) 3-Z-[1 -(4-(N-(4-methylpiperazin-1-ylmethylcarbonyl)-N-methylamino)anilino)-1-(4-(2-carboxyethyl)phenyl)methylene]-6-fluoro-2-indolinone
(e) 3-Z-[1-(4-(N-(2-dimethylaminoethyl)-N-methylsulphonylamino)anilino)-1-(4-(2-carboxyethyl)phenyl)methylene]-6-fluoro-2-indolinone
(f) 3-Z-[1-(4-(N-(3-dimethylaminopropyl)-N-acetylamino)anilino)-1-(4-(2-carboxyethyl)phenyl)methylene]-6-fluoro-2-indolinone
(g) 3-Z-[1-(4-(1-methylimidazol-2-yl)anilino)-1-(4-(2-carboxyethyl)phenyl)methylene]-6-fluoro-2-indolinone
(h) 3-Z-[1-(4-(N-(dimethylaminomethylcarbonyl)-N-methylamino)anilino)-1-(4-(2-carboxyethyl)phenyl)methylene]-6-fluoro-2-indolinone
(i) 3-Z-[1-(4-(N-(2-dimethylaminoethylcarbonyl)-N-methylamino)anilino)-1-(4-(2-carboxyethyl)phenyl)methylene]-6-fluoro-2-indolinone
(j) 3-Z-[1-(4-(pyrrolidin-1-ylmethyl)anilino)-1-(4-(2-carboxyethyl)phenyl)methylene]-6-fluoro-2-indolinone
(k) 3-Z-[1-(4-(diethylaminomethyl)anilino)-1-(4-(2-carboxyethyl)phenyl)methylene]-6-fluoro-2-indolinone
(l) 3-Z-[1-(4-(2-dimethylaminoethyl)anilino)-1-(4-(2-carboxyethyl)phenyl)methylene]-6-chloro-2-indolinone
(m) 3-Z-[1-(4-dimethylaminomethylanilino)-1-(4-(2-carboxyethyl)phenyl)methylene]-6-chloro-2-indolinone
(n) 3-Z-[1-(4-(pyrrolidin-1-ylmethyl)anilino)-1-(4-(2-carboxyethyl)phenyl)methylene]-6-chloro-2-indolinone
(o) 3-Z-[1-(4-(pyrrolidin-1-ylmethyl)anilino)-1-(4-(2-carboxyethyl)phenyl)methylene]-6-bromo-2-indolinone
(p) 3-Z-[1-(4-(dimethylaminomethyl)anilino)-1-(4-(2-carboxyethyl)phenyl)methylene]-6-bromo-2-indolinone
(q) 3-Z-[1-(4-(diethylaminomethyl)anilino)-1-(4-(2-carboxyethyl)-methylene]-6-bromo-2-indolinone
and the salts thereof.

6. The following compound of general formula I according to claim 1:
(b) 3-Z-[1-(4-dimethylaminomethylanilino)-1-(4-(2-carboxyethyl)phenyl)methylene]-6-fluoro-2-indolinone
and the salts thereof.

7. Physiologically acceptable salts of the compounds according to one of Claims 1 to 6.

8. A medicament, comprising a compound of general formula I according to one of Claims 1 to 6 or a physiologically acceptable salt according to Claim 7, optionally in addition to one or more inert carrier materials and/or diluents.

9. Use of a compound of general formula I according to at least one of Claims 1 to 6 or of a physiologically acceptable salt according to Claim 7 in the manufacture of a medicament for the treatment of excessive or abnormal cell proliferation.

10. A process for preparing a medicament according to Claim 8, **characterized in that**, by a non-chemical route, a compound of general formula I according to at least one of Claims 1 to 6 or a physiologically acceptable salt according to Claim 7 is incorporated into one or more inert carrier materials and/or diluents.

11. A process for preparing the compounds according to Claims 1 to 5, **characterized in that**
a. a compound of general formula 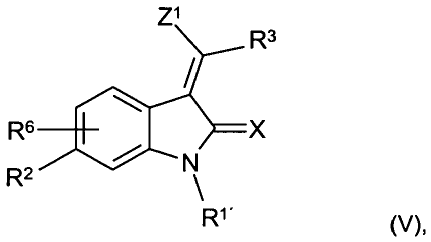 in which
groups Z¹ and R³ may optionally change their positions,
X, R², R³ and R⁶ are as defined in Claim 1,
R^{1,} has the meanings mentioned at the outset for R¹ or is a protecting group for the nitrogen atom of the lactam group, while R¹ may also represent a bond, optionally formed via a spacer, to a solid phase,
and Z¹ is a halogen atom, a hydroxy, alkoxy or arylalkoxy group, for example a chlorine or bromine atom, a methoxy, ethoxy or benzyloxy group,
is reacted with an amine of general formula 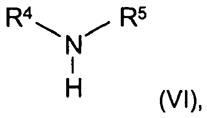 wherein
R⁴ and R⁵ are defined as mentioned at the outset,
and, if required, the product is subsequently cleaved from a protecting group used for the nitrogen atom of the lactam group or from a solid phase,
b. for preparing a compound of general formula I wherein R³ is a phenyl group substituted by a C₁₋₄-alkoxy-carbonyl-C₂₋₃-alkenyl group,
a compound of general formula 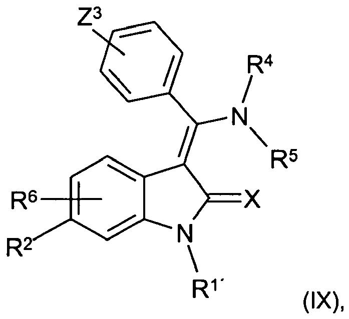 wherein
R², R⁴, R⁵, R⁶ and X are as defined in Claim 1,
R¹' has the meanings mentioned at the outset for R¹ or is a protecting group for the nitrogen atom of the lactam group, while R¹' may also represent a bond, optionally formed via a spacer, to a solid phase, and
Z³ is a leaving group, for example a halogen atom or an alkyl- or arylsulphonyloxy group, such as a chlorine, bromine or iodine atom or a methylsulphonyloxy, ethylsulphonyloxy, p-toluenesulphonyloxy or trifluoromethanesulphonyloxy group, is reacted with an alkene of general formula 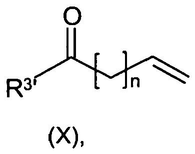 wherein
R³' is a C₁₋₄-alkoxy group and
n is the number 0 or 1,
c. to prepare a compound of general formula I, wherein R³ is a phenyl group substituted by a carboxy-C₁₋₃-alkyl, C₁₋₄-alkoxy-carbonyl-C₁₋₃-alkyl, aminocarbonyl-C₁₋₃-alkyl or (C₁₋₂-alkylamino)-carbonyl-C₁₋₃-alkyl group,
a compound of general formula 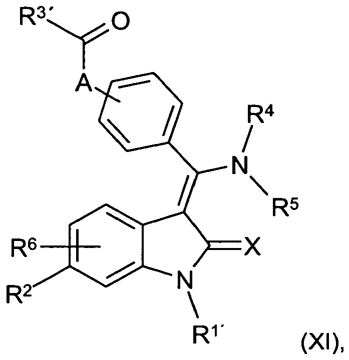 wherein
R², R⁴, R⁵, R⁶ and X are as defined in claim 1,
R¹' has the meanings mentioned at the outset for R¹ or is a protecting group for the nitrogen atom of the lactam group, where R¹' may also represent a bond, optionally formed via a spacer, to a solid phase,
A is a C₂₋₃-alkenyl group and
R³ is a hydroxy, C₁₋₄-alkoxy, amino or (C₁₋₂-alkylamino) group,
is hydrogenated
and subsequently cleaved from any protecting groups optionally used for the nitrogen atom of the lactam group or from a solid phase, as described above under process (a),
and an alkoxycarbonyl group is optionally subsequently converted by hydrolysis into a corresponding carboxyl compound, or
an amino or alkylamino group is converted by reductive alkylation into a corresponding alkylamino or dialkylamino compound, or
a dialkylamino group is converted by alkylation into a corresponding trialkylammonium compound, or
an amino or alkylamino group is converted by acylation or sulphonation into a corresponding acyl or sulphonyl compound, or
a carboxyl group is converted by esterification or amidation into a corresponding ester or aminocarbonyl compound, or
a nitro group is converted by reduction into a corresponding amino compound, or a cyano group is converted by reduction into a corresponding aminomethyl compound, or
an arylalkyloxy group is converted with an acid into a corresponding hydroxy compound, or
an alkoxycarbonyl group is converted by saponification into a corresponding carboxyl compound, or
a phenyl group substituted by an amino, alkylamino, aminoalkyl or N-alkyl-amino group is converted by reaction with an appropriate amidino-group-transferring compound or by reaction with an appropriate nitrile into a corresponding guanidine compound of general formula I.

12. The following compound of general formula I according to claim 1:
3-Z-[1-(4-dimethylaminomethylanilino)-1-(4-(2-ethoxycarbonylethyl)phenyl)methylene]-6-fluoro-2-indolinone.

13. 1-acetyl-6-fluoro-2-indolinone.

## Revendications

1. Composés de formule générale 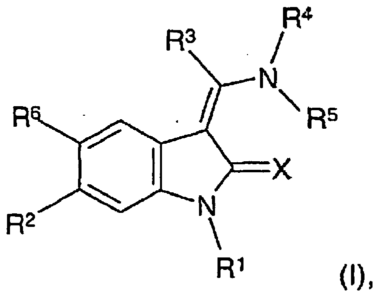 dans laquelle
X est un atome d'oxygène,
R¹ est un atome d'hydrogène,
R² est un atome de fluor, de chlore ou de brome ou un groupe cyano,
R³ est un groupe phényle, substitué en position 3 ou 4
par un groupe carboxy-alkyle en C₁ à C₃, alcoxy en C₁ à C₄-carbonyle-alkyle en C₁ à C₃, aminocarbonyl-alkyle en C₁ à C₃, (alkylamino en C₁ à C₂) - carbonyl-alkyle en C₁ à C₃, ou alcoxy en C₁ à C₄-carbonyl-alcényle en C₂ à C₃,
R⁴ est un groupe phényle ou un groupe phényle monosubstitué
par un groupe alkyle en C₁ à C₃ substitué au niveau terminal par un groupe amino, guanidino, mono- ou di-(alkyle en C₁ à C₂)-amino, N-[ω-di-(alkyle en C₁ à C₃)-amino-alkyle en C₂ à C₃] -N-(alkyle en C₁ à C₃)-amino, N-méthyl-(alkyle en C₃ à C₄) -amino, N-(alkyle en C₁ à C₃) -N-benzylamino, N-(alcoxycarbonyle en C₁ à C₄)-amino, N-(alcoxycarbonyle en C₁ à C₄)-alkylamino en C₁ à C₄, 4-(alkyle en C₁ à C₃)-pipérazin-1-yle, imidazol-1-yle, pyrrolidin-1-yle, azétidin-1-yle, morpholin-4-yle, pipérazin-1-yle, thiomorpholin-4-yle,
par un groupe di- (alkyle en C₁ à C₃)-amino-(alkyle en C₁ à C₃)-sulfonyle, 2-[di-(alkyle en C₁ à C₃) - amino]-éthoxy, 4-(alkyle en C₁ à C₃) -pipérazin-1-yl-carbonyle, {ω-[di-(alkyle en C₁ à C₃)-amino]-(alkyle en C₂ à C₃)}-N-(alkyle en C₁ à C₃)-aminocarbonyle, 1-(alkyle en C₁ à C₃)-imidazol-2-yle, (alkyle en C₁ à C₃)-sulfonyle, ou
par un groupe de formule 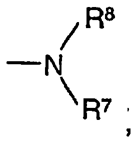 dans laquelle
R⁷ est un groupe alkyle en C₁ à C₂, alkyle en C₁ à C₂-carbonyle, di-(alkyle en C₁ à C₂)-aminocarbonyle-alkyle en C₁ à C₃ ou alkylsulfonyle en C₁ à C₃ et
R⁸ est un groupe alkyle en C₁ à C₃, ω-[di-(alkyle en C₁ à C₂)-amino]-alkyle en C₂ à C₃, ω-(mono-(alkyle en C₁ à C₂)-amino]-alkyle en C₂ à C₃, ou
un groupe (alkyle en C₁ à C₃)-carbonyle, (alkyle en C₄ à C₆)-carbonyle ou carbonyl-(alkyle en C₁ à C₃) substitué au niveau terminal par un groupe di-(alkyle en C₁ à C₂)-amino, pipérazin-1-yle ou 4-(alkyle en C₁ à C₃)-pipérazin-1-yle,
dans laquelle tous les groupes dialkylamino contenus dans le radical R⁴ peuvent se présenter également sous forme quaternisée, par exemple en tant que groupe N-méthyl-(N,N-dialkyl)-ammonium, le contre-ion étant choisi de préférence parmi un iodure, un chlorure, un bromure, un méthylsulfonate, un para-toluènesulfonate ou un trifluoro-acétate,
R⁵ désigne un atome d'hydrogène et
R⁶ désigne un atome d'hydrogène
dans laquelle les groupes alkyle mentionnés ci-dessus comprennent des groupes alkyle linéaires et ramifiés dans lesquels en outre un à trois atomes d'hydrogène peuvent être substitués par des atomes de fluor,
leurs tautomères, leurs énantiomères, leurs diastéréomères, leurs mélanges et leurs sels.

2. Composés de formule générale I selon la revendication 1, dans laquelle
X, R¹, R², R⁴, R⁵ et R⁶ sont tels que définis dans la revendication 1 et
R³ est un groupe phényle substitué
par un groupe carboxy-alkyle en C₁ à C₃, alcoxy en C₁ à C₄-carbonyl-alkyle en C₁ à C₃, aminocarbonyl-alkyle en C₁ à C₃, (alkylamino en C₁ à C₂) - carbonyl-alkyle en C₁ à C₃, ou alcoxy en C₁ à C₄-carbonyl-alcényle en C₂ à C₃.

3. Composés de formule générale I selon la revendication 1, dans laquelle
X, R¹, R² R⁴ R⁵ et R⁶ sont tels que définis dans la revendication 1 et
R³ est un groupe phényle substitué par un groupe carboxy-alkyle en C₁ à C₃ ou alcoxy en C₁ à C₄-carbonyle-alkyle en C₁ à C₃.

4. Composés de formule générale I selon l'une des revendications 1 à 3, dans laquelle
X, R¹, R³, R⁴, R⁵ et R⁶ sont tels que définis dans les revendications 1 à 3 et
R² est un atome de fluor ou de chrome.

5. Composés de formule générale I suivants, selon la revendication 1 :
(a) 3-Z-[1-(4-diméthylaminométhyl-anilino)-1-(3-(2-carboxy-éthyl)-phényl)-méthylène]-6-chloro-2-indolinone
(b) 3-Z-[1-(4-diméthylaminométhyl-anilino)-1-(4-(2-carboxy-éthyl)-phényl)-méthylène]-6-fluoro-2-indolinone
(c) 3-Z-[1-(4-diméthylaminométhyl-anilino)-1-(3-(2-carboxy-éthyl)-phényl)-méthylène]-6-fluoro-2-indolinone
(d) 3-Z-[1-(4-(N-(4-méthyl-pipérazin-1-yl-méthylcarbonyl)-N-méthyl-amino)-anilino)-1-(4-(2-carboxy-éthyl)-phényl)-méthylène]-6-fluoro-2-indolinone
(e) 3-Z-[1-(4-(N-(2-diméthylamino-éthyl)-N-méthylsulfonyl-amino)-anilino)-1-(4-(2-carboxy-éthyl)-phényl)-méthylène]-6-fluoro-2-indolinone
(f) 3-Z-[1-(4-(N-(3-diméthylamino-propyl)-N-acétyl-amino)-anilino)-1-(4-(2-carboxy-éthyl)-phényl)-méthylène]-6-fluoro-2-indolinone
(g) 3-Z-[1-(4-(1-méthyl-imidazol-2-yl)-anilino)-1-(4-(2-carboxy-éthyl)-phényl)-méthylène]-6-fluoro-2-indolinone
(h) 3-Z-[1-(4-(N-(diméthylamino-méthylcarbonyl)-N-méthyl-amino)-anilino)-1-(4-(2-carboxy-éthyl)-phényl)-méthylène]-6-fluoro-2-indolinone
(i) 3-Z-[1-(4-(N-(2-diméthylamino-éthylcarbonyl)-N-méthyl-amino)-anilino)-1-(4-(2-carboxy-éthyl)-phényl)-méthylène]-6-fluoro-2-indolinone
(j) 3-Z-[1-(4-(pyrrolidin-1-yl-méthyl)-anilino)-1-(4-(2-carboxy-éthyl)-phényl)-méthylène]-6-fluoro-2-indolinone
(k) 3-Z-[1-(4-(diéthylaminométhyl)-anilino)-1-(4-(2-carboxy-éthyl)-phényl)-méthylène]-6-fluoro-2-indolinone
(l) 3-Z-[1-(4-(2-diméthylamino-éthyl)-anilino)-1-(4-(2-carboxy-éthyl)-phényl)-méthylène]-6-chloro-2-indolinone (m) 3-Z-[1-(4-(diméthylaminométhyl)-anilino)-1-(4-(2-carboxy-éthyl)-phényl)-méthylène]-6-chloro-2-indolinone
(n) 3-Z-[1-(4-(pyrrolidin-1-yl-méthyl)-anilino)-1-(4-(2-carboxy-éthyl)-phényl)-méthylène]-6-chloro-2-indolinone
(o) 3-Z-[1-(4-(pyrrolidin-1-yl-méthyl)-anilino)-1-(4-(2-carboxy-éthyl)-phényl)-méthylène]-6-bromo-2-indolinone
(p) 3-Z-[1-(4-(diméthylaminométhyl)-anilino)-1-(4-(2-carboxy-éthyl)-phényl)-méthylène]-6-bromo-2-indolinone
(q) 3-Z-[1-(4-(diéthylaminométhyl)-anilino)-1-(4-(2-carboxy-éthyl)-méthylène]-6-bromo-2-indolinone et leurs sels.

6. Composé de formule générale I suivant selon la revendication 1 :
(b) 3-Z-[1-(4-diméthylaminométhyl-anilino)-1-(4-(2-carboxy-éthyl)-phényl)-méthylène]-6-fluoro-2-indolinone
et ses sels.

7. Sels physiologiquement acceptables des composés selon l'une des revendications 1 à 6.

8. Médicament contenant un composé de formule générale I selon l'une des revendications 1 à 6, ou un sel physiologiquement acceptable selon la revendication 7, éventuellement en plus d'un ou plusieurs véhicules et/ou diluants inertes.

9. Utilisation d'un composé de formule générale I selon au moins l'une des revendications 1 à 6 ou d'un sel physiologiquement acceptable selon la revendication 7, pour la production d'un médicament qui est approprié pour le traitement des proliférations cellulaires excessives ou anormales.

10. Procédé de production d'un médicament selon la revendication 8, **caractérisé en ce que**, de façon non chimique, un composé de formule générale I selon au moins l'une des revendications 1 à 6 ou un sel physiologiquement acceptable selon la revendication 7 est intégré dans un ou plusieurs véhicules et/ou diluants inertes.

11. Procédé de production des composés selon les revendications 1 à 5, **caractérisé en ce que** l'on fait réagir
a. un composé de formule générale 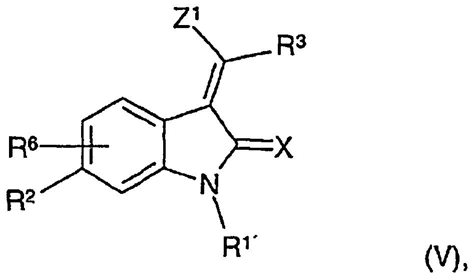 dans laquelle
les radicaux Z¹ et R³ peuvent éventuellement échanger leurs positions,
X, R² R³ et R⁶ sont tels que définis dans la revendication 1,
R^{1'} a les significations indiquées initialement pour R¹ ou représente un groupe protecteur de l'atome d'azote du groupe lactame, R¹ pouvant représenter aussi une liaison formée éventuellement par un segment d'espacement à une phase solide,
et Z¹ désigne un atome d'halogène, un groupe hydroxy, alcoxy ou aryl-alcoxy, par exemple, un atome de chlore ou de brome, un groupe méthoxy, éthoxy ou benzyloxy,
avec une amine de formule générale 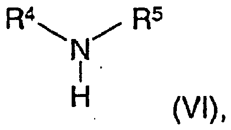 dans laquelle
R⁴ et R⁵ sont tels que définis initialement,
et si nécessaire par clivage consécutif d'un groupe protecteur utilisé pour l'atome d'azote du groupe lactame ou d'une phase solide,
b. pour produire un composé de formule générale I, dans laquelle R³ est un groupe phényle substitué par un groupe alcoxy en C₁ à C₄-carbonyle-alcényle en C₂ à C₃, on fait réagir
un composé de formule générale 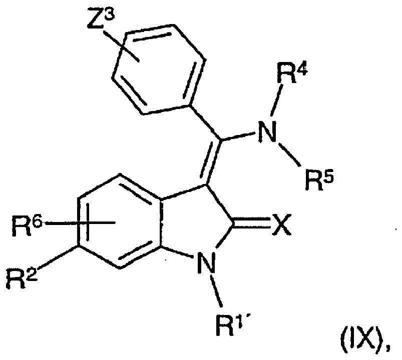 dans laquelle
R², R⁴, R⁵, R⁶ et X sont tels que définis dans la revendication 1,
R^{1'} a les significations mentionnées initialement pour R¹ ou représente un groupe protecteur pour l'atome d'azote du groupe lactame, R^{1'} pouvant également représenter une liaison formée éventuellement par un segment d'espacement à une phase solide, et
Z³ est un groupe partant, par exemple, un atome d'halogène ou un groupe alkyle ou arylsulfonyloxy tel que l'atome de chlore, de brome ou d'iode ou le groupe méthylsulfonyloxy, éthylsulfonyloxy, p-toluènesulfonyloxy ou trifluorométhanesulfonyloxy, avec un alcène de formule générale 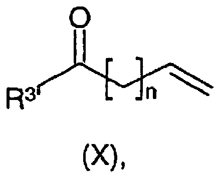 dans laquelle
R^{3'} est un groupe alcoxy en C₁ à C₄ et n désigne le nombre 0 ou 1,
c. pour la production d'un composé de formule générale I dans laquelle R³ est un groupe phényle substitué par un groupe carboxy-alkyle en C₁ à C₃, alcoxy en C₁ à C₄-carbonyle-alkyle en C₁ à C₃, aminocarbonyl-alkyle en C₁ à C₃ ou (alkylamino en C₁ à C₂)-carbonyl-alkyle en C₁ à C₃,
on hydrogène
un composé de formule générale 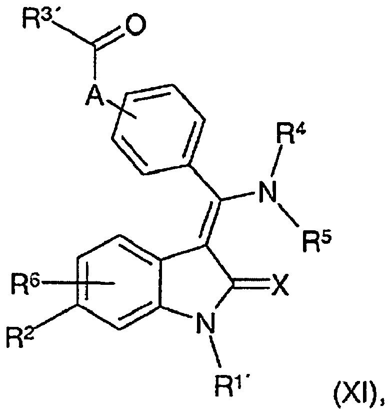 dans laquelle
R², R⁴, R⁵, R⁶ et X sont tels que définis dans la revendication 1,
R^{1'} a les significations mentionnées initialement pour R¹ ou représente un groupe protecteur pour l'atome d'azote du groupe lactame, R^{1'} pouvant également représenter une liaison formée éventuellement par un segment d'espacement à une phase solide, et
A est un groupe alcényle en C₂ à C₃ et
R^{3'} est un groupe hydroxy, alcoxy en C₁ à C₄, amino- ou (alkylamino en C₁ à C₂)
et ensuite, des groupes protecteurs éventuellement utilisés pour l'atome d'azote du groupe lactame ou d'une phase solide telle que décrite précédemment pour le procédé (a) sont clivés,
et ensuite, éventuellement, un groupe alcoxycarbonyle est converti par hydrolyse en un composé carboxy correspondant, ou
un groupe amino ou alkylamino est converti par alkylation réductrice en un composé alkylamino ou dialkylamino correspondant, ou
un groupe dialkylamino est converti par alkylation en un composé trialkylammonium correspondant,
un groupe amino ou alkylamino est converti par acylation ou sulfonation en un composé acyle ou sulfonyle correspondant, ou
un groupe carboxy est converti par estérification ou amidation en un composé ester ou aminocarbonyle correspondant, ou
un groupe nitro est converti par réduction en un composé amino correspondant, ou
un groupe cyano est converti par réduction en un composé aminométhyle correspondant, ou
un groupe arylalkyloxy est converti par un acide en un composé hydroxy correspondant, ou
un groupe alcoxycarbonyle est converti par saponification en un composé carboxy correspondant, ou un groupe phényle substitué par un groupe amino, alkylamino, aminoalkyle ou N-alkylamino est converti en réagissant avec un composé correspondant transmettant le groupe amidino ou en réagissant avec un nitrile correspondant en un composé guanidino correspondant, de formule générale I.

12. Composé de formule générale I suivant, selon la revendication 1 _{:}
3-Z-[1-(4-(diméthylaminométhyl)-anilino)-1-(4-(2-éthoxycarbonyl-éthyl)-phényl)-méthylène]-6-fluoro-2-indolinone.

13. 1-acétyl-6-fluoro-2-indolinone.
